# EUROPEAN PATENT SPECIFICATION

(11) **EP 3 820 453 B1**
(45) Date of publication and mention of the grant of the patent: **04.09.2024**
(21) Application number: 19834067.1
(22) Date of filing: 09.07.2019
(51) Int. Cl.: A61K 39/00, A61K 39/385, C12N 5/00, C12N 5/0783, A61K 9/14, A61K 9/51, A61K 31/337, A61K 35/17, B82Y 5/00, C07H 21/00

(54) **BIOMOLECULE COATED PARTICLES AND FILMS AND USES THEREOF**
MIT BIOMOLEKÜLEN BESCHICHTETE PARTIKEL UND FILME UND DEREN VERWENDUNGEN
PARTICULES ET FILMS ENROBÉS DE BIOMOLÉCULES ET LEURS UTILISATIONS

(30) Priority: 10.07.2018 US 201862696191 P; 21.03.2019 US 201962821879 P
(43) Date of publication of application: 19.05.2021
(73) Proprietor: The Regents of the University of California, Oakland, CA 94607 (US)
(72) Inventor: DESAI, Tejal A., San Francisco, California 94118 (US); HUANG, Xiao, Gushi, Henan 465200 (CN); CHANG, Ryan, San Francisco, California 94105 (US); WILLIAMS, Jasper Z., San Francisco, California 94158 (US); LIM, Wendell A., San Francisco, California 94118 (US)
(74) Representative: Williams Powell
(86) International application number: PCT/US2019/041064
(87) International publication number: WO 2020/014270

(56) References cited:
- WO-A2-03/035829
- US-A1- 2004 219 526
- US-A1- 2014 371 432
- US-A1- 2015 000 404
- US-A1- 2015 157 737
- US-A1- 2016 008 399
- HUANG X ET AL: "DNA scaffolds enable efficient and tunable functionalization of biomaterials for immune cell modulation", NATURE NANOTECHNOLOGY, vol. 16, no. 2, February 2021 (2021-02-01), pages 214 - 223, XP037364612, ISSN: 1748-3387, DOI: 10.1038/S41565-020-00813-Z
- XIAO HUANG, JASPER Z. WILLIAMS, RYAN CHANG, ZHONGBO LI, ERIC GAI, DAVID M. PATTERSON, YU WEI, WENDELL A. LIM, TEJAL A. DESAI: "DNA-Scaffolded Biomaterials Enable Modular and Tunable Control of Cell -Based Cancer Immunotherapies", BIORXIV, 23 March 2019 (2019-03-23), pages 1 - 41, XP055768179, DOI: 10.1101/587105
- STEPHAN ET AL.: "Biopolymer Implants Enhance the Efficacy of Adoptive T Cell Therapy", NAT BIOTECHNOL, vol. 33, no. 1, 15 December 2014 (2014-12-15), pages 97 - 101, XP055277821, DOI: 10.1038/nbt.3104
- WANG ET AL.: "Bioengineering of Artificial Antigen Presenting Cells and Lymphoid Organs", THERANOSTICS, vol. 7, no. 14, 17 August 2017 (2017-08-17), pages 3504 - 3516, XP055676416

## Description

### INTRODUCTION

A major goal in the traditional biomolecule therapeutics and modern synthetic cell mimics has been the development of methods and compositions to facilitate the delivery of a biomolecule efficiently and effectively and/or to present targeting or modulatory signals to the appropriate cells and tissues that would benefit from such treatment. It is desirable to increase the efficiency, specificity and modularity of administration of therapeutic agents to the cells in a variety of pathological states. This is particularly important as relates to activation of certain cells. Thus, an efficient system made of synthetic biocompatible materials with controlled surface presentation of various biomolecules as well as efficient core loading would empower various cell modulations in diseases and targeted delivery of biomolecules to specific cells to reduce the associated "side effects" of treatments.

Also desirable are methods for increasing in vivo half-life of a biomolecule by attaching it to a solid support as well as providing solid supports with multiple biomolecules at controlled ratios and densities.

Additionally, it is desirable to provide strategies to improve ex vivo and in vivo expansion, persistence and killing potential of chimeric antigen receptor (CAR)-T cells.

### SUMMARY

The present disclosure provides polymeric particles comprising biomolecules of interest attached thereto, methods for using the same, and methods for making the same. The surface of the polymeric particles can be functionalized by attaching biomolecules of interest for presentation on the surface of the synthetic particles. Multiple different biomolecules of interest may be attached to the surface of the polymeric particles in a desired ratio for co-presentation. In addition, the polymeric particles may also encapsulate biomolecules, such as, therapeutic nucleic acids, peptide and/or polypeptides for release in vivo.

The present disclosure also provides methods for enhancing proliferation of a CAR-T cell, said methods involving contacting the CAR-T cell with a CAR-antigen presenting synthetic particle. The synthetic particles may be polymeric particles, magnetic particles, or liposomes.

Further, the present disclosure provides biomolecule-coated films, methods for using the same, and methods for making the same.

### BRIEF DESCRIPTION OF THE DRAWINGS

The invention may be best understood from the following detailed description when read in conjunction with the accompanying drawings. Included in the drawings are the following figures:
**FIG. 1** illustrates multi-functionalization of PLGA microparticles using DNA scaffolds.
**FIG. 2** shows activation of engineered circuit human primary synNotch CAR-T cells through biodegradable polymeric microparticles for gated antigen-specific cancer targeting.
**FIG. 3** illustrates multi-functionalization of PLGA microparticles and their impact on primary synNotch T cell activity.
**FIG. 4** shows stability of DNA scaffold on PLGA microparticles.
**FIG. 5** provides a schematic for local activation of synNotch CAR T cells for HER2-specific antigen targeting by intratumoral injection of PLGA particles.
**FIG. 6** illustrates a method for covalently attaching DNA to a block-co-polymer PLGA-PEG-MAL for generating a nucleic acid-polymer conjugate.
**FIGS. 7A**-K. AICE with ratiometrically controlled moieties for human primary T lymphocytes *ex vivo* expansion.
**FIGS. 8A-8F****.** Selective tumor killing *in vivo* by local activation of synNotch CAR-T cells using AICE particles.
**FIG. 9** illustrates the density dependent activation of synNotch receptor for cytokine release.
**FIG. 10** shows the serum cytokine and chemokine quantification after administration of DNA-scaffolded particle constructs.
**FIG. 11** provides a schematic for expansion of CAR-T cells by contacting the CAR-T cells with CAR-antigen presenting particles (CAPP).
**FIG. 12** shows activation of CAR-T cells through CAPP presenting different antigens.
**FIG. 13** illustrates the ability of CAPP to induce cell expansion across a range of particle sizes and antigen densities.
**FIG. 14** shows CAR-antigen presentation on magnetic beads and liposomes were also able to induce CAR-T cell expansion.
**FIG. 15** illustrates CAPP-mediated cell proliferation using polymeric particles without inducing cytokine production and without cell exhaustion.
**FIG. 16** shows CAR-antigen presentation on magnetic beads and liposomes results in a similar CAR-T cell exhaustion profile (A) and cytokine release (B) as those CAR-T cells activated by polymeric particles (CAPP-EGFR).
**FIGS. 17A-17D** demonstrate the inclusion of DNA scaffolds in a porous film device.

### DETAILED DESCRIPTION

The present disclosure provides polymeric particles comprising biomolecules of interest attached thereto, methods for using the same, and methods for making the same. The polymeric particles can be loaded with multiple different biomolecules of interest in a desired ratio for co-presentation, for example.

The present disclosure also provides methods for enhancing proliferation of a CAR-T cell, said methods involving contacting the CAR-T cell with a CAR-antigen presenting synthetic particle. The methods can enhance proliferation of a CAR-T cell without significant increase in cytokine production or CAR-T cell exhaustion, for example.

Further, the present disclosure provides biomolecule-coated films, methods for using the same, and methods for making the same. The films can be loaded with multiple different biomolecules of interest and can be used in various biomedical applications such as adoptive cell transplantation, for example.

Before exemplary embodiments of the present invention are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials may now be described. Any and all publications mentioned herein are incorporated herein by reference to disclose and describe the methods and/or materials in connection with which the publications are cited. It is understood that the present disclosure supersedes any disclosure of an incorporated publication to the extent there is a contradiction.

It must be noted that as used herein and in the appended claims, the singular forms "a", "an", and "the" include plural referents unless the context clearly dictates otherwise. Thus, for example, reference to "a first nucleic acid" includes a plurality of such first nucleic acid and reference to "the nucleic acid" includes reference to one or more nucleic acids, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. To the extent such publications may set out definitions of a term that conflicts with the explicit or implicit definition of the present disclosure, the definition of the present disclosure controls.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

### DEFINITIONS

The term "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the term "nucleotide" includes those moieties that contain hapten or fluorescent labels and may contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, are functionalized as ethers, amines, or the likes.

The term "nucleic acid" refers to a polymer of a length greater than about 5 bases, greater than about 10 bases, greater than about 15 bases, or greater than about 20 bases. A nucleic acid may be 5-200 bases in length, e.g., 5-50 bases, 50-200 bases, 10-80 bases, 10-50 bases, 10-30 bases, 10-20 bases, or 12-20 bases in length. The term "nucleic acid" refers to a polymer of nucleotides, e.g., deoxyribonucleotides, ribonucleotides, or peptide nucleic acid (PNA) and may be produced enzymatically or synthetically (e.g., PNA as described in U.S. Patent No. 5,948,902) which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine and thymine (G, C, A and T, respectively). The nucleic acid can be single stranded or double stranded.

The term "hybridization" refers to the specific binding of a nucleic acid to a complementary nucleic acid via Watson-Crick base pairing.

The term "hybridization conditions" as used herein refers to conditions that allow hybridization of a nucleic acid to a complementary nucleic acid, e.g., a nucleic acid immobilized on a polymeric particle may specifically bind to a complementary nucleic acid via Watson-Crick base pairing under hybridization conditions.

The terms "antibodies" and "immunoglobulin" include antibodies or immunoglobulins of any isotype, fragments of antibodies which retain specific binding to antigen, including, but not limited to, Fab, Fv, scFv, and Fd fragments, chimeric antibodies, humanized antibodies, single-chain antibodies, and fusion proteins comprising an antigen-binding portion of an antibody and a non-antibody protein.

"Antibody fragments" comprise a portion of an intact antibody, for example, the antigen binding or variable region of the intact antibody. Examples of antibody fragments include Fab, Fab', F(ab')₂, and Fv fragments; diabodies; linear antibodies; single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Single-chain Fv" or "sFv" antibody fragments comprise the V_{H} and V_{L} domains of antibody, wherein these domains are present in a single polypeptide chain. In some embodiments, the Fv polypeptide further comprises a polypeptide linker between the V_{H} and V_{L} domains, which enables the sFv to form the desired structure for antigen binding.

The term "binding" refers to a direct association between two molecules, due to, for example, covalent, electrostatic, hydrophobic, and ionic and/or hydrogen-bond interactions, including interactions such as salt bridges and water bridges. An antibody that binds "specifically" to an epitope within a particular polypeptide binds with an affinity of 10⁻⁷ M or greater, e.g., binding with an affinity of 10⁻⁸ M, 10⁻⁹ M, 10⁻¹⁰ M, etc. Non-specific binding would refer to binding with an affinity of less than about 10⁻⁷ M, e.g., binding with an affinity of 10⁻⁶ M, 10⁻⁵ M, 10⁻⁴ M, etc.

The terms "patient" or "subject" are used interchangeably to refer to a human or a non-human animal (e.g., a mammal).

The terms "treat", "treating", treatment," "prevent," "preventing," and the like refer to a course of action (such as administering an agent or a pharmaceutical composition comprising an agent) initiated after a disease, disorder or condition, or a symptom thereof, has been diagnosed, observed, and the like so as to eliminate, reduce, suppress, mitigate, or ameliorate, either temporarily or permanently, at least one of the underlying causes of a disease, disorder, or condition afflicting a subject, or at least one of the symptoms associated with a disease, disorder, or condition afflicting a subject. Thus, treatment includes inhibiting (i.e., arresting the development or further development of the disease, disorder or condition or clinical symptoms association therewith) an active disease.

The term "in need of treatment" as used herein refers to a judgment made by a physician or other caregiver that a subject requires or will benefit from treatment. This judgment is made based on a variety of factors that are in the realm of the physician's or caregiver's expertise.

The phrase "therapeutically effective amount" refers to the administration of an agent to a subject, either alone or as a part of a pharmaceutical composition and either in a single dose or as part of a series of doses, in an amount that is capable of having any detectable, positive effect on any symptom, aspect, or characteristics of a disease, disorder or condition when administered to a patient. The therapeutically effective amount can be ascertained by measuring relevant physiological effects.

The terms "polypeptide," "peptide," and "protein", used interchangeably herein, refer to a polymeric form of amino acids of any length, which can include genetically coded and non-genetically coded amino acids, chemically or biochemically modified or derivatized amino acids, and polypeptides having modified polypeptide backbones. The terms include fusion proteins, including, but not limited to, fusion proteins with a heterologous amino acid sequence, fusion proteins with heterologous and homologous leader sequences, with or without N-terminus methionine residues; immunologically tagged proteins; and the like.

### POLYMERIC PARTICLES

Polymeric particles functionalized by attaching one or more biomolecules of interest are provided. In certain embodiments, polymeric particles functionalized by attaching two or more biomolecules of interest, where the ratio number of each of the two or more biomolecules is controlled, are provided. In certain embodiments, polymeric particles functionalized by attaching three or more biomolecules of interest, where the ratio number of each of the three or more biomolecules is controlled, are provided. In certain embodiments, polymeric particles functionalized by attaching one or more biomolecules of interest, where the density of each is controlled, are provided. In certain embodiments, polymeric particles may encapsulate biomolecules in addition to having one or more biomolecules presented on the surface of the polymeric particles. As used herein, the term "biomolecule" refers to an organic molecule having an activity in a biological system in vivo or in vitro. Examples of biomolecules includes nucleic acid (e.g., DNA or RNA), peptides, and proteins. The term biomolecule encompasses members of a specific-binding pair, such as, an antibody, an antigen, a ligand, a receptor, an enzyme, a substrate, and the like.

In certain embodiments, a polymeric particle may include a polymeric core; a nucleic acid-polymer conjugate comprising a first single stranded nucleic acid covalently attached to a polymer, where the polymer is non-covalently associated with the polymeric core thereby presenting the first single stranded nucleic acid on a surface of the polymeric core; and a first binding member-nucleic acid conjugate comprising a second single stranded nucleic acid covalently associated with the first binding member, where the second single stranded nucleic acid is complementary to the first single stranded nucleic acid and is associated with the first single stranded nucleic acid via hybridization thereby presenting the first binding member on a surface of the polymeric particle, where the first binding member is a member of a specific-binding pair, where the first binding member specifically binds to a second binding member that is a member of the specific-binding pair.

The nucleic acid-polymer conjugate may be formed using the same polymer as that used to form the polymeric core or by using a different polymer. In certain embodiments, the polymer of the nucleic acid-polymer conjugate may associate non-covalently with the polymer or polymers to form the polymeric core such that the nucleic acid-polymer conjugate acts as a surfactant to produce the particles where the hydrophilic nucleic acid is displayed on the outside of the particles while the hydrophobic polymer in the conjugate is inside the particle. Thus, the nucleic acid-polymer conjugate serves as a surfactant for formation of particles from a mixture of nucleic acid-polymer conjugate and polymers.

The nucleic acid in the nucleic acid-polymer conjugate may be a single stranded nucleic acid composed of deoxyribonucleotides or ribonucleotides of a length of at least 5 bases and up to 200 bases. In certain embodiments, the nucleic acid may be 5-100 bases in length. In certain embodiments, the nucleic acid may be 10-25 bases in length and may be a polymer of deoxyribonucleotides. The nucleic acid may be conjugated to the polymer via a covalent bond. For example, the nucleic acid may be modified on its 3'-end or 5'-end by attachment of a reactive group which may react with a suitably modified polymer to form a covalent bond between the nucleic acid and the polymer. In certain embodiments, the nucleic acid may be modified to include a thiol group at the 3'-end and the polymer may be modified to include a maleimide group which may react to covalently attach the nucleic acid to the polymer.

In certain embodiments, the polymer used to form the polymeric particle may be any polymer, e.g. a biodegradable and biocompatible polymer. In certain cases, the polymer may be polylactic acid (PLA), polyglycolic acid (PGA), polyethylene glycol (PEG), and/or poly(e-caprolactone) (PCL). In another aspect, the polymer may be a copolymer such as poly(lactide-co-glycolide) (PLGA) or poly(lactide-co-glycolide) poly(e-caprolactone) (PLGA/PCL). embodiments, the polymer used to form the polymeric particle may be a combination of one or more of PLA, PGA, PEG, PCL, PLGA, and PLGA/PCL. In certain embodiments, the polymeric core of the polymeric particle comprises PLGA and PLA. In certain cases, the polymeric core of the polymeric particle comprises PLGA, PEG and PLA.

In certain cases, the polymer in the nucleic acid-polymer conjugate may be a block polymer. In certain embodiments, the polymer in the nucleic acid-polymer conjugate may be a block copolymer. In certain embodiments, the polymer in the nucleic acid-polymer conjugate may be a block copolymer of PLGA and PEG (PLGA-block-PEG). In certain embodiments, the polymer in the nucleic acid-polymer conjugate may a block copolymer of PLA and PEG (PLA-block-PEG). In certain cases, the PLGA in the block copolymer may range in molecular weight from 8 kDa-30 kDa (e.g. 10 kDa) and that of the PEG may range from 3 kDa-7 kDa (e.g. 5 kDa). In certain embodiments, the polymer in the nucleic acid-polymer conjugate may be selected such that the length of the polymer is sufficient to be inserted into the polymer core for stable association with the polymers in the core. In some cases, the polymer in the nucleic acid-polymer conjugate is non-covalently associated with the polymers forming the core of the particle. Such a non-covalent association may be via a hydrophobic interaction or via van der Walls forces. In some cases, the polymer in the nucleic acid-polymer conjugate is covalently associated with the polymers forming the core of the particle, e.g., via polymerization.

The formation of the core of the polymeric particle and association with a nucleic acid-polymer conjugate may occur simultaneously such that a plurality of hydrophobic polymers may associate with each other and away from a hydrophilic environment and may form particles that are surrounded by the nucleic acid-polymer conjugates which form an interface between the hydrophobic core and the hydrophilic environment with the polymer part of the nucleic acid-polymer conjugates interacting with the core and the nucleic acid part of the nucleic acid-polymer conjugates interacting with the hydrophilic environment. The polymers in the core may polymerize to form a stable particle which may be associated covalently (e.g., polymerized) or non-covalently (e.g., hydrophobic interaction) with the polymer in the nucleic acid-polymer conjugates.

The term particle or polymeric particle are used interchangeably to refer to one or a plurality of such particles. The polymeric particles may be substantially spherical, such as, spherical, oval, semi-spherical, hemispherical, an irregular sphere with flattened sections or concave or convex sections, semi-oval, an irregular oval with flattened sections or concave or convex sections.

The diameter of the particle refers to length from one end of the particle to the diametrically opposite end and may range from nanometers to micrometers. The size of the particles may be controlled by the amount and/or molecular weight of the polymer(s) used to form the particle. In some embodiments, the diameter of the particles may range from 1-50 µm, e.g., 1-40 µm, 1-30 µm, 1-20 µm, 1-5 µm, 1-4 µm, 1-3 µm, or 1-2 µm. In particular embodiments, the diameter of the particles range from 1-2 µm. In some embodiments, the diameter of the particles may range from 50-1000 nm, e.g., 50-1000 nm, 50-800 nm, 50-500 nm, or 100-500 nm.

Polymeric particles described herein may be made from biodegradable and substantially non-toxic polymers. The term "biodegradable polymer" refers to a polymer or polymers which degrade in vivo. A biodegradable polymer may be a homopolymer, a copolymer, or a polymer comprising more than two different polymeric units. The polymeric particles may be substantially degraded 100 days, 30 days, 10 days, or 3 days, e.g., 3-100 days, 3-10 days, 3-4 days, after being administered to a subject in need thereof. In certain embodiments, the biodegradability of the particles may be tuned based on the desired residence time in vivo. For example, the molecular weight of the polymer used to form the particles may be selected based on the desired in vivo half-life. A lower molecular weight polymer may be selected for forming particles having a lower in vivo half-life and vice versa.

In certain embodiments, the molecular weight of polymers used for generating the particles may be less than 100 kDa, e.g., less than 90 kDa, less than 80 kDa, less than 70 kDa, less than 60 kDa, less than 50 kDa, such as, 5-100 kDa, 15-90 kDa, 20-80 kDa, 30-70 kDa, 30-60 kDa, or 30-50 kDa.

In certain embodiments, the molecular weight of polymers used to form the single stranded nucleic acid-polymer conjugate may be different from the molecular weight of polymers used to form the core of the particle. For example, the molecular weight of polymers used for generating the particles may be less than 100 kDa, e.g., less than 90 kDa, less than 80 kDa, less than 70 kDa, less than 60 kDa, less than 50 kDa, such as, 5-100 kDa, 15-90 kDa, 20-80 kDa, 30-70 kDa, 30-60 kDa, or 30-50 kDa and the molecular weight of the polymer used for forming the single stranded nucleic acid-polymer conjugate may be less than 30 kDa, e.g., less than 20 kDa, less than 15 kDa, or less than 10 kDa, such as, 1 kDa-30 kDa, 3 kDa-20 kDa, 5 kDa-20 kDa, 5 kDa-15 kDa, 5 kDa-10 kDa. In certain embodiments, the polymer used for generating the single stranded nucleic acid-polymer conjugate may be a copolymer, where one of the polymers may have a molecular weight from 10 kDa-30 kDa and the other polymer may have a molecular weight from 3 kDa- 8 kDa.

The first binding member-nucleic acid conjugate may include a second single stranded nucleic acid covalently attached with the first binding member. The second single stranded nucleic acid may be composed of deoxyribonucleotides or ribonucleotides or peptide nucleic acid and may have a length of at least 5 bases and up to 200 bases. In certain embodiments, the nucleic acid may be 5-100 bases in length. In certain embodiments, the nucleic acid may be 10-25 bases in length. In certain embodiments, the nucleic acid may be 10-25 bases or 10-20 bases in length and may be a polymer of deoxyribonucleotides. The first and second single stranded nucleic acids may include a contiguous stretch of at least 4 bases that are complementary to each other. The first and second single stranded nucleic acids may include a plurality of contiguous stretches of at least 4 bases that are complementary to each other, which contiguous stretches are separated by bases that are not complementary to each other. In certain embodiments, the region of complementarily may be about 7-20 bases, 7-19 bases, 7-18 bases, 7-10 bases, or 4-10 bases. In certain embodiments, the contiguous stretch of bases may be less than 100% complementary. For example, in a stretch of at least 7 bases, there may be at least a 99% complementary, 98% complementary, 97% complementary, 96% complementary, or 95% complementarity. In certain embodiments, the region of complementarily may be about 7-20 bases, 7-19 bases, 7-18 bases, 7-10 bases, or 4-7 bases, where all of the bases are complementary.

The nucleic acid may be conjugated to the first binding member in the first binding member-nucleic acid conjugate via a covalent bond directly or indirectly. In certain embodiments, the second single stranded nucleic acid is covalently attached to the first binding member via a reactive group. For example, the second single stranded nucleic acid may be modified to include an amino group at the 3'-end and the first binding member may be modified to include a carboxyl group which may react to covalently attach the nucleic acid to the first binding member or the second single stranded nucleic acid may be modified to include a thiol group at the 3'-end and the first binding member may be modified to include a maleimide group which may react to covalently attach the nucleic acid to the first binding member. In some embodiments, covalent attachment between the second single stranded nucleic acid and the first binding member may be achieved indirectly via a linker. For example, the second single stranded nucleic acid may be modified to include an amino group at the 3'-end which reacts with a carboxyl group in the linker, the linker may further include a maleimide group to react with a thiol group in the first binding member.

In certain embodiments, the first binding member and the second binding member may be a member of a specific-binding pair that includes antibody-antigen, receptor-ligand, and the like. In certain embodiments, the first binding member may be an antigen and the second binding member may be an antibody. In certain embodiments, the first binding member may be an antibody and the second binding member may be an antigen.

In certain embodiments, the second binding member may be present on the surface of a cell, such as, a cancer cell, a stem cell, or an immune cell. Examples of immune cells include T-cells (such as a CD4+ T cell, a CD8+ T cell or a regulatory T cell), natural killer (NK) cells, dendritic cells, macrophages, neutrophils, myeloid immune cells and B-cells. In certain embodiments, the second binding member may be present on the surface of a T-cell. In certain embodiments, the second binding member may be soluble, for example, may be present in an extracellular matrix in a tissue or in blood of a subject.

In certain embodiments, the second binding member may be present on the surface of a cell that has been genetically engineered, for example a T-cell that has been genetically engineered. By "genetically engineered" it is intended to mean that the genome of the cell has been manipulated to express an expression product that is not normally naturally expressed by the cell. Examples of cells that have been genetically engineered include chimeric antigen receptor (CAR)-T cells, that are T-cell that have been genetically engineered to express a CAR, and cells that are engineered to express a binding-triggered transcriptional switch such as a synNotch receptor.

By "binding-triggered transcriptional switch" or "BTSS" it is intended to mean a synthetic modular polypeptide or system of interacting polypeptides having an extracellular domain that includes a second member of a specific binding pair that binds a first member of the specific binding pair, e.g., an antigen), a binding-transducer and an intracellular domain. Upon binding of the first member of the specific binding pair to the BTTS the binding signal is transduced to the intracellular domain such that the intracellular domain becomes activated and performs a function, e.g., transcription activation, within the cell that it does not perform in the absence of the binding signal.

Examples of BTSS include the synNotch system, the MESA system, the TANGO system, the A2 Notch system, etc. The synNotch receptor may be for example as described in U.S. Patent No. 9,670,281 and described in more detail below. The MESA system may be as described in WO 2018/081039 A1 and comprises a self-containing sensing and signal transduction system, such that binding of a ligand (first member of the specific binding pair) to the receptor (second member of the specific binding pair) induces signaling to regulate expression of a target gene. In the MESA system, binding of the ligand to the receptor induces dimerization that results in proteolytic trans-cleavage of the system to release a transcriptional activator previously sequestered at the plasma membrane. The TANGO system may be as described in Barnea et al., 2008 Proc. Natl. Acad. Sci. U.S.A., 105(1): 64-9. Briefly, the TANGO system sequesters a transcription factor to the cell membrane by physically linking it to a membrane-bound receptor (e.g., GPCRs, receptor kinases, Notch, steroid hormone receptors, etc.). Activation of the receptor fusion results in the recruitment of a signaling protein fused to a protease that then cleaves and releases the transcription factor to activate genes in the cell. The A2 Notch system may be as described in WO 2019099689 A1. Briefly, the A2 Notch system incorporates a force sensor cleavage domain which, upon cleavage induced upon binding of a ligand to the receptor, releases the intracellular domain into the cell.

In certain embodiments, the second binding member may be present on the surface of a genetically engineered cell, such as, a cell expressing a BTTS and a CAR under the control of the BTTS. In certain embodiments, the second binding member may be present on the surface of a genetically engineered cell, such as, a cell expressing the BTTS and a CAR under control of the BTTS.

In certain cases, the first binding member may bind to a synNotch receptor as described in U.S. Patent No. 9,670,281. For example, the synNotch receptor may include an extracellular domain that includes the second binding member, where the second binding member is a single-chain Fv (scFv) or a nanobody and the first binding member present on the particles is an antigen to which the single-chain Fv (scFv) or a nanobody binds. In certain cases, the second binding member may be an anti-CD19, anti-mesothelin, anti-GFP antibody, scFv, or a nanobody and the first binding member may be CD19, mesothelin, GFP, respectively.

In certain embodiments, the first binding member comprises interleukin-2 (IL-2) and the second binding member may be a receptor that specifically binds to the IL-2 presented on the surface of the polymeric particle. The IL-2 may be human IL-2 and may have the amino acid sequence set forth in UniProt accession number Q0GK43-1 (version 1, last modified October 3, 2006). In certain embodiments, the first binding member may comprise an anti-IL-2 antibody which specifically binds to IL-2 in order to present IL-2 on a surface of the polymeric particle. In certain, exemplary embodiments, the anti-IL-2 antibody is antibody clone #5355, available for example from ThermoFisher #MA523696.

In certain cases, the particle may be functionalized by attaching more than one member of a specific-binding pair. For example, a particle of the present disclosure may include a first biomolecule and a second biomolecule. The first biomolecule may be a member of a first specific-binding pair and the second biomolecule may be a member of a second specific-binding pair. In yet other embodiments, the particle may be functionalized by attaching a biomolecule that is a therapeutic agent. While the therapeutic agent can be administered in a free form, administering the therapeutic agent in an immobilized form may increase its in vivo half-life. In other embodiments, the particles may include a first binding member that targets the particles to a particular tissue or cells and the particles may include a therapeutic agent that is released in the target tissue or cell.

In certain embodiments, a therapeutic antibody may be attached to the particles, where the therapeutic antibody is an antibody that inhibits cell signaling from a receptor (e.g., HER2 receptor) or activates cell signaling from a receptor. In certain embodiments, a therapeutic antibody may be attached to the particles, where the therapeutic antibody is an antibody that binds and sequesters a soluble molecule, such as, cytokines or antibodies. In certain embodiments, the first binding member on the particles may be one or more of infliximab, adalimumab and certolizumab (anti-TNFα), bevacizumab (anti-vascular endothelial growth factor), cetuximab and panitumumab [anti-EGFR (epidermal growth factor receptor) or HER1 (human epidermal growth factor receptor)] or trastuzumab (anti-HER2).

In certain cases, a first binding member of a first specific-binding pair and a first binding member of a second specific-binding pair may be attached to the polymeric particles. Use of single stranded nucleic acid to attach the binding members to the particles allows for controlling the number of binding members attached to the particles. For example, to obtain a 50:50 ratio, the same amounts of a first nucleic acid-polymer conjugate and a second nucleic acid-polymer conjugate may be used. Using a ratio of 10:1 of the first nucleic acid-polymer conjugate to the second nucleic acid-polymer conjugate provide particles in which 10 times of a biomolecule attached to the first nucleic acid-polymer compared to the biomolecule attached to the second nucleic acid-polymer is present. For example, the polymer particles may include on their surface an antigen that bind to a chimeric Notch polypeptide expressed on the surface of a CAR-T which when bound to the antigen results in expression of a cancer associated CAR on the cell surface and the polymeric particles may further include an antigen that binds the cancer associated CAR, where binding of the antigen on the particle to the cancer associated CAR results in activation of the T cell in absence of significant expression of cytokines.

In certain embodiments, one or more antibodies may be attached to the particles, where the antibodies bind to binding members located on the surface of an immune cell, for example a T-cell. In certain embodiments, the first binding member of the first specific binding pair on the polymeric particles is an antibody that binds to a second binding member of the first specific binding pair, and the second binding member is an antibody that binds to a second binding member of the second specific binding pair, wherein the second binding members of the first and second specific binding pair are both expressed on the surface of a T-cell. In certain embodiments, one of the second binding members expressed on the surface of the T-cell is CD3 and the other one is CD28. In certain embodiments, the first binding member that binds CD3 (anti-CD3) and the first binding member that binds CD28 (anti-CD28) are present at a ratio of 1:5 to 5:1, a ratio of 1:4 to 5:1, a ratio of 1:3 to 5:1, a ratio of 1:2 to 5:1, a ratio of 1:1 to 5:1, a ratio of 1:5 to 4:1, a ratio of 1:4 to 4:1, a ratio of 1:3 to 4:1, a ratio of 1:2 to 4:1, a ratio of 1:1 to 4:1, a ratio of 2:1 to 5:1, a ratio of 2:1 to 4:1. In certain, exemplary embodiments the ratio of anti-CD3 antibodies to anti-CD28 antibodies is 3:1.

In certain embodiments, the particles may be attached to a self-peptide in order to reduce or avoid an immune response to the particles when administered to a subject. For example, the particles may be attached to self -peptides which enable recognition of cells by phagocytes as endogenous cells that are not phagocytosed. In certain example, the self-peptide may be a human CD47 peptide, such as, those disclosed in Science. 2013 Feb 22; 339 (6122): 971-5.

In certain embodiments, the particle includes a (i) first nucleic acid-polymer conjugate comprising a first single stranded nucleic acid covalently attached to the polymer, wherein the polymer is non-covalently associated with the polymeric core thereby presenting the first single stranded nucleic acid on the surface of the polymeric core and a first binding member-nucleic acid conjugate comprising a second single stranded nucleic acid covalently attached with the first binding member, wherein the second single stranded nucleic acid is complementary to the first single stranded nucleic acid and is associated with the first single stranded nucleic acid via hybridization thereby presenting the first binding member on a surface of the polymeric particle; and (ii) a second nucleic acid-polymer conjugate comprising a third single stranded nucleic acid covalently attached to the polymer, wherein the polymer is non-covalently associated with the polymeric core thereby presenting the third single stranded nucleic acid on the surface of the polymeric core and a second first binding member-nucleic acid conjugate comprising a fourth single stranded nucleic acid covalently attached to the first binding member of a second specific-binding pair, wherein the fourth single stranded nucleic acid is complementary to the third single stranded nucleic acid and is associated with the third single stranded nucleic acid via hybridization thereby presenting the first binding member of the second specific-binding pair on a surface of the polymeric particle. As noted herein, the ratio of the first and second nucleic acid-polymer conjugates may be varied based in the amount of the binding members desired to be presented by the particles. In certain cases, the ratio of the first and second nucleic acid-polymer conjugates may be 100:1, 50:1, 10:1, 5:1, 3:1, 2:1, 1:1, 1:2, 1:3, 1:5, 1:10, 1:50, or 1:100. In certain cases, the ratio of the first and second nucleic acid-polymer conjugates may be 10:1, 5:1, 3:1, 2:1, 1:1, 1:2, 1:3, 1:5, or 1:10.

The sequences of the nucleic acids may be selected such that only complementary sequences hybridize and non-complementary sequences do not substantially hybridize. For example, only sequences that have a complementarity of at least 95% or more hybridize. Thus, the sequence of the first single stranded nucleic acid is substantially different from that of the third single stranded nucleic acid such that the second single stranded nucleic acid hybridizes only to the first single stranded nucleic acid and the fourth single stranded nucleic acid specifically hybridizes to the third single stranded nucleic acid.

In certain embodiments, the polymeric particles provided herein may additionally include biomolecules encapsulated in the polymeric core. For example, the polymeric particles may encapsulate one or more of a nucleic acid, peptide, or polypeptide. Such polymeric particles may be utilized for release of the encapsulated biomolecules over a prolonged period in vivo.

The present disclosure also provides a composition comprising the polymeric particles disclosed herein and a pharmaceutically acceptable excipient.

The pharmaceutical compositions of the present disclosure can be formulated to be compatible with the intended method or route of administration; exemplary routes of administration are set forth herein. Furthermore, the pharmaceutical compositions may be used in combination with other therapeutically active agents or compounds to treat or prevent the diseases, disorders and conditions as contemplated by the present disclosure. Suitable pharmaceutically acceptable or physiologically acceptable diluents, carriers or excipients include, but are not limited to, antioxidants (e.g., ascorbic acid and sodium bisulfate), preservatives (e.g., benzyl alcohol, methyl parabens, ethyl or n-propyl, p-hydroxybenzoate), emulsifying agents, suspending agents, dispersing agents, solvents, fillers, bulking agents, detergents, buffers, vehicles, diluents, and/or adjuvants. For example, a suitable vehicle may be physiological saline solution or citrate buffered saline, possibly supplemented with other materials common in pharmaceutical compositions for parenteral administration. Neutral buffered saline or saline mixed with serum albumin are further exemplary vehicles. Typical buffers include, but are not limited to, pharmaceutically acceptable weak acids, weak bases, or mixtures thereof. As an example, the buffer components can be water soluble materials such as phosphoric acid, tartaric acids, lactic acid, succinic acid, citric acid, acetic acid, ascorbic acid, aspartic acid, glutamic acid, and salts thereof. Acceptable buffering agents include, for example, a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), and N-tris[Hydroxymethyl]methyl-3-aminopropanesulfonic acid (TAPS).

In addition, the present disclosure also provides kits that include the polymeric particles disclosed herein and a cell comprising a BTTS, wherein the BTTS comprises: a) an extracellular domain comprising the second member of the specific-binding pair that specifically binds to the first member of the specific-binding pair; b) a binding-transducer; and c) an intracellular domain comprising a transcriptional activator, wherein binding of the first member of the specific-binding pair to the second member of the specific-binding pair activates the intracellular domain; and a transcriptional control element, responsive to the transcriptional activator, operably linked to a nucleotide sequence encoding a chimeric antigen receptor (CAR). In certain embodiments, the BTTS is a chimeric Notch polypeptide comprising, from N-terminus to C-terminus and in covalent linkage: a) an extracellular domain comprising the second member of the specific-binding pair that is not naturally present in a Notch receptor polypeptide and that specifically binds to the first member of the specific-binding pair; b) a Notch regulatory region comprising a Lin 12-Notch repeat, an S2 proteolytic cleavage site, and a transmembrane domain comprising an S3 proteolytic cleavage site; c) an intracellular domain comprising a transcriptional activator or a transcriptional repressor that is heterologous to the Notch regulatory region and replaces a naturally-occurring intracellular Notch domain, wherein binding of the first member of the specific-binding pair to the second member of the specific-binding pair induces cleavage at the S2 and S3 proteolytic cleavage sites, thereby releasing the intracellular domain; and a transcriptional control element, responsive to the transcriptional activator, operably linked to a nucleotide sequence encoding a chimeric antigen receptor (CAR). In certain cases, the cell may be a T-cell, such as, those described in U.S. Patent No. 9,670,281, which is herein incorporated by reference.

### METHODS OF USE

The present disclosure provides methods for using the polymeric particles disclosed herein. The polymeric particles may be used in a variety of in vitro, ex vivo and in vivo methods.

In certain embodiments, the particles may be used in in vivo methods for therapeutic use. For example, the particles may be used to administer therapeutic agents, such as, peptides or proteins such as antibodies. As used herein, the term "peptide" refers to a relatively short chain of amino acids while the term "polypeptide" and "protein" are used interchangeably to refer to longer chains of amino acids, such as, those longer than 50 amino acids. In some cases, the particles may be used to provide a first binding member of a specific-binding pair to a cell expressing a second member of the specific-binding pair by contacting the cells with the particles. In some cases, the particles may be used to provide a first binding member of a specific-binding pair to a subject expressing a second member of the specific-binding pair in a soluble form, e.g., cytokines, secreted antibodies (e.g., IgE antibodies). In some cases, the particles may be used to provide the first binding member to a target organ, tissue, or cell. In some cases, the particles may be used to deliver biomolecules that need to be released over a period of days to weeks. For example, therapeutic biomolecules such as nucleic acid, peptide, and/or polypeptide may be encapsulated in the particles and released over a period of days to weeks as the polymer is dissolved, e.g., by hydrolysis. Also provided are polymeric particles for use in the in vivo methods of therapeutic use described herein.

The particles may be used to provide an antibody to a subject in need thereof. For example, the first binding member of a specific-binding pair may be an antibody such as, Natalizumab (targeting a4 subunit of α4β1 and a47 integrins (as used in the treatment of MS and Crohn's disease); Vedolizumab targeting α4β7 integrin (as used in the treatment of UC and Crohn's disease); Belimumab targeting BAFF (as used in the treatment of SLE); Atacicept (TACI-Ig; Merck/Serono) targeting BAFF and APRIL (as used in the treatment of SLE); Alefacept targeting CD2 (as used in the treatment of Plaque psoriasis, GVHD); Otelixizumab targeting CD3 (as used in the treatment of TID); Teplizumab targeting CD3 (as used in the treatment of TID); Rituximab targeting CD20 (as used in the treatment of Non-Hodgkin's lymphoma, RA (in patients with inadequate responses to TNF blockade) and CLL); Ofatumumab targeting CD20 (as used in the treatment of CLL, RA); Ocrelizumab targeting CD20 (as used in the treatment of RA and SLE); Epratuzumab targeting CD22 (as used in the treatment of SLE and non-Hodgkin's lymphoma); Alemtuzumab targeting CD52 (as used in the treatment of CLL, MS); Abatacept targeting CD80 and CD86 (as used in the treatment of RA and JIA, UC and Crohn's disease, SLE); Eculizumab targeting C5 complement protein (as used in the treatment of Paroxysmal nocturnal haemoglobinuria); Omalizumab targeting IgE (as used in the treatment of Moderate to severe persistent allergic asthma); Canakinumab targeting IL-Iβ (as used in the treatment of Cryopyrin-associated periodic syndromes, Systemic JIA, neonatal-onset multisystem inflammatory disease and acute gout); Mepolizumab targeting IL-5 (as used in the treatment of Hyper-eosinophilic syndrome); Reslizumab targeting IL-5 (as used in the treatment of Eosinophilic oesophagitis); Tocilizumab targeting IL-6R (as used in the treatment of RA, JIA); Ustekinumab targeting IL-12 and IL-23 (as used in the treatment of Plaque psoriasis, Psoriatic arthritis, Crohn's disease); Briakinumab targeting IL-12 and IL-23 (as used in the treatment of Psoriasis and plaque psoriasis); Etanercept targeting TNF (as used in the treatment of RA, JIA, psoriatic arthritis, AS and plaque psoriasis); Infliximab targeting TNF (as used in the treatment of Crohn's disease, RA, psoriatic arthritis, UC, AS and plaque psoriasis); Adalimumab targeting TNF (as used in the treatment of RA, JIA, psoriatic arthritis, Crohn's disease, AS and plaque psoriasis); Certolizumab pegol targeting TNF (as used in the treatment of Crohn's disease and RA); Golimumab targeting TNF (as used in the treatment of RA, psoriatic arthritis and AS); and the like.

In some cases, the antibody on the polymeric particle, or encapsulated by the polymeric particle, or expressed by the cell in response to induction by the intracellular domain of a synNotch polypeptide of the present disclosure is a therapeutic antibody for the treatment of cancer. Such antibodies include, e.g., Ipilimumab targeting CTLA-4 (as used in the treatment of Melanoma, Prostate Cancer, RCC); Tremelimumab targeting CTLA-4 (as used in the treatment of CRC, Gastric, Melanoma, NSCLC); Nivolumab targeting PD-1 (as used in the treatment of Melanoma, NSCLC, RCC); MK-3475 targeting PD-1 (as used in the treatment of Melanoma); Pidilizumab targeting PD-1 (as used in the treatment of Hematologic Malignancies); BMS-936559 targeting PD-L1 (as used in the treatment of Melanoma, NSCLC, Ovarian, RCC); MEDI4736 targeting PD-L1 ; MPDL33280A targeting PD-L1 (as used in the treatment of Melanoma); Rituximab targeting CD20 (as used in the treatment of Non-Hodgkin's lymphoma); Ibritumomab tiuxetan and tositumomab (as used in the treatment of Lymphoma); Brentuximab vedotin targeting CD30 (as used in the treatment of Hodgkin's lymphoma); Gemtuzumab ozogamicin targeting CD33 (as used in the treatment of Acute myelogenous leukaemia); Alemtuzumab targeting CD52 (as used in the treatment of Chronic lymphocytic leukaemia); IGN101 and adecatumumab targeting EpCAM (as used in the treatment of Epithelial tumors (breast, colon and lung)); Labetuzumab targeting CEA (as used in the treatment of Breast, colon and lung tumors); huA33 targeting gpA33 (as used in the treatment of Colorectal carcinoma); Pemtumomab and oregovomab targeting Mucins (as used in the treatment of Breast, colon, lung and ovarian tumors); CC49 (minretumomab) targeting TAG-72 (as used in the treatment of Breast, colon and lung tumors); cG250 targeting CAIX (as used in the treatment of Renal cell carcinoma); J591 targeting PSMA (as used in the treatment of Prostate carcinoma); MOvl8 and MORAb-003 (farletuzumab) targeting Folate -binding protein (as used in the treatment of Ovarian tumors); 3F8, chl4.18 and KW-2871 targeting Gangliosides (such as GD2, GD3 and GM2) (as used in the treatment of Neuroectodermal tumors and some epithelial tumors); hu3S193 and IgN311 targeting Le y (as used in the treatment of Breast, colon, lung and prostate tumors); Bevacizumab targeting VEGF (as used in the treatment of Tumor vasculature); IM-2C6 and CDP791 targeting VEGFR (as used in the treatment of Epithelium-derived solid tumors); Etaracizumab targeting Integrin αVβ3 (as used in the treatment of Tumor vasculature); Volociximab targeting Integrin α5β1 (as used in the treatment of Tumor vasculature); Cetuximab, panitumumab, nimotuzumab and 806 targeting EGFR (as used in the treatment of Glioma, lung, breast, colon, and head and neck tumors); Trastuzumab and pertuzumab targeting ERBB2 (as used in the treatment of Breast, colon, lung, ovarian and prostate tumors); MM-121 targeting ERBB3 (as used in the treatment of Breast, colon, lung, ovarian and prostate, tumors); AMG 102, METMAB and SCH 900105 targeting MET (as used in the treatment of Breast, ovary and lung tumors); AVE1642, IMC-A12, MK-0646, R1507 and CP 751871 targeting IGF1R (as used in the treatment of Glioma, lung, breast, head and neck, prostate and thyroid cancer); KB004 and IIIA4 targeting EPHA3 (as used in the treatment of Lung, kidney and colon tumors, melanoma, glioma and haematological malignancies); Mapatumumab (HGS-ETR1) targeting TRAILR1 (as used in the treatment of Colon, lung and pancreas tumors and haematological malignancies); HGS-ETR2 and CS-1008 targeting TRAILR2; Denosumab targeting RANKL (as used in the treatment of Prostate cancer and bone metastases); Sibrotuzumab and F19 targeting FAP (as used in the treatment of Colon, breast, lung, pancreas, and head and neck tumors); 81C6 targeting Tenascin (as used in the treatment of Glioma, breast and prostate tumors); Blinatumomab targeting CD3 (as used in the treatment of ALL); pembrolizumab targeting PD-1 as used in cancer immunotherapy; 9E10 antibody targeting c-Myc; and the like.

The contacting may be performed by administering the particles to a subject in a therapeutically effective amount. The phrase "therapeutically effective amount" refers to the administration of the particles to a subject, either alone or as a part of a pharmaceutical composition and either in a single dose or as part of a series of doses, in an amount that is capable of having any detectable, positive effect on any symptom, aspect, or characteristics of a disease, disorder or condition when administered to a patient. The therapeutically effective amount can be ascertained by measuring relevant physiological effects. For example, in the case of a cancer, a lowering or reduction of size of tumor or other cancer cells can be used to determine whether the amount of the particles is effective to treat the cancer.

The present disclosure contemplates the administration of the disclosed particles, and compositions thereof, in any appropriate manner. Suitable routes of administration include parenteral (e.g., intramuscular, intravenous, subcutaneous (e.g., injection or implant), intraperitoneal, intracisternal, intraarticular, intraperitoneal, intracerebral (intraparenchymal) and intracerebroventricular), oral, nasal, vaginal, sublingual, intraocular, rectal, topical (e.g., transdermal), sublingual, inhalation, local, e.g., injection directly into a target organ or tissue such as a tumor.

The present disclosure contemplates the administration of the disclosed particles, and compositions thereof in combination with a cell, such as a T cell expressing a synthetic notch polypeptide or another CAR-T cell. As used herein, "combination" is meant to include therapies that can be administered separately, for example, formulated separately for separate administration (e.g., as may be provided in a kit), and therapies that can be administered together in a single formulation (i.e., a "co-formulation"). In certain embodiments, particles and cells as disclosed herein are administered sequentially, e.g., where particles are administered prior to or after administering one or more cells. In other embodiments, the particles and the cells are administered simultaneously, e.g., where cells and particles are administered at or about the same time; the particles and cells may be present in two or more separate formulations or combined into a single formulation (i.e., a co-formulation).

In some embodiments, the particles may be used in a method for contacting a cell expressing a second binding member of a specific-binding pair with a first member of the specific-binding pair. The contacting may occur in vitro, ex vivo, or in vivo. In certain embodiments, the cell may be in vivo, e.g., in a subject and the particles may be administered to the subject. In certain embodiments, the cell and the particles may be administered to the subject.

In certain embodiments, the method may include a method of activating a T cell, such as a CAR-T cell, e.g., a CAR-T cell expressing a chimeric Notch polypeptide as described herein. In certain embodiments, the method of the present disclosure may be used for inducing T-cell proliferation without significantly increasing cytokine production by the T cell. For example, the method may include administering a CAR-T cell expressing a chimeric Notch polypeptide and particles having an antigen displayed on the surface, where the antigen binds to the Notch polypeptide and results in expression of a cancer associated CAR on the cell surface. The polymeric particle further includes an antigen that binds the cancer associated CAR, where binding of the antigen on the particle to the cancer associated CAR results in activation of the T cell in absence of significant expression of cytokines. In certain embodiments, the level of cytokines produced by the T cells in absence of the presence of cancer cells expressing the CAR antigen is substantially lower than the level of the cytokines produced by the T cells in presence of cancer cells expressing the CAR antigen. Thus, use of particles functionalized with both a protein that binds to the chimeric Notch polypeptide and the protein that binds to the CAR expressed by the binding to the chimeric Notch polypeptide provides for proliferation of the T-cells while having a substantially lower production of cytokines by the activated T cell.

In certain aspects, contacting a cell expressing a BTTS, e.g. a chimeric Notch receptor polypeptide, as described herein with the particles of the present disclosure may modulate an activity of the cell. In some cases, release of the intracellular domain modulates proliferation of the cell or of cells surrounding the cell. In some cases, release of the intracellular domain modulates apoptosis in the cell or in cells surrounding the cell. In some cases, release of the intracellular domain induces cell death by a mechanism other than apoptosis. In some cases, release of the intracellular domain modulates gene expression in the cell through transcriptional regulation, chromatin regulation, translation, trafficking or post-translational processing. In some cases, release of the intracellular domain modulates differentiation of the cell. In some cases, release of the intracellular domain modulates migration of the cell or of cells surrounding the cell. In some cases, release of the intracellular domain modulates the expression and secretion of a molecule from the cell. In some cases, release of the intracellular domain modulates adhesion of the cell to a second cell or to an extracellular matrix. In some cases, release of the intracellular domain induces de novo expression a gene product in the cell. In some cases, where release of the intracellular domain induces de novo expression a gene product in the cell, the gene product is a transcriptional activator, a transcriptional repressor, a chimeric antigen receptor, a second chimeric Notch receptor polypeptide, a translation regulator, a cytokine, a hormone, a chemokine, or an antibody.

The terms "chimeric antigen receptor" and "CAR", used interchangeably herein, refer to artificial multi-module molecules capable of triggering or inhibiting the activation of an immune cell which generally but not exclusively comprise an extracellular domain (e.g., a ligand/antigen binding domain), a transmembrane domain and one or more intracellular signaling domains. The term CAR is not limited specifically to CAR molecules but also includes CAR variants. CAR variants include split CARs wherein the extracellular portion (e.g., the ligand binding portion) and the intracellular portion (e.g., the intracellular signaling portion) of a CAR are present on two separate molecules. CAR variants also include ON-switch CARs which are conditionally activatable CARs, e.g., comprising a split CAR wherein conditional hetero-dimerization of the two portions of the split CAR is pharmacologically controlled. CAR variants also include bispecific CARs, which include a secondary CAR binding domain that can either amplify or inhibit the activity of a primary CAR. CAR variants also include inhibitory chimeric antigen receptors (iCARs) which may, e.g., be used as a component of a bispecific CAR system, where binding of a secondary CAR binding domain results in inhibition of primary CAR activation. CAR molecules and derivatives thereof (i.e., CAR variants) are described, e.g., in PCT Application No. US2014/016527.

In certain embodiments, the method may include a method of activating a T cell, e.g. a T-cell expressing CD3 and CD28. In certain embodiments, the method of the present disclosure may be used for inducing T-cell proliferation without significantly increasing cytokine production by the T cell and/or causing T-cell exhaustion. For example, the method may include contacting a T cell with a particle having first binding members (e.g. antibodies) displayed on its surface, where the first binding members bind to second binding members expressed on the surface of the T cell (e.g. CD3 and CD28), and where binding of the first binding members to the second binding members induces T-cell proliferation without significant increase in cytokine production. In certain embodiments, the level of cytokines produced by the T cells in the presence of the particles described herein is lower than the level of the cytokines produced by the T cells in the presence of non-polymeric particles (e.g. magnetic beads) having the same first binding members. In certain embodiments, the T-cells exhibit a lower exhaustion profile in the presence of the particles described herein compared to the exhaustion profile exhibited by T-cells in the presence of non-polymeric particles (e.g. magnetic beads) having the same first binding members.

Cell proliferation can be determined and quantified, for example, using a cell counter, such as is described further in the Examples herein. The levels of cytokines produced by T-cells can be determined and quantified, for example, by measuring the levels of (extracellular and/or intracellular) interferon-gamma produced by the cells, such as is further described in the Examples herein. The exhaustion profile can be determined, for example, by measuring the levels of T-cell exhaustion markers such as LAG-3, PD-1 and/or TIM-3, such as is further described in the examples. A lower exhaustion profile may be revealed by a lower expression profile of one, two or all three of LAG-3, PD-1 and TIM-3.

### METHODS OF MAKING POLYMERIC PARTICLES

The present disclosure also provides methods for making the polymeric particles disclosed herein. The method may include the formation of an emulsion to generate the polymeric particles functionalized with at least one biomolecule on a surface thereof, such as, a first, a second, a third, and so forth biomolecules on the surface thereof. In certain embodiments, the method may include the formation of a double emulsion to generate polymeric particles encapsulating a biomolecule inside the particles and functionalized with at least one biomolecule on a surface thereof, such as, a first, a second, a third, and so forth biomolecules on the surface thereof.

In certain embodiments, a method of making a polymeric particle method may include covalently linking a nucleic acid to a first polymer to generate a nucleic acid-polymer conjugate, where the nucleic acid is a first single stranded nucleic acid; sonicating a solution that includes the nucleic acid-polymer conjugate and a second polymer to generate polymeric particles comprising a polymeric core comprising the second polymer, wherein the polymer region of the nucleic acid-polymer conjugate is non-covalently associated with the polymeric core thereby presenting the first single stranded nucleic acid on a surface of the polymeric core; attaching to the polymeric core a second single stranded nucleic acid having a sequence complementary to the first single stranded nucleic acid by hybridization; and covalently or non-covalently attaching the second single stranded nucleic acid to a first binding member of a specific-binding pair to generate the polymeric particle.

Hybridization may be performed in conditions and for a period of time sufficient for formation of specific-base pairing between complementary bases in the nucleic acids (e.g., for formation DNA:DNA or DNA:RNA or RNA:RNA or DNA:PNA or PNA:PNA double stranded regions). In certain cases, the hybridization may be performed at a temperature of at least 30°C, such as, 30°C-50°C, 37°C-50°C or 37°C-45°C for at least 10 min, e.g., 10 min- 60 min, 10 min- 45 min, or 10 min- 30 min.

In certain embodiments, the method comprises covalently attaching the second single stranded nucleic acid to the first binding member prior to attaching the second single stranded nucleic acid to the polymeric core.

In certain embodiments, the method comprises covalently attaching the second single stranded nucleic acid to the first binding member after attaching the second single stranded nucleic acid to the polymeric core.

The single stranded nucleic acid and the first binding member may be attached directly or indirectly via a linker.

In certain embodiments, the method comprises covalently attaching the second single stranded nucleic acid to a biotin molecule and non-covalently attaching an avidin-first binding member conjugate to the second single stranded nucleic acid.

In certain embodiments, the method comprises generating a plurality of nucleic acid-polymer conjugates, where the plurality of nucleic acid-polymer conjugates at least include a first nucleic acid-polymer conjugate comprising a first single stranded nucleic acid covalently linked to a first polymer molecule; and a second nucleic acid-polymer conjugate comprising a third single stranded nucleic acid covalently linked to a first polymer molecule, where the first single stranded nucleic acid and the third single stranded nucleic acid have different sequences. In certain cases, the plurality of nucleic acid-polymer conjugates includes at least three, at least four, at least five, or more different nucleic acid-polymer conjugates each comprising a different single stranded nucleic acid, where the sequences are sufficiently different to enable hybridization to different nucleic acids which ate in turn attached to different first binding members of a specific binding pair.

In certain embodiments, the method comprises sonicating a solution comprising the plurality of nucleic acid-polymer conjugates and a second polymer to generate polymeric particles comprising a polymeric core comprising the second polymer, wherein each polymer region of the plurality of nucleic acid-polymer conjugates is non-covalently associated with the polymeric core thereby presenting the first single stranded nucleic acid and the third single stranded nucleic on a surface of the polymeric core; attaching to the polymeric core: the second single stranded nucleic acid having a sequence complementary to the first single stranded nucleic acid by hybridization and a fourth single stranded nucleic acid having a sequence complementary to the third single stranded nucleic acid by hybridization; and covalently or non-covalently attaching: the second single stranded nucleic acid to a first binding member of a first specific-binding pair and the fourth single stranded nucleic acid to a biomolecule to generate the polymeric particle.

In certain case, the biomolecule is a self-peptide. In certain cases, the biomolecule is a first binding member of a second specific-binding pair. As noted herein, the plurality of nucleic acid-polymer conjugates can included in the desired ratio. For example, the first nucleic acid-polymer conjugate and the second nucleic acid-polymer conjugate may be included in the solution at a ratio of 100:1, 50:1, 10:1, 5:1, 3:1, 2:1, 1:1, 1:2, 1:3, 1:5, 1:10, 1:50, or 1:100. In certain embodiments where one of first binding members binds CD3 and the other first binding member binds CD28, the nucleic acid-polymer conjugate to be attached to the first binding member that binds CD3 and the nucleic acid-polymer conjugate to be attached to the first binding member that binds CD28 may be included in the solution at a ratio of 1:3 to 5:1, a ratio of 1:1 to 5:1, a ratio of 2:1 to 4:1, or a ratio of 3:1. In another example, the first nucleic acid-polymer conjugate, the second nucleic acid-polymer conjugate, and a third nucleic acid-polymer conjugate may be included in the solution at a ratio of 10:1:1, 5:1:1, 3:1:1, 2:1:1, 1:1:1, 1:1:2, 1:1:3, 1:1:5, or 1:1:10.

The step of covalently linking a nucleic acid to a first polymer to generate a nucleic acid-polymer conjugate may be carried out using standard chemistry suitable for the reactive groups being used for the covalent attachment. Examples of suitable reactive groups include thiol-maleimide, amino and carboxyl groups, thiol and carboxyl groups, and the like. Any suitable solution may be used for forming an emulsion from the polymers to generate the polymeric particles. In certain embodiments, the solution may be substantially hydrophilic. Sonication may be carried out for a period of time and under conditions sufficient for generation of the particles.

In certain embodiments, a double emulsion procedure may be performed for encapsulating substantially hydrophilic biomolecules or amphipathic biomolecules in the polymeric core of the particles. In certain embodiments, the method of making a polymeric particle comprising peptide, polypeptide, and/or nucleic acid encapsulated in a polymeric core and a nucleic acid-polymer conjugate comprising a first single stranded nucleic acid covalently attached to a first polymer, wherein the polymer is non-covalently associated with the polymeric core thereby presenting the first single stranded nucleic acid on a surface of the polymeric core may include sonicating a solution comprising the peptide, polypeptide, and/or nucleic acid and a second polymer; adding the nucleic acid-polymer conjugate to the solution and further sonicating the solution to generate polymeric particles comprising a polymeric core comprising the second polymer and encapsulating the peptide, polypeptide, and/or nucleic acid, wherein the polymer region of the nucleic acid-polymer conjugate is non-covalently associated with the polymeric core thereby presenting the first single stranded nucleic acid on a surface of the polymeric core; attaching to the polymeric core a second single stranded nucleic acid having a sequence complementary to the first single stranded nucleic acid by hybridization; and covalently or non-covalently attaching the second single stranded nucleic acid to a first binding member of a specific-binding pair to generate the polymeric particle.

In certain cases, the initial emulsification may be carried out in an organic solution such that the hydrophilic biomolecule is distributed to the interior and surrounded by the polymer, followed by further emulsification in an aqueous phase, for example, by adding to the emulsion a solution of the nucleic acid-polymer conjugate dissolved in water, followed by sonication, resulting in insertion of the polymer region of the nucleic acid-polymer conjugate into the particles to generate the polymeric particles encapsulating the biomolecules. The functionalization of the particle to add the first binding member may then be carried out as outlined herein.

### CAR-ANTIGEN PRESENTING PARTICLES (CAPP)

Synthetic particles presenting an antigen that binds to a chimeric antigen receptor (CAR) expressed on a cell are provided. Such particles are termed CAR-antigen presenting particles (CAPP). As further described herein and without wishing to be bound by theory, CAPP are believed to exhibit certain advantages in activating CAR-T cells as compared to non-synthetic particles (e.g. cells, such as antigen presenting cells) presenting CAR antigens.

In certain embodiments the synthetic particle is a polymeric particle, a magnetic particle or a liposome. In certain embodiments, the synthetic particle is a polymeric particle, such as a polymeric particle having the features and properties as described in the preceding section and as further described herein. Methods of producing CAPP where the particle is a polymeric particle are described herein. For example, where the particle is a polymeric particle, the CAR antigen can be presented on a surface of the polymeric particle through the use of a nucleic acid-polymer conjugate as described in more detail above.

The CAR antigen may be presented on a surface of the synthetic particle through any suitable means. For example, this may involve the use of a polymer such as PEG (Polyethylene glycol) with functional groups (e.g. NH2-, -SH, NHS, MAL, azide, alkyne, DBCO, epoxy, aldehyde, biotin, avidin, streptavidin, etc.) on both ends. The PEG may have a molecule weight of between 50 - 10,000 Da. One functional group may interact with the synthetic particle and the other functional group may interact with the antigen. Also contemplated are the use of magnetic beads functionalized with avidin or biotin. Biotinylated CAR antigens can be added to the synthetic particle that includes surface streptavidin as described in more detail in the examples herein.

In certain embodiments, the antigen that binds to a CAR expressed on a cell may be CD19, HER2, epidermal growth factor receptor (EGFR), green fluorescent protein (GFP), fluorescein isothiocyanate (FITC), CD20, CD38, CD30, CA125, MUC-1, prostate-specific membrane antigen (PSMA), CD44 surface adhesion molecule, mesothelin, carcinoembryonic antigen (CEA), EGFRvIII, vascular endothelial growth factor receptor-2 (VEGFR2), high molecular weight-melanoma associated antigen (HMW-MAA), MAGE-A1, IL-13R-a2, GD2, MET, GPC3, CD70, EphA2, EpCAM, CLDN18, or CA9. In certain embodiments, the antigen that binds to a CAR expressed on a cell is selected from the group consisting of CD19, HER2, epidermal growth factor receptor (EGFR), MET, GPC3, CD70, EphA2, EpCAM, CLDN18, BCMA, and CA9. In certain embodiments, the antigen that binds to a CAR expressed on a cell is selected from the group consisting of CD19, HER2, and epidermal growth factor receptor (EGFR). The CAR may be expressed on an effector T cell or a regulatory T cell.

In certain embodiments, the antigen binds to a CAR expressed on a regulatory T cell (CAR-Tregs). Such antigens may be useful for inducing proliferation of CAR-Treg cells and may have application in cellular therapy in transplantation and autoimmune diseases. Examples of CAR-Treg cells and the antigens that they have been targeted against are provided in Zhang et al. 2018, Front. Immunol., 9:2359, which is herein incorporated by reference. See, in particular, Table 1 of Zhang et al.

The present disclosure provides methods for using the CAPP disclosed herein. The CAPP may be used in a variety of in vitro, ex vivo and in vivo methods.

In certain embodiments, the disclosure provides methods of enhancing proliferation of a CAR-T cell, the method comprising contacting the CAR-T cell with a CAR-antigen presenting particle (CAPP) as described herein. In certain embodiments, the methods of the present disclosure may be used for enhancing proliferation of a CAR-T cell without significantly increasing cytokine production by the CAR-T cell and/or causing CAR-T cell exhaustion. In certain embodiments, the level of cytokines produced by the CAR-T cells followed by contact with the CAPP described herein is lower than the level of the cytokines produced by the CAR-T cells followed by contact with cells, e.g. antigen presenting T cells, presenting the CAR-antigen. In certain embodiments, CAR-T cells exhibit a lower exhaustion profile following contact with CAPP described herein compared to the exhaustion profile exhibited by CAR-T cells contacted with cells, e.g. antigen presenting T cells, presenting the CAR-antigen. Exemplary methods of measuring cell proliferation, cytokine production and cell exhaustion are described herein.

In certain embodiments, the methods of enhancing proliferation of a CAR-T cell are carried out ex vivo. In certain embodiments, the ex vivo method comprises contacting a population of T cells comprising the CAR-T cell, wherein the population of T cells have been isolated from a subject. In certain embodiments, the method further comprises administering the CAR-T cells to the subject following proliferation.

In certain embodiments, the methods of enhancing proliferation of a CAR-T cell are carried out in vivo. In certain embodiments, the in vivo method comprises administering the synthetic particle to the subject. Also disclosed herein are CAPP and CAR-T cells for use in the in vivo methods described herein.

In the ex vivo or in vivo methods described herein, the subject may have a cancer such as a B cell cancer. In certain embodiments, the B cell cancer is leukemia. In certain embodiments, the leukemia is relapsed or refractory CD 19+ leukemia. In certain embodiments, the subject is undergoing or has previously undergone CAR-T cell immunotherapy.

In addition, the present disclosure also provides kits that include the CAPP disclosed herein and a CAR-T cell that specifically binds to the antigen presented on the CAPP. The present disclosure also provides kits that include the CAPP disclosed herein and nucleic acid(s) or viral particle(s) encoding the CAR, where the CAR, once expressed on a T cell, specifically binds to the antigen presented on the CAPP. The nucleic acid(s) encoding the CAR may be part of a vector, e.g. a viral vector such as a lentiviral vector. The viral particle(s) encoding the CAR may be a lentiviral particle, e.g. a harvested lentiviral particle.

### BIOMOLECULE-COATED FILMS

Biomolecule-coated films functionalized by attaching one or more biomolecules of interest are also provided. In certain embodiments, biomolecule-coated films functionalized by attaching two or more biomolecules of interest, optionally where the ratio number of each of the two or more biomolecules is controlled, are provided. In certain embodiments, biomolecule-coated films functionalized by attaching one or more biomolecules of interest, where the density of each is controlled, are provided. In certain embodiments, biomolecule-coated films may encapsulate biomolecules in addition to having one or more biomolecules presented on the surface of the films. The biomolecule may be as further described herein. In certain embodiments, the biomolecule is a CAR-antigen and may have the features and properties of the CAPP and/or used in the methods of enhancing proliferation of the CAR-T cells as further described herein.

In certain embodiments, the biomolecule-coated film comprises a polymeric film comprising one or more pores; a nucleic acid-polymer conjugate comprising a first single stranded nucleic acid covalently attached to a polymer, wherein the polymer is covalently or non-covalently associated with the polymeric film thereby presenting the first single stranded nucleic acid on a surface of the polymeric film; and a first biomolecule-nucleic acid conjugate comprising a second single stranded nucleic acid covalently attached to a biomolecule, wherein the second single stranded nucleic acid is complementary to the first single stranded nucleic acid and is associated with the first single stranded nucleic acid via hybridization thereby presenting the first biomolecule on a surface of the polymeric film.

In certain embodiments, the nucleic acid and/or nucleic acid-polymer conjugate may be as further described in the context of the polymeric particles herein.

In certain embodiments, the polymer used to form the polymeric film may be any polymer, e.g. a biodegradable and biocompatible polymer. In certain cases, the polymer may be polylactic acid (PLA), polyglycolic acid (PGA), poly(D,L-lactide-co-glycolide) (PLGA), polyethylene glycol (PEG), and/or poly(e-caprolactone) (PCL). In another aspect, the polymer may be a copolymer such as poly(lactide-co-glycolide) (PLGA) or poly(lactide-co-glycolide) poly(e-caprolactone) (PLGA/PCL). In certain embodiments, the polymer used to form the polymeric film may be a combination of one or more of PLA, PGA, PEG, PCL, PLGA, and PLGA/PCL. In certain embodiments, the polymeric film comprises PCL and PEG.

In certain embodiments, the polymeric film comprises pores having a diameter of between 0.1 µm to 10 µm. For example, the pore may have a diameter of between 0.1 µm to 5 µm, 0.1 µm to 3 µm, 0.1 µm to 2 µm, 0.5 µm to 10 µm, 0.5 µm to 5 µm, 0.5 µm to 3 µm, 0.5 µm to 2 µm, 1 µm to 10 µm, 1 µm to 5 µm, 1 µm to 3 µm, or 1 to 2 µm. In certain embodiments, the polymeric film comprises pores having a diameter of between 1 µm to 2 µm. In certain embodiments, the polymeric film comprises pores having a diameter of between 10 nm to 100 nm. For example, the pore may have a diameter of between 10 nm to 90 nm, 10 nm to 80 nm, 20 nm to 90 nm, 30 nm to 90 nm, 30 nm to 80 nm, 40 nm to 90 nm, 40 nm to 80 nm, 50 nm to 90 nm, or 50 nm to 80 nm.

In certain embodiments, the thickness of the polymeric film is between 0.1 µm and 100 µm. For example, the polymeric film may have a thickness of between 0.1 µm and 1 µm, 1 µm and 80 µm, 1 µm and 70 µm, 1 µm and 60 µm, 50 µm, 1 µm and 40 µm, 1 µm and 30 µm, 1 µm and 20 µm, 5 µm and 80 µm, 5 µm and 70 µm, 5 µm and 60 µm, 5 µm and 50 µm, 5 µm and 40 µm, 5 µm and 30 µm, 5 µm and 20 µm, 10 µm and 80 µm, 10 µm and 70 µm, 10 µm and 60 µm, 10 µm and 50 µm, 10 µm and 40 µm, 10 µm and 30 µm, or 10 µm and 20 µm. In certain embodiments, the polymeric film has a thickness of between 10 µm and 20 µm.

In certain embodiments, the biomolecule-coated film comprises a biomolecule that is selected from the group consisting of a protein, a peptide, an antibody, and a nucleic acid. In certain embodiments, the biomolecule-coated film comprises a biomolecule that is a first binding member, wherein the first binding member is a member of a specific-binding pair, where the first binding member specifically binds to a second binding member that is a member of the specific-binding pair. The biomolecule may have the properties and features of the first binding member described herein in the context of the polymeric particles. In certain embodiments, the first binding member is an antigen and the second binding member is a CAR expressed on a CAR-T cell. In certain cases, the biomolecule-coated film may comprise more than one member of a specific-binding pair. For example, a biomolecule-coated film of the present disclosure may include a first biomolecule and a second biomolecule. The first biomolecule may be a member of a first specific-binding pair and the second biomolecule may be a member of a second specific-binding pair.

In certain embodiments, the biomolecule is a co-stimulatory receptor agonist that can be used to modulate immune cell locally. For example, the co-stimulatory receptor agonist may be a binding member, e.g. an antibody, that binds CD28, CD137, OX40, GITR, ICOS, CD27, CD30, and HVEM. In certain embodiments, the biomolecule is a cytokine, e.g. a cytokine selected from the group consisting of IL-2, IL-15, IL-12, and GM-CSF. In certain embodiments, the biomolecule is a binding member that binds a checkpoint inhibitor, e.g. an antibody that binds a checkpoint inhibitor such as one selected from the group consisting of PD-1, PD-L1, CTLA, B7-H1, IDO, TGF-β, BTLA, VISTA, LAG-3, B7-H4, Arginase, MICA, MICB, and TIM-3. In certain embodiments, the biomolecule is an adjuvant, such as CpG, TLR agonist or a STING agonist.

The present disclosure provides methods for using the biomolecule-coated films disclosed herein. The biomolecule-coated films may be used in a variety of in vitro, ex vivo and in vivo methods.

In certain embodiments, the biomolecule-coated films may be used in in vivo methods for therapeutic use. For example, the biomolecule-coated films may be used to administer therapeutic agents, such as, peptides or proteins such as antibodies, or small molecules, such as drugs. As used herein, the term "small molecule" refers to synthetic or naturally occurring organic compound that typically has a molecule weight of 500 daltons or less. In some cases, the biomolecule-coated film may be used to provide a first binding member of a specific-binding pair to a cell expressing a second member of the specific-binding pair by contacting the cells with the biomolecule-coated film. In some cases, the biomolecule-coated film may be used to provide a first binding member of a specific-binding pair to a subject expressing a second member of the specific-binding pair in a soluble form, e.g., cytokines, secreted antibodies (e.g., IgE antibodies). In some cases, the biomolecule-coated film may be used to provide the first binding member to a target organ, tissue, or cell. In some cases, the biomolecule-coated films may be used to deliver therapeutic agents. For example, therapeutic agents such as nucleic acid, peptide, polypeptide, and/or small molecules may be encapsulated in the biomolecule-coated films and released through the pores. In other cases, the biomolecule-coated films may be used to deliver cells secreting therapeutic agents. For example, a cell or population of cells may be encapsulated in the biomolecule-coated films and therapeutic agents secreted by the cell released through the pores. Also provided are biomolecule-coated films for use in the in vivo methods of therapeutic use described herein.

In certain embodiments, the biomolecule-coated films are used in a method of enhancing proliferation of a CAR-T cell. In certain embodiments, the method involves the use of a biomolecule-coated film comprising a biomolecule that is a first binding member, wherein the first binding member is an antigen that specifically binds to a second binding member that is a CAR expressed on a CAR-T cell. The CAR-T cell may be an effector T cell or a regulatory T cell. The biomolecule may have the properties and features of the first binding member described herein in the context of the polymeric particles. In certain embodiments, the first binding member is an antigen and the second binding member is a CAR expressed on a CAR-T cell. The method may be carried out in vivo or ex vivo, for example as further described herein in the context of methods involving CAPP.

The present disclosure provides methods for making the biomolecule-coated films disclosed herein. In certain embodiments, the method comprises covalently or non-covalently linking a nucleic acid to a first polymer to generate a nucleic acid-polymer conjugate, wherein the nucleic acid is a first single stranded nucleic acid; mixing a solution comprising the nucleic acid-polymer conjugate and a second polymer in a solvent; film casting the solution to generate a polymeric film comprising the second polymer, wherein the polymer region of the nucleic acid-polymer conjugate is non-covalently associated with the polymeric film thereby presenting the first single stranded nucleic acid on a surface of the polymeric film; attaching to the polymeric film a second single stranded nucleic acid having a sequence complementary to the first single stranded nucleic acid by hybridization; and covalently or non-covalently attaching the second single stranded nucleic acid to a biomolecule to generate the biomolecule coated film.

In certain embodiments, the method comprises covalently attaching the second single stranded nucleic acid to the first binding member prior to attaching the second single stranded nucleic acid to the polymeric film.

In certain embodiments, the method comprises covalently attaching the second single stranded nucleic acid to the first binding member after attaching the second single stranded nucleic acid to the polymeric film.

In certain embodiments, the method comprises generating a plurality of nucleic acid-polymer conjugates, wherein the plurality of nucleic acid-polymer conjugates comprises: a first nucleic acid-polymer conjugate comprising a first single stranded nucleic acid covalently linked to a first polymer molecule; and a second nucleic acid-polymer conjugate comprising a third single stranded nucleic acid covalently linked to a first polymer molecule, wherein the first single stranded nucleic acid and the third single stranded nucleic acid have different sequences.

As noted herein, the plurality of nucleic acid-polymer conjugates can included in the desired ratio. For example, the first nucleic acid-polymer conjugate and the second nucleic acid-polymer conjugate may be included in the solution at a ratio of 100:1. 50:1, 10:1, 5:1, 3:1, 2:1, 1:1, 1:2, 1:3, 1:5, 1:10, 1:50, or 1:100. The step of covalently linking a nucleic acid to a first polymer to generate a nucleic acid-polymer conjugate may be carried out using standard chemistry suitable for the reactive groups being used for the covalent attachment. Examples of suitable reactive groups include thiol-maleimide, amino and carboxyl groups, thiol and carboxyl groups, and the like.

The step of mixing the solution comprising the nucleic acid-polymer conjugate and second polymer can be carried out in any solvent suitable for film casting. Examples of suitable common solvents that may be used include, but are not limited to, dimethyl oxalate (DMO), ethylene carbonate (EC), N-methyl acetamide (NMA), dimethyl sulfoxide (DMSO), acetic acid (AA), 1,4-dioxane (DO), dimethyl carbonate (DMC), chloroform, dichloromethane (DCM), naphthalene, sulfalene, trimethylurea, ethylene glycol or other glycols and polyglycols, N-methyl pyrrolidone (NMP), ethylene carbonate, hexane, cyclohexane, trifluoroethanol (TFE), ethanol, acetic acid, and water, and combinations thereof. In certain embodiments, the solvent is a combination of TFE and water.

### EXAMPLES

As can be appreciated from the disclosure provided above, the present disclosure has a wide variety of applications. Accordingly, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Those of skill in the art will readily recognize a variety of noncritical parameters that could be changed or modified to yield essentially similar results. Thus, the following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, dimensions, etc.) but some experimental errors and deviations should be accounted for.

### Materials and methods

### 1. Synthesis of Thiol-modified DNA

3' Thiol-modified DNA with 17 bases are synthesized on 3' thiol-modifier 6 S-S CPG beads (Glen Research #10-1936-02) using an Expedite 8909 DNA synthesizer. DNA are retrieved from the beads by 70°C incubation for 20 mins in the presence of AMA solution (Ammonium hydroxide:Methylamine=1:1, v/v), followed by vacuum evaporation (SpeedVac, ThermoFisher #SPD121P-230) for 3 hours to remove AMA. DNA reconstituted in TE buffer (Tris-EDTA, 10mM, pH7.5) are then filtered through 0.22 µm filter (Millipore #UFC30GV00), and kept in -20 °C for stock.

### 2. Synthesis of amphiphilic polymer-DNA

Synthesized DNA are treated with Tris(2-carboxyethyl)phosphine hydrochloride solution (TCEP, Sigma #646547) with 100x molar excess at 37 °C for 1 hour to remove the disulfide protection, and then purified using a size-exclusion chromatography (Glen Research #61-5010). Freshly prepared thiol-DNA are mixed with 200 µM maleimide-functionalized polymers (i.e. Poly(lactide-co-glycolide)-b-poly(ethylene glycol)-maleimide, Mw 10,000:5000 Da, Akina #AI053, PLGA-PEG-MAL) at different ratios in the solvent of dimethylformamide (DMF)/H₂O (90:10, v/v) with the addition of 0.2% triethanolamine, and incubated at room temperature for overnight to complete the reaction. The next day the solvent is removed by vacuum evaporation heated to 70°C for 3 hours, and the polymer-DNA is stocked in -20°C. The product quality is determined by running TBE-Urea gel electrophoresis (15%, Invitrogen #EC68855).

### 3. Fabrication of polymeric particles with DNA scaffolds

Polymeric PLGA particles are fabricated using a single emulsion method in the mixture of aqueous buffer and organic solvent. Amphiphilic polymer-DNA is reconstituted in 200 µL solvent mixture (ethyl acetate:H₂O=1:1, v/v), and mixed with certain amount of the mainstream (unmodified) polymer (Poly-lactide-co-glycolide, 50:50, Mw 38,000-54,000, Sigma #719900, or Polylactic acid, Mw 60,000, Sigma #38534) dissolved in ethyl acetate or dichloromethane (DCM) according to the target particle size and degradation profile, with the addition of aqueous buffer (10mM sodium citrate, 600 mM Na⁺, pH3.0). For PLGA microparticles with 1-5 µm diameter, 100 nmol PLGA-DNA and 50mg PLGA (Sigma #719900) are mixed in 0.5 mL ethyl acetate and 0.5 mL aqueous buffer; for PLA microparticles with 1-5 µm diameter, 200 nmol PLGA-DNA and 50mg PLA (Sigma #38534) are mixed in 0.5 mL DCM and 0.5 mL aqueous buffer; for PLGA particles with around 500 nm diameter, 100 nmol PLGA-DNA and 10mg PLGA (Sigma #719900) are mixed in 0.5 mL ethyl acetate and 1 mL aqueous buffer; and for PLGA particles with around 200 nm diameter, 100 nmol PLGA-DNA and 10mg PLGA (Sigma #719900) are mixed in 0.5 mL ethyl acetate and 1 mL aqueous buffer with 1 % polyvinyl alcohol (PVA, Sigma #81381) addition. The whole mixture is then vortexed and probe-sonicated on ice at 7-8 W for 5 × 5 s with 10 s intervals, and immediately added with 9 mL 0.2% PVA and stirred in the hood for 3 hours for ethyl acetate to evaporate. Particles are filtered through 40 µm cell strainer (Sigma #CLS431750), and centrifuged at 10,000x g for 10 mins to collect the pellet, and then resuspended in TE buffer (10mM Tris-HCl, pH8.0) with 0.01% Tween-20 for washing. This protocol remains the same for later washing steps unless specifically noted. After three washes, particles are resuspended in TE buffer with 1% PVA and lyophilized for long term storage.

### 4. Surface hybridization and step-by-step conjugation to attach biomolecules

Lyophilized particles are reconstituted in water, and measured the optical density at 550 nm as an indication of particle concentration. NH₂-modified DNA strands (Biosearch Technologies) complementary to the scaffolds are added at ~200 nM/OD₅₅₀ and incubated at 37°C for 30 mins for hybridization, followed by centrifugations to wash off unhybridized DNA. A large excess of MAL-dPEG₄-NHS linker (Quanta Biodesign #10214) is added at 6 µM/OD₅₅₀ and incubated at RT for 1 hour to endow the particles with thiol-reactive function, followed by three washes to remove the excess. Biomolecules with free thiols are then added at 50 nM/OD₅₅₀ and incubated at RT for 1 hour to conjugate to the surface of particles. Particles with biomolecules loaded are washed for three times and lyophilized in PBS buffer with 1% PVA supplemented. Biomolecules labeled with fluorescent dyes, once loaded on particles, are analyzed the efficiency by dissolving the particles in 95% Dimethyl sulfoxide (DMSO) and further diluting for 10 folds in PBS for fluorescence-based quantification.

To fabricate control particles without DNA scaffold, 100 nmol block-co-polymer PLGA-PEG-MAL (Akina #AI053) is mixed with 50 mg PLGA (Sigma #719900) in the solvent composed of 0.5 mL ethyl acetate and 0.5 mL H₂O. Following the probe-sonication process described above, the particles formed are filtered through 40 µm filter (Sigma #CLS431750), and washed by centrifugation. MAL-presenting particles are then added with biomolecules with free thiol exposed at 50 nM/OD₅₅₀ and incubated at 37°C for 1 hour for conjugation.

### 5. Attachment of DNA to antibody or Fc-tagged protein and its purification

Antibody (anti-PD-L1, Bio-X-Cell #BE0285; anti-CD28, Bio-X-Cell #BE0248) or Fc-tagged protein (HER2, Aero Biosystems #HE2-H5253) is exchanged the buffer to the reducing buffer (PBS with 10 mM EDTA supplemented) by size exclusion chromatography (Zeba Spin Desalting Column, Thermo Scientific #89882), and selectively reduced the disulfide bond at the hinge region by adding TCEP with 4.5 molar excess and incubating at 37°C for 1 hour. Excess amount of TCEP is removed through size exclusion chromatography. 3'-NH₂ modified DNA complementary to the scaffold (Biosearch Technologies) is conjugated with MAL-dPEG₄-NHS linker (Quanta Biodesign #10214) with 30-fold molar excess in HEPES buffer (pH7.0) at 37°C for 1 hour, followed by the removal of the unconjugated linker through 70% ethanol precipitation and size exclusion chromatography. Reduced antibody or Fc-tagged protein, and conjugated DNA are combined with the molar ratio of 1:10, and incubated at 37°C for 1 hour and 4°C for overnight. The next day, DNA-protein conjugates are purified using protein G affinity chromatography (Genscript #L00209) to remove unconjugated DNA.

### 6. Attachment of DNA to his-tag GFP and its purification

His-tag GFP are expressed by *Escherichia coli* BL21 (DE3) (Novagen) transduced with pRSET-EmGFP vector (ThermoFisher, #V35320) in E. coli expression medium (MagicMedia, Invitrogen #K6803), and extracted using cell lysis reagent (Sigma, #B7435) followed by the purification using nickel-nitrilotriacetic acid affinity chromatography (Invitrogen #R90115). MAL-PEG₄-NHS linker is mixed with GFP at 30-fold molar excess, and incubated at 37°C for 1 hour followed by the size-exclusion chromatography to remove the excess. Thiolated complementary DNA (Biosearch Technologies) is treated with TCEP at 100 molar excess at 37°C for 1 hour to remove the protection cap and precipitated in 70% ethanol to remove excess amount of TCEP. Modified GFP and thiol-DNA are combined at 1:10 molar ratio and incubated at 37°C for 1 hour and 4°C for overnight. The next day, GFP-DNA conjugates are purified using nickel-nitrilotriacetic acid affinity chromatography to remove unconjugated DNA.

### 7. Surface loading of biotinylated proteins

3'-biotinylated DNA complementary to the scaffolds (Biosearch Technologies) is hybridized to the particle surface using the protocol described above. After that, a large excess of streptavidin (Prozyme #SA10) is added at 1.1 mg/mL per OD₅₅₀, mixed immediately, and incubated at RT for 30 mins, followed by three washes. Biotinylated antibody, protein or peptide is added subsequently and incubated at RT for 30 mins to bind with streptavidin for particle surface loading followed by three washes.

### 8. Surface functionalization of polymeric particles with multiple proteins at intended ratio

### 3' Thiolated DNA with different sequences are synthesized: R

(5'AGTGGGAGCGCGTGATG3'); G (5'GTTCATCTGCACCACCG3'); B (5'GCCTTTACGATGTCCTT3'). Following the conjugation with PLGA-PEG-MAL (Akina, #AI053), polymer-DNA with different sequences at intended ratios, together with mainstream polymer and solvents, are mixed and fabricated the particles as described above. Complementary DNA strands pre-conjugated with proteins (described above) or biotinylated complementary strand (Biosearch Technologies) are hybridized onto the particles with 60 nM/OD₅₅₀ total and the ratios same as the input ratio during fabrication. For the proportion of biotinylated DNA strand, streptavidin and biotinylated protein/peptide/antibody are assembled on the particles surface as described above. Surface protein species are quantified the loading through the fluorescently labeled DNA part or protein part.

### 9. Enzymatic challenge assay

Particles hybridized with fluorescently labeled complementary strands, with or without IgG coverage are treated with DNase (RQ1 RNase-free DNase, Promega #M6101) at 5 U per 1 OD₅₅₀ × 50 µL and incubated at 37°C for 20 mins, followed by the centrifugation at 10,000 × g for 10 mins to analyze the supernatant fluorescence signal.

### 10. IVIS imaging-based particle stability assay

NH₂-modified PLGA polymer (Poly(lactide-co-glycolide)-NH₂, LG 50:50, Mw 30,000-40,000 Da) dissolved in DMF is reacted with IR800CW-NHS Ester (Li-COR #P/N 929-70020) dye with 30-fold molar excess at RT for 1 hour, followed by the repeated 70% ethanol precipitation and DMF re-dissolving to remove unconjugated dye. 1 mg of IR800CW-labeled polymer is mixed with 50 mg mainstream polymer and 100 nmol PLGA-DNA to fabricate particles with 1-5 µm diameter using the protocol described above. 5'Quasar705-modified complementary strand (Biosearch Technology) is hybridized to track the surface DNA scaffold, while IR800 for the core tracking. NSG mice (female, ~8-12-weeks old) are implanted with xenograft tumors - 5 × 10⁶ K562 tumor cells subcutaneously on the left and right flank, respectively. 10 days after tumor implantation, 50 µL fluorescence-labeled particles at 200 OD₅₅₀ are injected intratumorally, and imaged under IVIS 100 preclinical imaging system (Xenogen #124262) every 3-4 hours for the first 48 hours and every 8 hours for the rest of the week. Images are analyzed using Living Image Software (PerkinElmer).

### 11. Encapsulation of peptide and DNA in particles with DNA-scaffold

Polymeric PLGA particles with DNA scaffold on the surface as well as peptide/DNA in the core are fabricated using a double-emulsion method. 0.25 mg peptide (Genscript) and 50 nmol DNA (Bioresearch Technologies) dissolved in 50 µL PBS is combined with 50 mg PLGA (Sigma #719900) dissolved in 0.5 mL ethyl acetate, mixed and probe-sonicated at 7-8 W for 5 × 5 s with 10 s intervals on ice. Then 100 nmol amphiphilic polymer-DNA reconstituted in 100 µL solvent mixture (ethyl acetate:H₂O = 1:1, v/v) is added along with 400 µL aqueous buffer (10mM sodium citrate, 600 mM Na⁺, pH3.0). The whole mixture is quickly vortexed and probe-sonicated at 7-8 W for 5 × 5 s with 10 s intervals on ice, and immediately added with 9 mL 0.2% PVA and stirred in the hood for 3 hours for ethyl acetate to evaporate. Particles are filtered through 40 µm filter, and centrifuged at 10,000 × g for 10 mins to collect the pellet, and then resuspended in TE buffer with 0.01% Tween-20 for washing. After three washes, particles are resuspended in TE buffer with 1% PVA and lyophilized for long term storage.

### 12. Primary human T cell isolation and culture

Primary CD4+ and CD8+ T cells are isolated from anonymous donor blood after apheresis by negative selection (STEMCELL Technologies #15062 and #15063). Blood is obtained from Blood Centers of the Pacific, as approved by the University Institutional Review Board. T cells are cryopreserved in RPMI-1640 (UCSF cell culture core) with 20% human AB serum (Valley Biomedical, #HP1022) and 10% DMSO. After thawing, T cells are cultured in human T cell medium consisting of X-VIVO 15 (Lonza #04-418Q), 5% Human AB serum, and 10 mM neutralized N-acetyl L-Cysteine (Sigma-Aldrich #A9165) supplemented with 30 units/mL IL-2 (NCI BRB Preclinical Repository) for all experiments.

### 13. Transduction of synthetic Notch CAR T cells

SynNotch receptor is built by fusing the LaG17 nanobody to the mouse Notch1 (NM_008714) minimal regulatory region (Ile1427 to Arg1752) and Gal4 DBD VP64. It also contains an n-terminal CD8a signal peptide (MALPVTALLLPLALLL HAARP) for membrane targeting and a myc-tag (EQKLISEEDL) for easy determination of surface expression with a-myc A647 (cell-signaling #2233). The receptors are cloned into a modified pHR'SIN:CSW vector containing a PGK promoter for all primary T cell experiments. The pHR'SIN:CSW vector is also modified to make the response element plasmids. Five copies of the Gal4 DNA binding domain target sequence (GGAGCACTGTCCTCC GAACG) are cloned 50 to a minimal CMV promoter. Also included in the response element plasmids is a PGK promoter that constitutively drives mCherry expression to easily identify transduced T cells. For inducible HER2-CAR vectors, the CARs are cloned via a BamHI site in the multiple cloning site 30 to the Gal4 response elements. All constructs are cloned via in fusion cloning (Clontech #ST0345).

Pantropic VSV-G pseudotyped lentivirus is produced via transfection of Lenti-X 293T cells (Clontech #11131D) with a pHR'SIN:CSW transgene expression vector and the viral packaging plasmids pCMVdR8.91 and pMD2.G using Fugene HD (Promega #E2312). Primary T cells are thawed the same day and, after 24 hours in culture, are stimulated with Human T-Activator CD3/CD28 Dynabeads (Life Technologies #11131D) at a 1:3 cell:bead ratio. At 48 hours, viral supernatant is harvested and the primary T cells are exposed to the virus for 24 hours. At day 4 after T cell stimulation, the Dynabeads are removed, and the T cells expanded until day 9 when they are rested and could be used in assays. T cells are sorted for assays with a Beckton Dickinson (BD) FACs ARIA II. AND-gate T cells exhibiting basal CAR expression were gated out during sorting.

### 14. Cancer cell lines

The cancer cell lines used are K562 myelogenous leukemia cells (ATCC #CCL-243) and A375 malignant melanoma cells (ATCC #CRL-1619). K562 and A375 are lentivirally transduced to stably express human HER2 and GFP. All cell lines were sorted for expression of the transgenes. K562 cells are cultured in Iscove's Modified Dulbecco's Medium with 10% fetal bovine serum, and A375 cells are cultured in Dulbecco's Modified Eagle's Medium with 10% fetal bovine serum.

### 15. T cell in vitro stimulation and proliferation assay

For all *in vitro* synNotch T cell assay, 2.5 × 10⁴ T cells are co-cultured with target cancer cells at a 1:1 ratio, together with PLGA microparticles at 0.075 OD₅₅₀ × 200 µL final (composed of 100 µL T cell medium and 100 µL cancer cell medium). After mixing the T cells and cancer cells in round bottom 96-well tissue culture plates, the cells are centrifuged for 2 min at 300 g to force interaction of the cells. The cultures are analyzed at 24 hours for markers of activation (e.g., CD69) for T cells. For proliferation assay, T cells are pre-stained with CellTrace Violet Cell Proliferation Kit (Invitrogen #34557) before the co-culturing, and analyzed at 96 hours. All flow cytometry analysis was performed in FlowJo software (TreeStar).

### 16. Cytokine analysis

Primary CD4+ or CD8+ synNotch AND-Gate T cells are stimulated with the target cancer cell line and PLGA microparticles as described above for 48 hours and supernatant is harvested. IL-2 levels in the supernatant of CD4+ T cells are determined via IL-2 ELISA (eBiosciences #BMS2221HS), and IFN gamma levels from CD8+ T cells are determined via IFN gamma ELISA (Invitrogen, #KHC4021).

### 17. In vitro target cell killing assay

For all *in vitro* target cell killing assay, 2.5 × 10⁴ A375 cells are seeded on flat-bottom 96-well tissue culture plate and cultured for 8 hours to settle, then CD8+ T cells are co-cultured with cancer cells at 1:1 ratio, together with PLGA microparticles at 0.075 OD₅₅₀ × 200 µL final. 48 hours later, cells are gently washed with PBS for 2 times, and PrestoBlue Cell Viability Reagent (Invitrogen #A13262) is added to analyze the cell viability.

### 18. In vivo tumor targeting

NSG mice are implanted with two xenograft tumors - 5 × 10⁶ GFP+ K562 tumor cells subcutaneously on the left and right flank, respectively. Seven days after tumor implantation, 5 × 10⁶ primary human CD4+ and CD8+ T cells (1 × 10⁷ total T cells) are injected intravenously into the mice. These T cells were either untransduced (control) or engineered with the a-GFP synNotch Gal4VP64 receptor and the corresponding response elements regulating HER2 4-1BBδ CAR expression. Functionalized PLGA microparticles are injected intratumorally at one side of the two flanks, leaving the other as the control. Tumor size is monitored via caliper over 20 days after T cell injection. For Kaplan-Meier experiments, the same protocol is used but single tumors are injected into the mice. Mice are considered dead when the tumor size reaches euthanasia criteria.

### EXAMPLE 1: MULTI-FUNCTIONALIZATION OF PLGA MICROPARTICLES USING DNA SCAFFOLDS

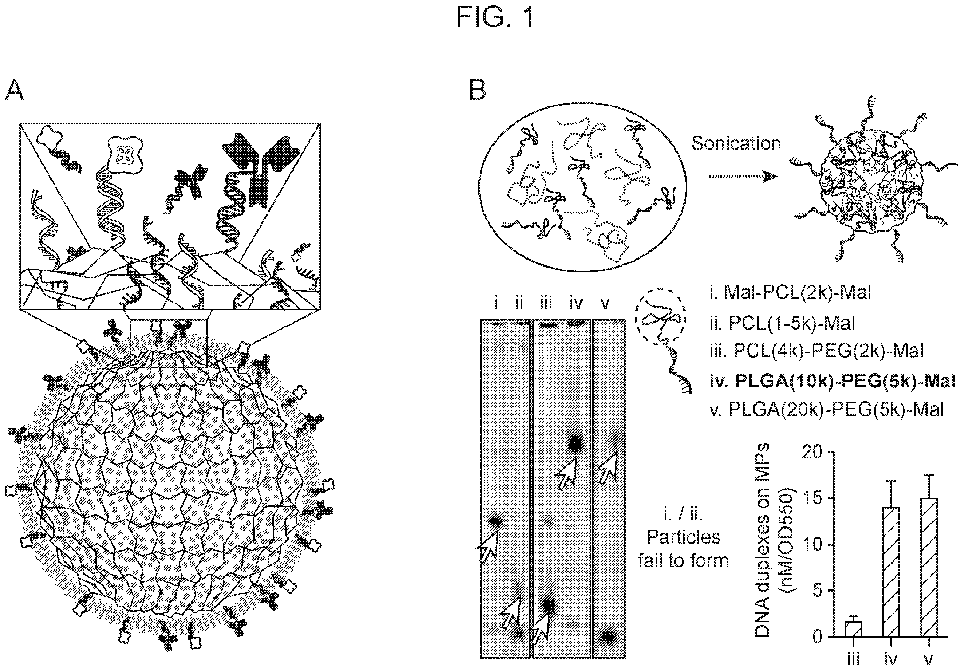

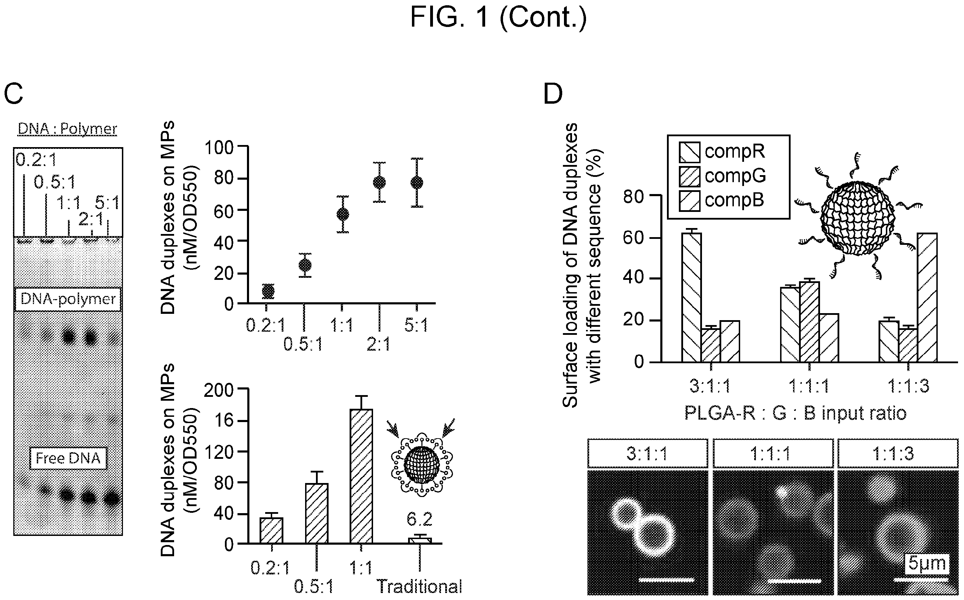

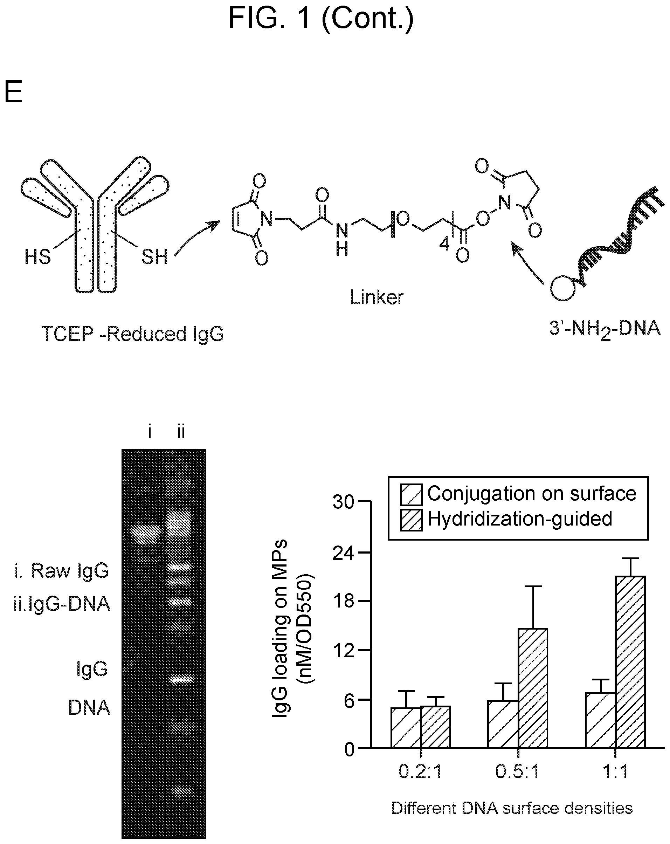

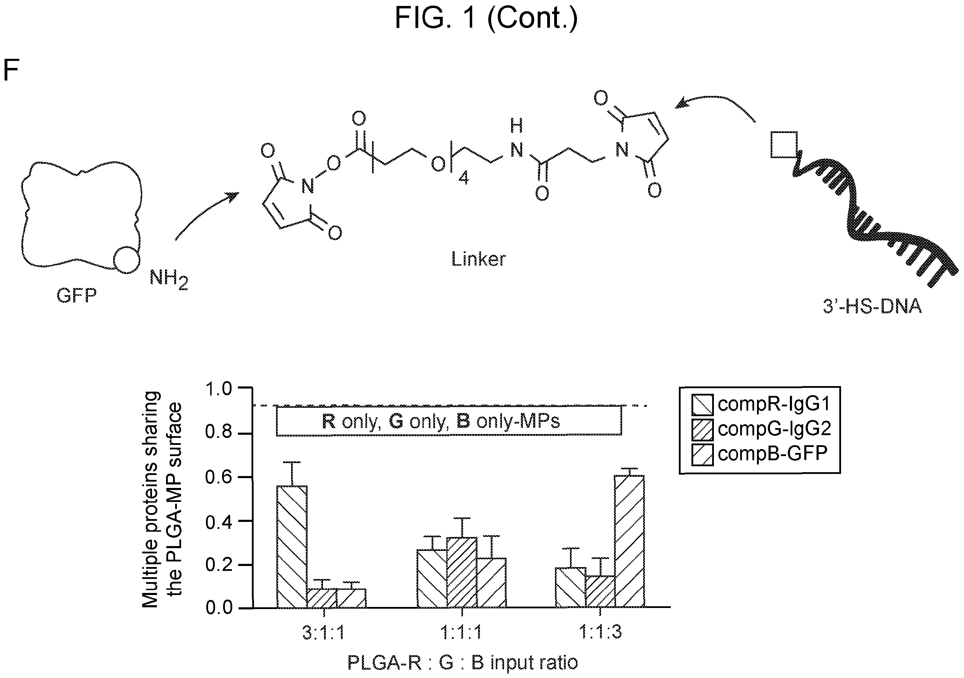

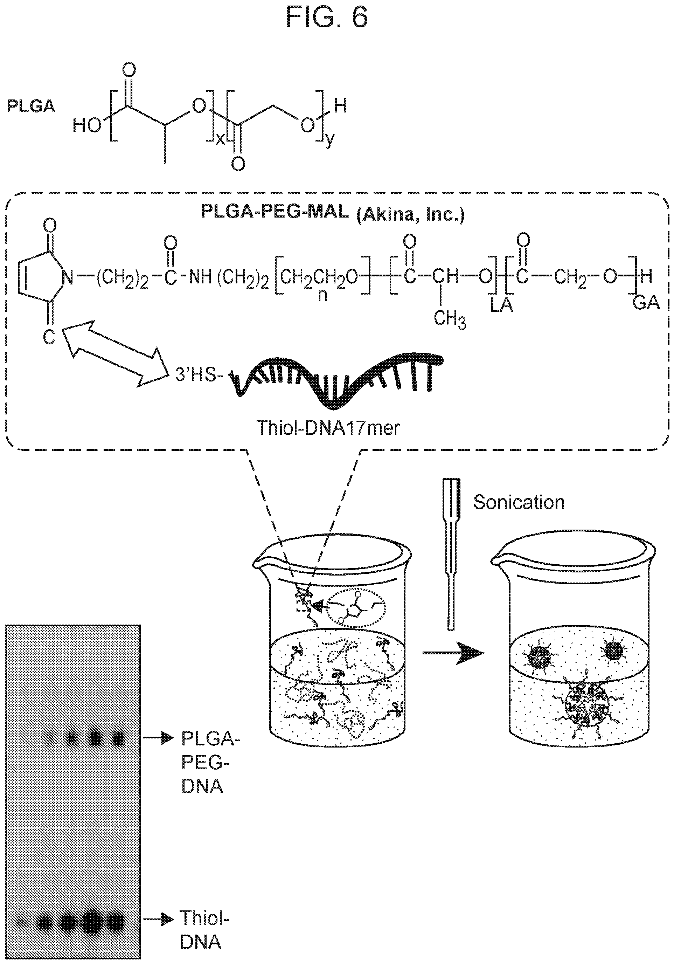

**Figure 1****.** The multi-functionalization of PLGA microparticles using DNA scaffolds. **(a)** Schematic of versatile therapeutic proteins loading on biodegradable polymeric microparticles surface through DNA scaffolds. Therapeutic proteins pre-conjugated with DNA strands complementary to scaffolds on particles are assembled on surface through DNA hybridization, **(b)** The synthesis of amphiphilic block-co-polymer for the fabrication of PLGA microparticles and the DNA scaffold presentation. Thiolated DNA and maleimide (Mal)-functionalized polymer are conjugated through the Michael addition chemistry to form amphiphilic co-polymer (see also Figure 6), followed by the mixture with PLGA polymer (Resomer, 38-54 kDa, 50:50) and sonication-mediated emulsion to generate microparticles. PLGA(10k)-PEG(5k)-Mal (PolySciTech Inc.) with thiolated DNA 17mer is optimal for microparticles formation and efficient DNA presentation. (c) Surface density of DNA scaffolds can be controlled by different ratios of DNA to polymer during the conjugation. The efficiency of biomolecule loading through this strategy is significantly higher than the current best surface conjugation chemistry. **(d)** DNA scaffolds with different sequences can be adjusted at intended ratios on surface for versatile cargo loading. The fluorescent images of particles hybridized with different dye-labeled complementary strands match with the color (bar above the images) generated from chemistry analysis. **(e)** The modification of complementary DNA to antibodies and their surface loading strategy. Antibodies are treated with TCEP to selectively reduce the hinge region disulfide bond to conjugate with amine-functionalized DNA through a MAL-PEG-NHS linker. The loading of DNA-pre-conjugated antibodies through hybridization shows higher efficiency than step-by-step chemical conjugation on surface. **(f)** The loading of multiple protein targets at controlled ratios. GFP proteins are modified with DNA utilizing free amines.

### EXAMPLE 2: ACTIVATION OF SYNNOTCH T CELLS THROUGH BIODEGRADABLE POLYMERIC MICROPARTICLES FOR ANTIGEN-SPECIFIC CANCER TARGETING

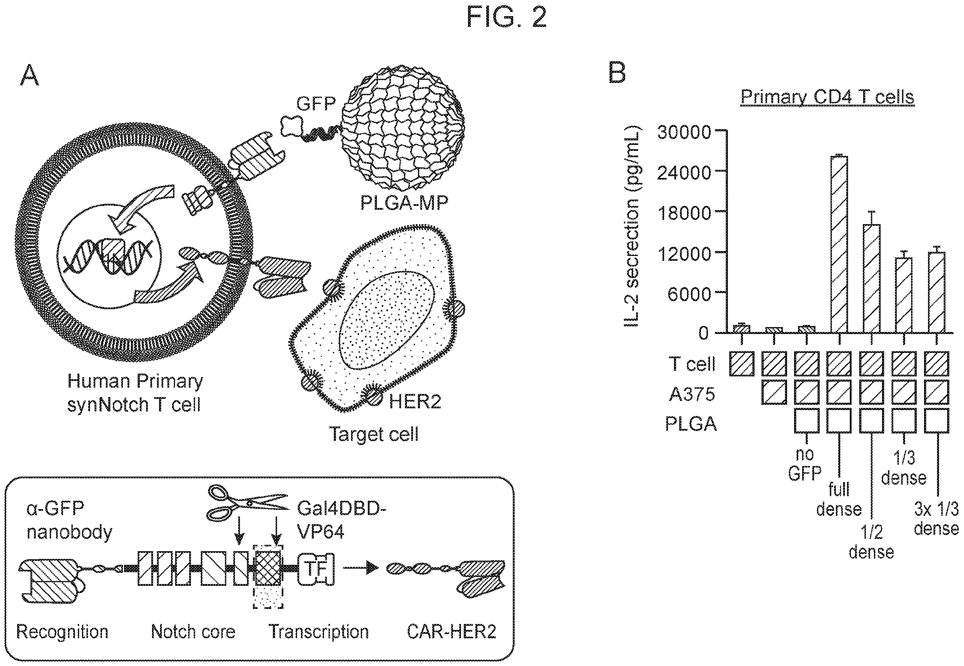

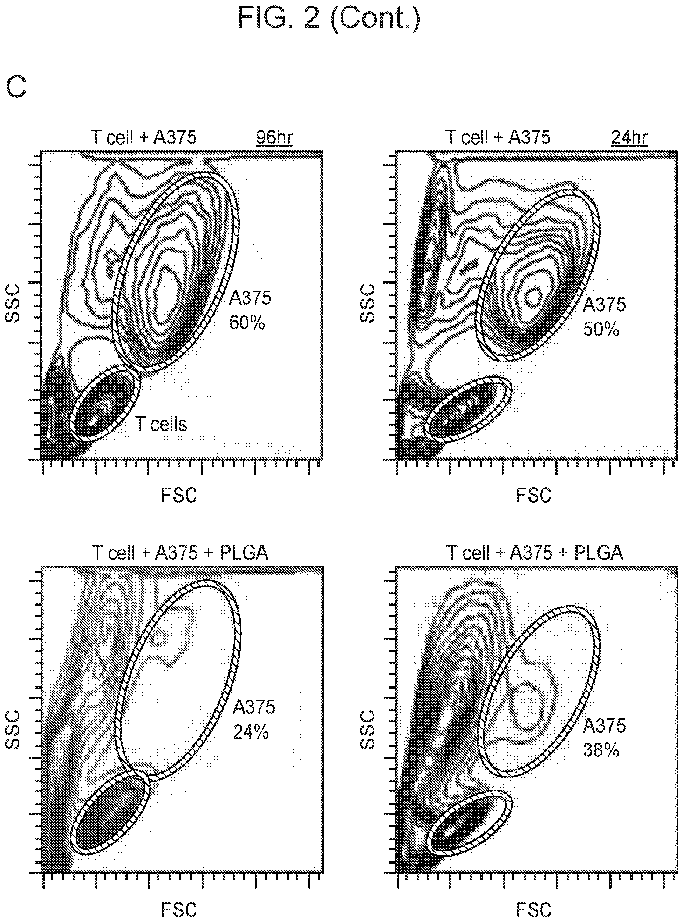

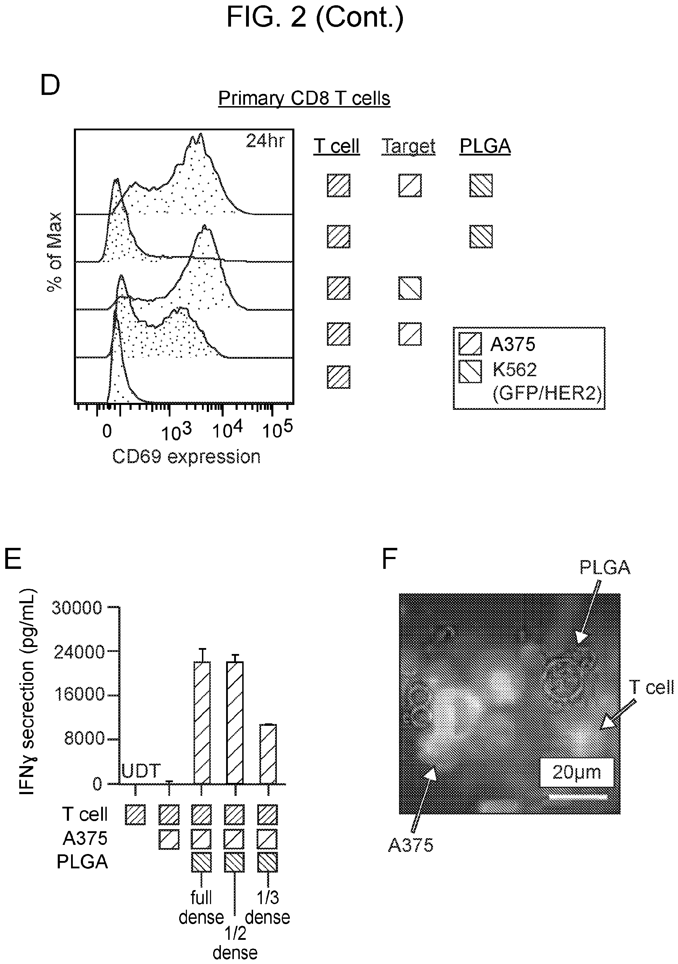

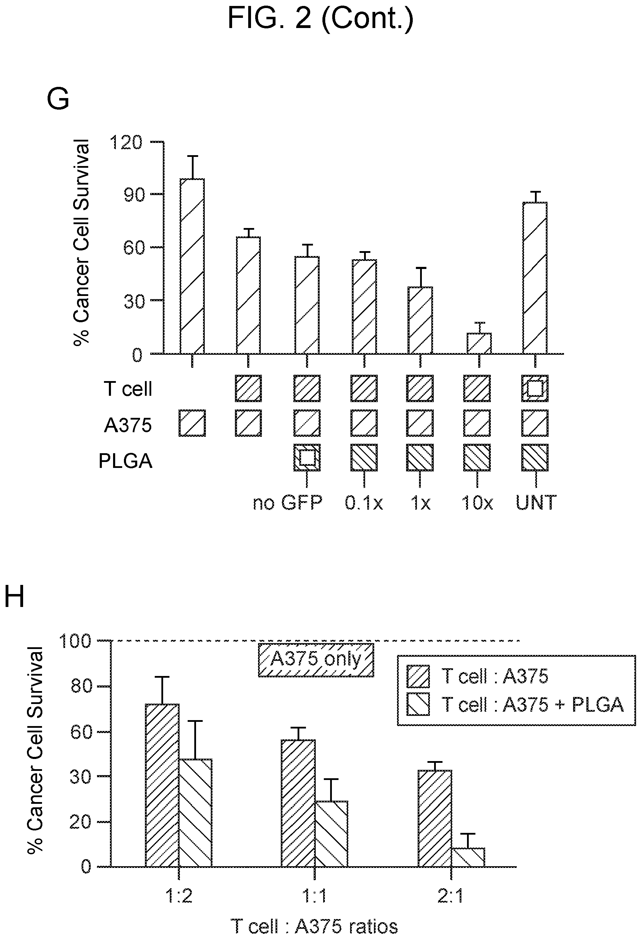

**Figure 2****.** Activation of synthetic circuit human primary T cells through biodegradable polymeric microparticles for antigen-specific cancer targeting. **(a)** Schematic of the priming of synthetic Notch T cells by GFP-presented PLGA microparticles for CAR expression to target HER2 antigens on cancer cells. The primary T cells are engineered with a new class of modular synthetic Notch (synNotch) receptor with an extracellular recognition domain to bind GFP, and a transcriptional activator domain, being released to activate the expression of HER2-CAR once the extracellular domain binds. **(b)** The co-incubation of GFP-presenting PLGA microparticles with human primary CD4 T cell and HER2-overexpressed melanoma cells A375 leads to the secretion of IL-2 cytokine, and more secreted with the increase of GFP density on particles. **(c)** A375 cells lose the viability as the CD4 synNotch T cells are primed by PLGA-GFP particles, largely due to the boosted level of cytokines. **(d)** The priming of CD8 synNotch T cells by PLGA-GFP and the CAR binding by A375 leads to the expression of early activation marker CD69. (e) The co-incubation of GFP-presenting PLGA microparticles with human primary CD8 T cell and HER2-overexpressed melanoma cells A375 leads to the secretion of interferon gamma cytokine, and more secreted with the increase of GFP density on particles. **(f, g, h)** CD8 synNotch T cells primed by GFP-presenting PLGA microparticles target and kills HER2 positive cancer cells. (i) Cell survival test of target Her2+ A375 target cells after 2 days of treatment with a different type of synNotch CAR-T cells engineered with a synNotch receptor targeting the PNE peptide with ("PNE+ ICEp") or without ("ICEp-"off"") addition of PNE-presented microparticles. Data are mean ± s.d. (n = 4 biologically independent samples). Killing was significantly enhanced by PNE+ ICEp addition.

The co-incubation of target cells with synNotch CAR-T cells in the absence of microparticles ICEp also showed moderate cytotoxicity **(****Fig. 2g** and Fig. 2i), which is likely due to the leakiness of inducible CAR expression also observed from previous reports and can be improved through further optimization of synNotch receptors.

### EXAMPLE 3: MULTI-FUNCTIONALIZATION OF PLGA MICROPARTICLES AND THEIR IMPACT ON PRIMARY SYNNOTCH T CELL ACTIVITY

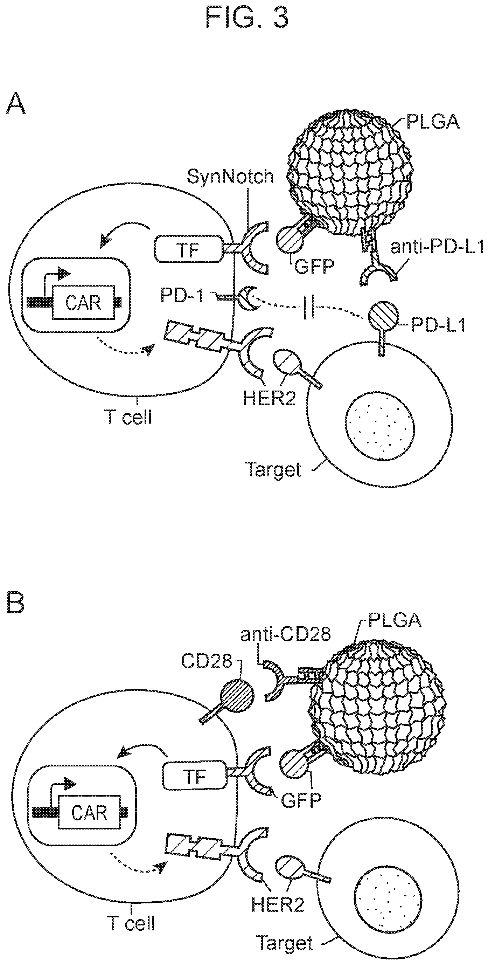

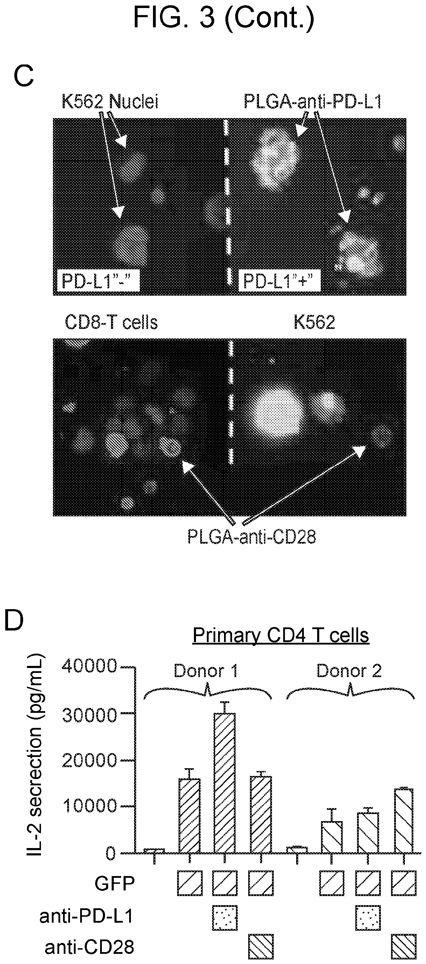

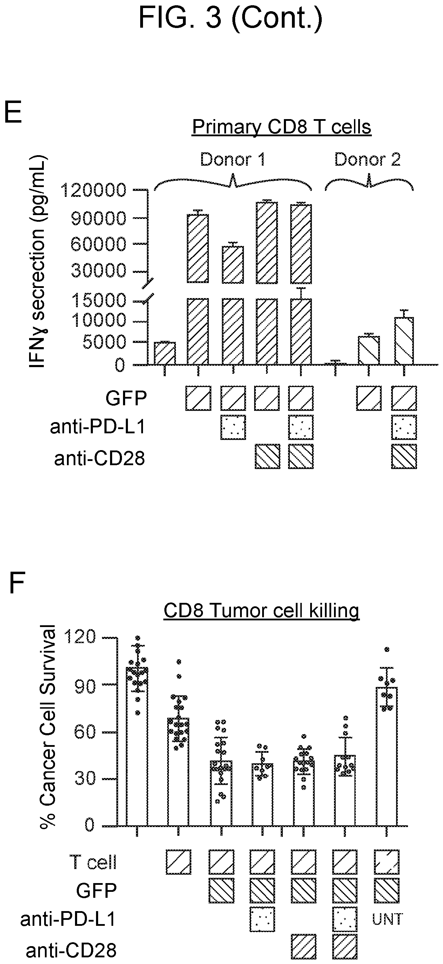

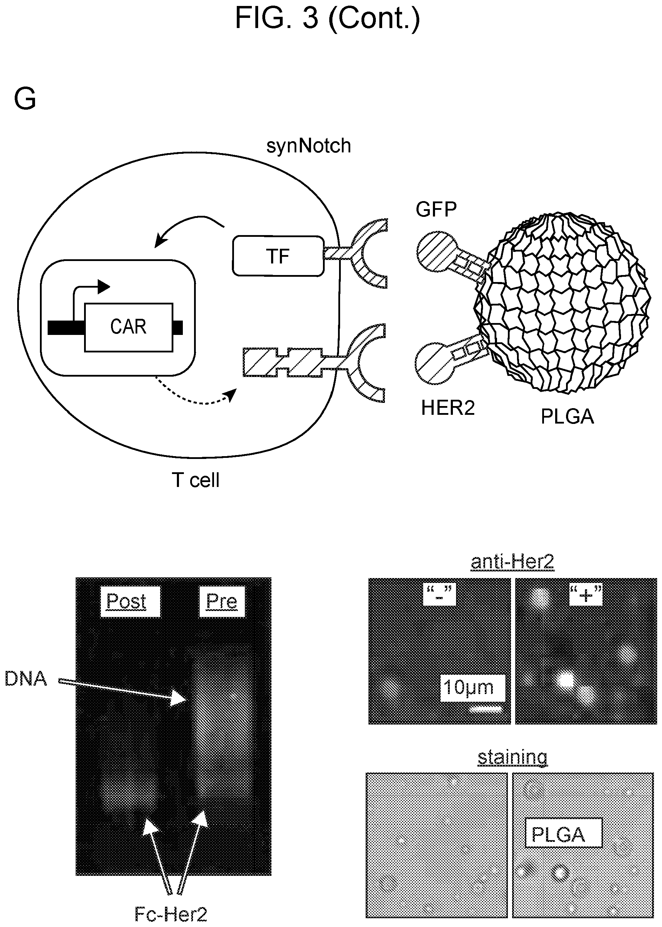

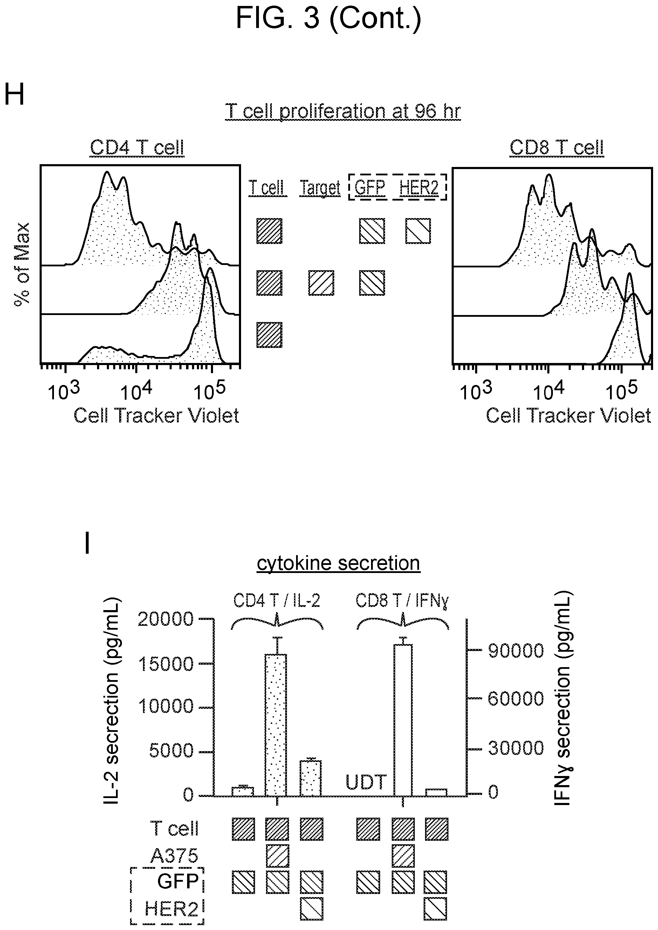

**Figure 3****.** Multi-functionalization of PLGA microparticles and their impact on primary synNotch T cell activity. **(a, b)** Immune checkpoint inhibitor anti-PD-L1 and T cell co-stimulatory agonist anti-CD28 antibodies are loaded on GFP-presenting PLGA microparticles to enhance the T cell activity towards target cells. (c) Anti-PD-L1 and anti-CD28 antibodies loaded PLGA microparticles bind efficiently with PD-L1 positive cancer cells and CD8 primary T cells, respectively. **(d, e)** The impact of anti-PD-L1 and anti-CD28 antibodies co-presenting PLGA microparticles to the cytokines secretion profile of CD4 and CD8 synNotch T cells varies among different patient donors, **(f)** Multi-functionalization of PLGA microparticles does not show apparent advantage on target killing for *in vitro* assays. **(g)** HER2 antigens are co-loaded on PLGA microparticles to bind with CAR once primed by GFP. **(h)** CAR antigens present on PLGA microparticles prompt more proliferation of CD4 and CD8 T cells than antigens provided by target cells A375, but with much lower cytokine secretion (i).

### EXAMPLE 4: STABILITY OF DNA SCAFFOLD ON PLGA MICROPARTICLES

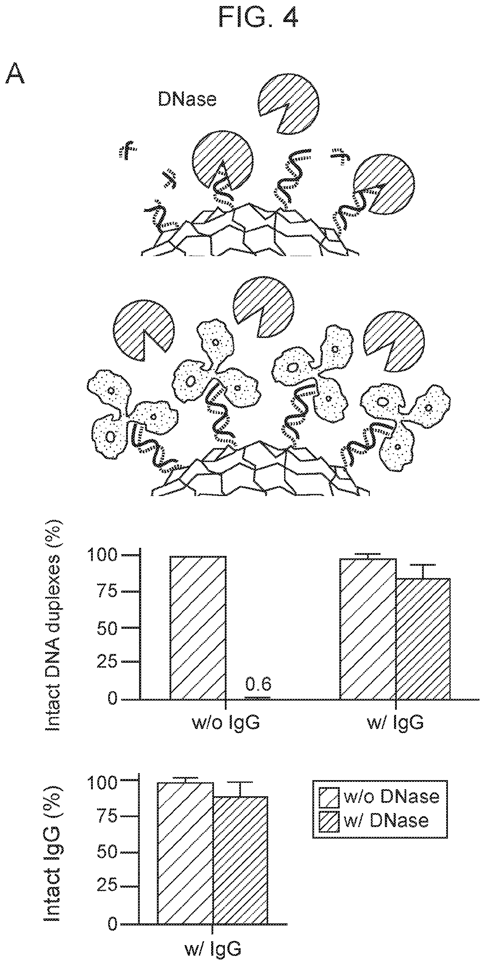

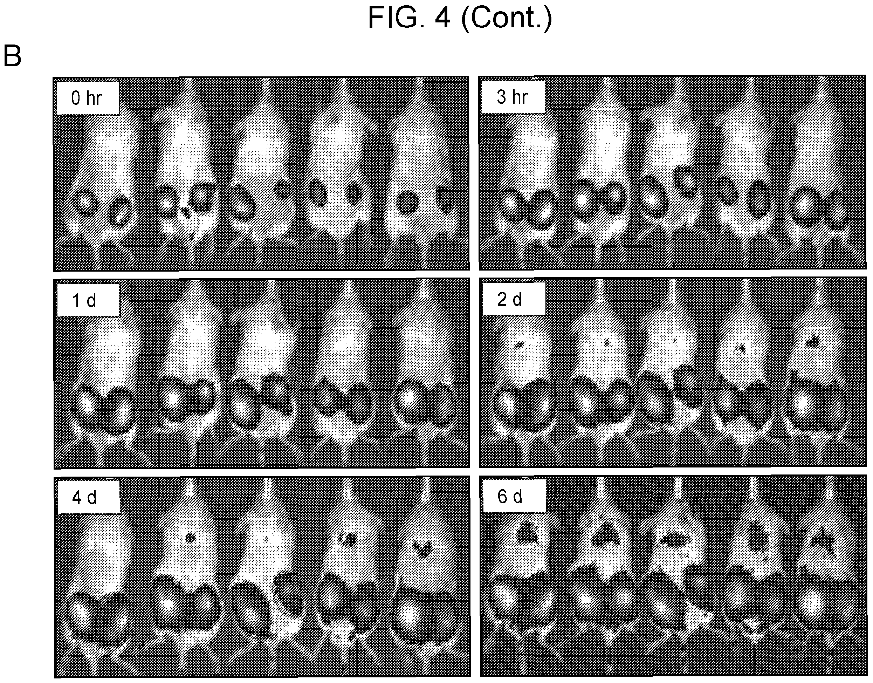

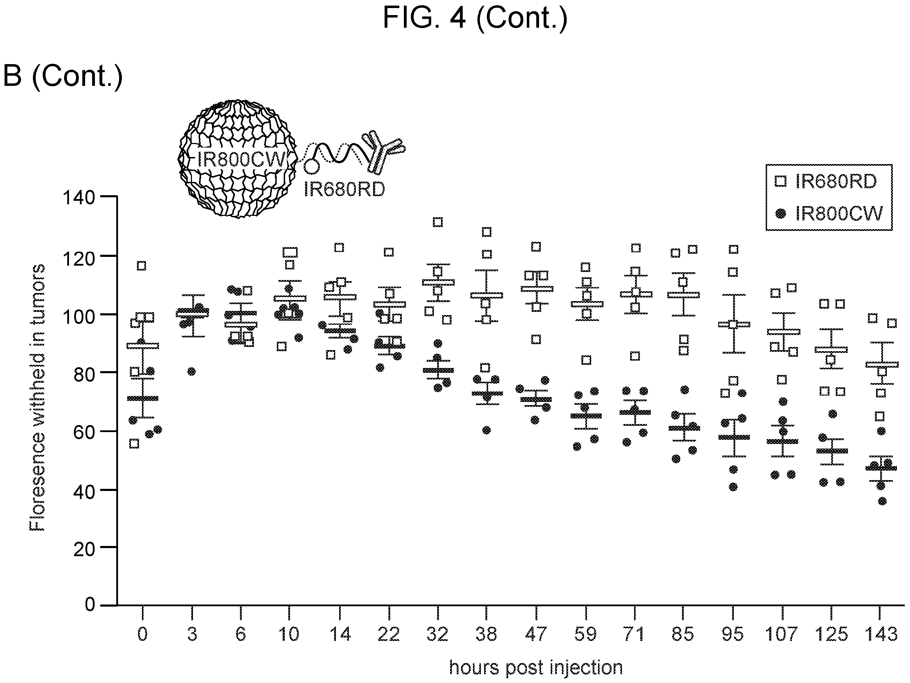

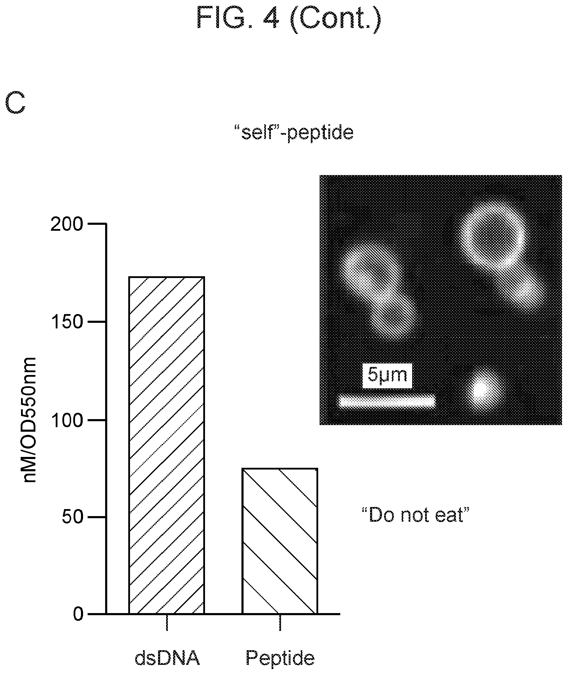

**Figure 4****.** Stability of DNA scaffold on PLGA microparticles. **(a)** DNA scaffolds are protected from enzymatic degradation once loaded with proteins (i.e. antibodies) *in vitro.* **(b)** The DNA scaffolds are relatively stable for a week in K562 tumor of NSG mice. PLGA microparticles with near-infrared dyes labeled on surface DNA and in the core are tracked the intensity by IVIS imaging once injected intratumorally. **(c)** "Self'-peptides are functionalized on PLGA microparticles as "do not eat me" signal to prevent the uptake by macrophages.

### EXAMPLE 5: LOCAL ACTIVATION OF SYNNOTCH CAR T CELLS FOR HER2-SPECIFIC ANTIGEN TARGETING BY INTRATUMORAL INJECTION OF PLGA PARTICLES

PLGA particles functionalized with binding members that specifically bind to synNotch CAR T cells for activation of expression of a HER2-specific CAR will be injected into mice implanted with HER2 expressing tumors. Mice will be injected with HER2 expressing tumor cells via tail vein. After establishment of tumors, at about 14 days, synNotch T cells or control untransduced (e.g., CD4/CD8 T cells) will be injected intravenously into mice via the tail vein. Mice will then receive intratumoral injections of functionalized PLGA particles presenting the synNotch antigen. Tumor size and/or growth will be monitored until mice reach euthanasia criteria at about 28 days.

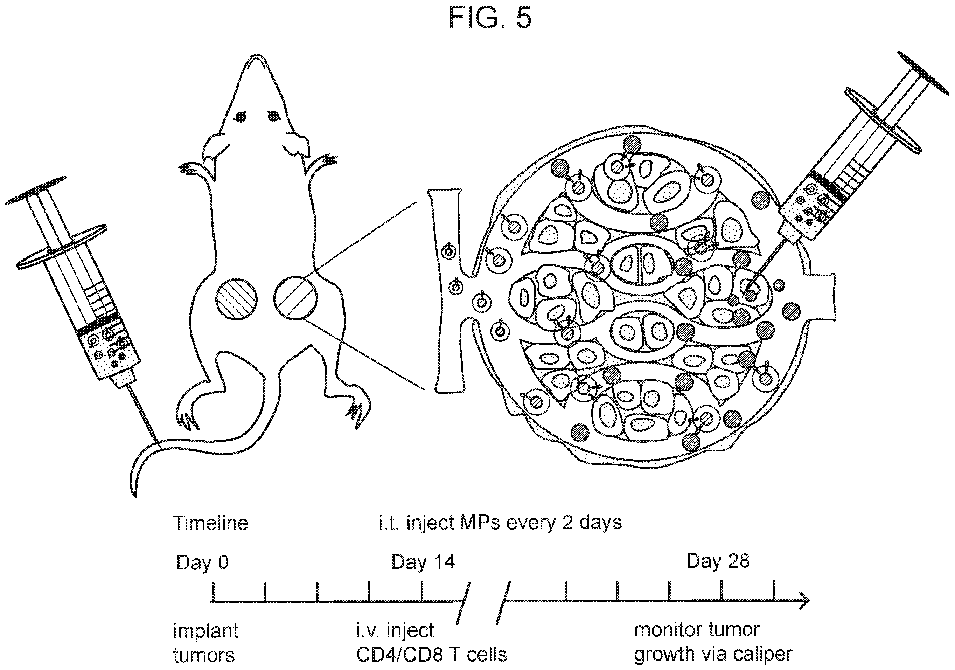

**Figure 5****.** Local activation of synNotch CAR T cells for HER2-specific antigen targeting by intratumoral injection of PLGA particles.

### EXAMPLE 6: DESIGN OF PARTICLES WITH RATIOMETRICALLY CONTROLLED MOIETIES FOR EX VIVO HUMAN T CELL ACTIVATION

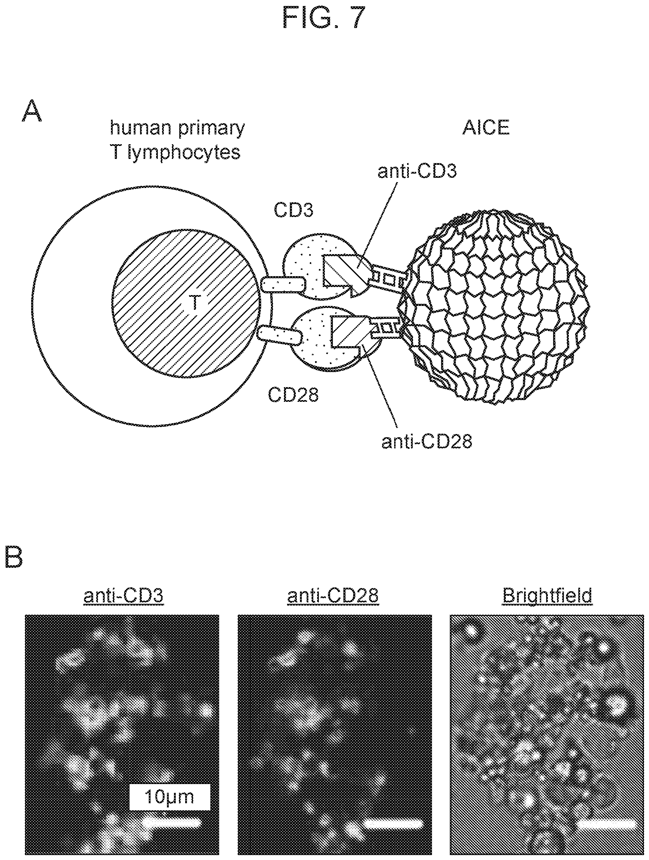

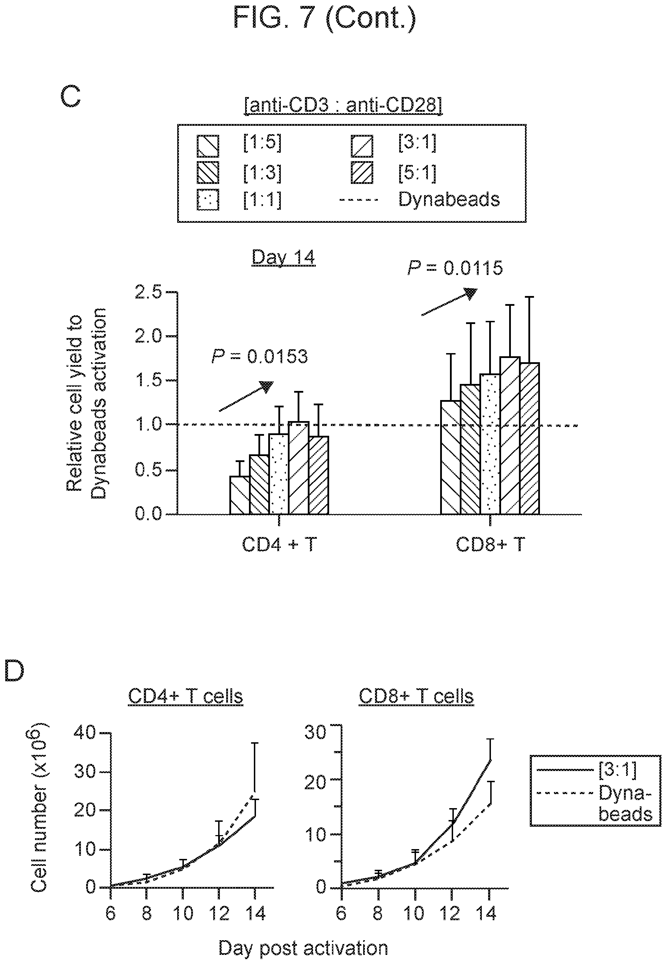

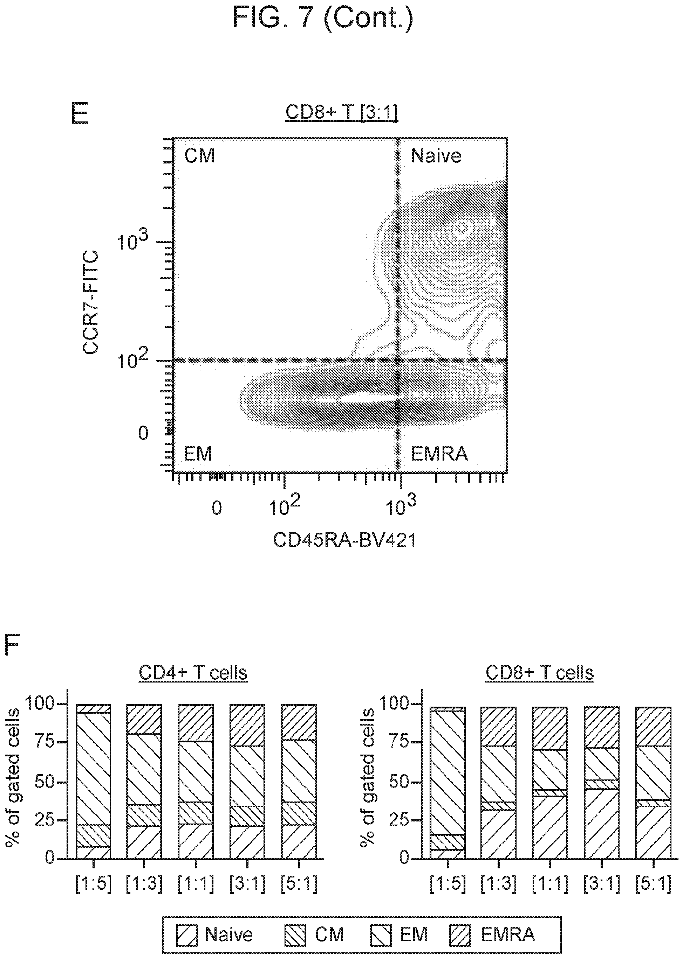

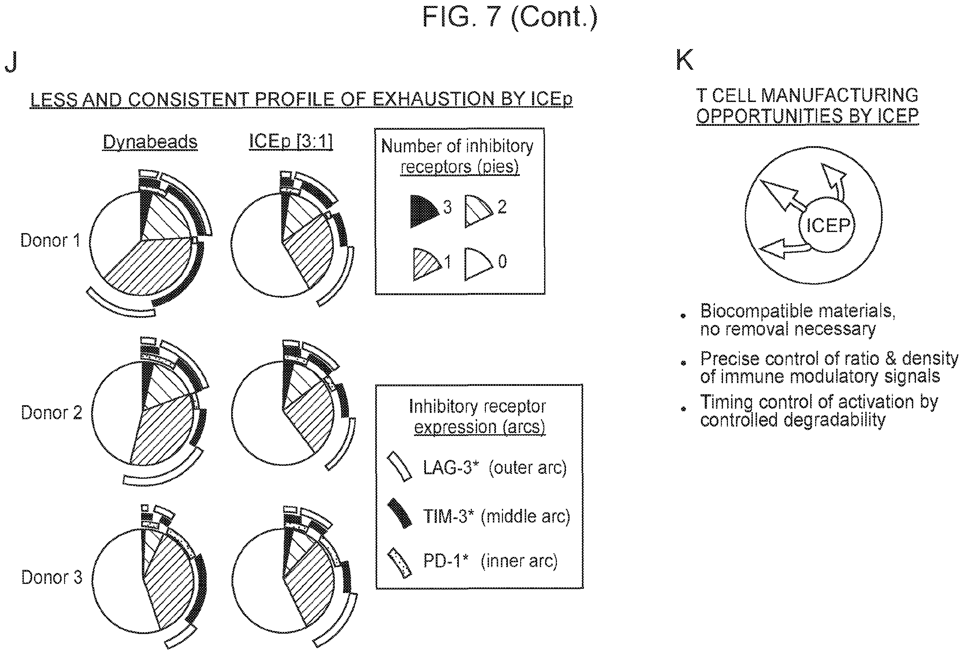

Polymeric particles (e.g. PLGA particles) loaded with proteins are referred to interchangeably herein as artificial immune cell engager (AICE) particles and immune cell engaging particles (ICEp). We loaded anti-CD3 and anti-CD28 antibodies on AICE particles-at varying ratios from 1:5 to 5:1. Anti-CD3/CD28 antibodies loaded AICE particles were benchmarked against commercial Dynabeads loaded with both anti-CD3 and anti-CD28 antibodies at the same particle to cell ratio to compare their ability to expand human primary T cells with minimized exhaustion **(****Fig. 7A****).** AICE particles loaded with anti-CD3/ anti-CD28 antibodies ("functionalized AICE") activated T cells **(****Fig. 7B****)** and yielded higher or equivalent T cell expansion when compared to Dynabeads expansion across three human T cell donors **(****Fig. 7C**,7D). Although there was large donor-to-donor differences (also observed for Dynabeads), we observed a linear trend of cell yield increase from AICE-[1:5, anti-CD3:anti-CD28] to AICE-[3:1, anti-CD3:anti-CD28] for both CD4+ and CD8+ Tcells at day 14 **(****Fig. 7C****).** The phenotype of expanded T cells at day 14 was then explored by measuring expression of CD45RA and CCR7 surface markers **(****Fig. 7E****).** Interestingly, the population distribution of the 4 differentiation states, including naive, central memory (CM), effector memory (EM) and terminally differentiated central memory (EMRA), displayed a pattern **(****Fig. 7F****)** corresponding to the cell expansion trend among AICE with different anti-CD3 to anti-CD28 ratios **(****Fig. 7C****).** Through staining for T cell exhaustion markers LAG-3, PD-1 and TIM-3, we found that the population of exhausted cells at the optimal condition AICE-[3:1, anti-CD3:anti-CD28] was consistently lower than those activated by Dynabeads among the three donors **(****Fig. 7J****).** All these data demonstrate that the surface ratio control of functional moieties on synthetic materials is essential for the quantity and quality of cell yield from ex vivo T cell expansion.

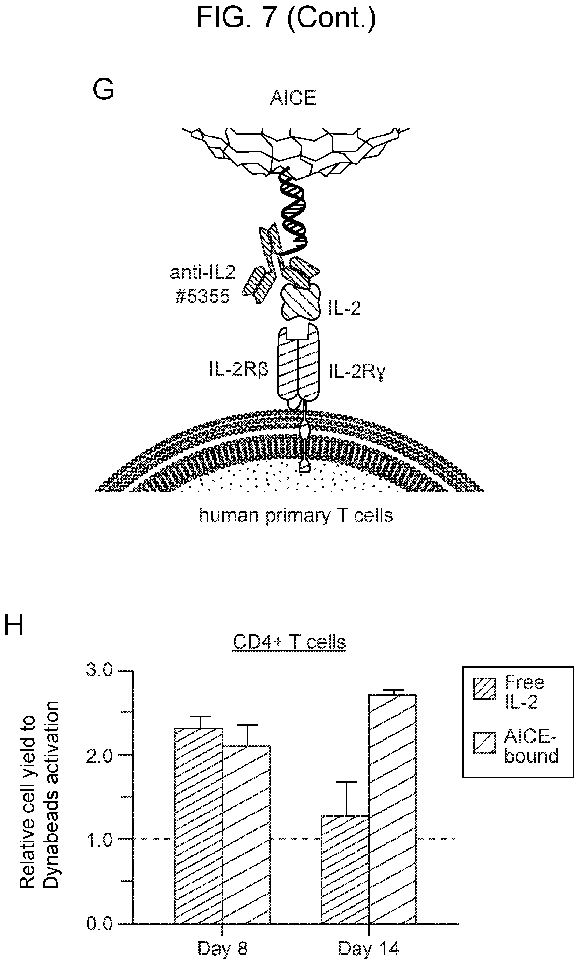

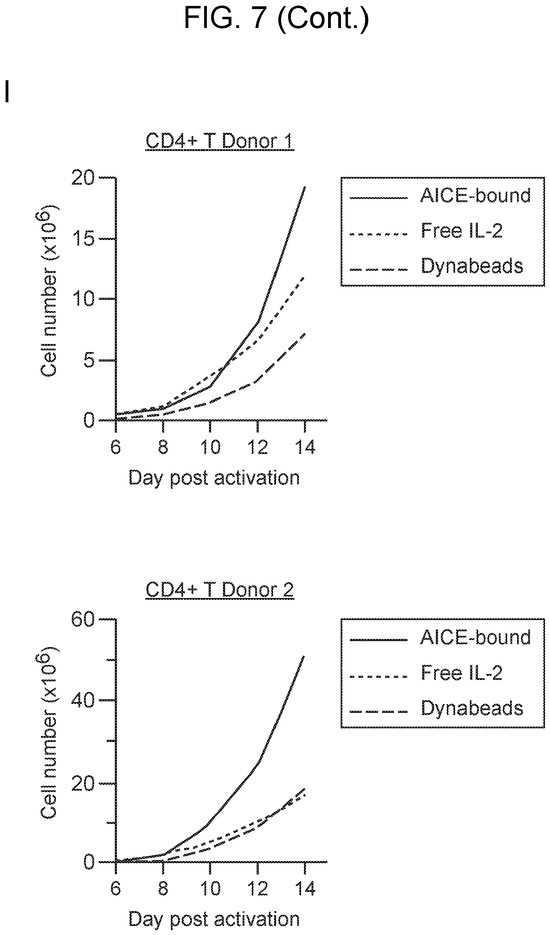

As an alternative to the routine protocol of supplementing free IL-2 in the media for *ex vivo* T cell culture, we loaded IL-2 on AICE through the surface presentation of its antibody clone #5355 **(****Fig. 7G****).** This particular anti-IL-2 antibody was engineered to facilitate the binding of IL-2 to its β and γ receptor on T cells thus promoting the proliferation of non-Treg T cells **(****Fig. 7G****).** Based on the optimal condition of AICE[3:1], we then compared the influence to primary T cell expansion through two ways of same amount of IL-2 dosing: free versus surface bound. Surface IL-2 loading enhanced CD4+ and CD8+ T cell expansion, and particularly improved expansion of CD4+ primary T cells after day 8 **(****Fig. 7H**,7I), compensating for the comparative decrease of yield at day 14 with free IL-2. Additionally, the populations of LAG-3 and PD-1 positive CD4+ T cells were reduced in the two tested donors, demonstrating that loading cytokines on the surface of AICE has the potential to reduce T cell exhaustion during *ex vivo* expansion.

Taken together, this platform using biocompatible and biodegradable materials with precise control of immune modulatory signals can provide new opportunities for optimization in T cell manufacturing for adoptive cell therapy (ACT) (Fig. 4K).

**Figure 7A-7K****.** AICE with ratiometrically controlled moieties for human primary T lymphocytes *ex vivo* expansion. **(a)** Schematic of AICE (in this figure PLGA microparticles loaded with CD3 and CD28 antibodies of varying ratios from [1:5] to [5:1]) for primary T cell expansion, compared with commercial available T cell expander Dynabeads (Invitrogen)). **(b)** Representative confocal microscopy images of human primary CD8+ T cell co-incubated with AICE [1:1] for overnight, showing AICE-induced cell clumps (n = 3 biologically independent samples). (c) Cell yield of CD4+ and CD8+ T cells at day 14 post the activation by AICE with CD3 and CD28 antibodies at different ratios in (a), relative to Dynabeads. Data are mean ± s.e.m., and *P* values were determined by one-way ANOVA test for linear trend (n = 3 independent donors of two independent experiments). **(d)** Growth curve of primary T cells from three different donors upon the activation by AICE [3:1], compared to Dynabeads control. Data are mean ± s.e.m. (n = 3 independent donors of two independent experiments). **(e)** Gating strategy through CCR7 and CD45RA expression levels for distinguishing cells at specific differentiation stages: naive, central memory (CM), effector memory (EM), and terminally differentiated effector memory (EMRA). **(f)** Differentiation profile of T cells 14 days post the activation by AICE of varying ratios of surface moieties. Data are mean of n = 3 independent donors. **(g)** Schematic of IL-2 presented on particles through its antibody (clone5355) exposes the epitope for β and γ units of its receptor on T cells, promoting the cell proliferation. **(h)** Cell yield of primary CD4+ T cells activated by AICE [3:1] with surface-bound IL-2 or free IL-2 at day 8 and 14, relative to the Dynabeads method. Data are mean ± s.d. (n = 2 independent donors). **(i)** Growth curve of CD4+ primary T cells from two different donors treated with AICE [3:1] together with surface bound IL-2 or free IL-2, compared to Dynabeads control. Data are mean of n = 2 technical replicates, (j) Exhaustion marker analysis of CD8+ T cells after stimulation with AICE-[3:1, anti-CD3:anti-CD28] (also termed "ICEp [3:1]") for 14 days shows consistent and reduced exhaustion marker expression profile from all three donors, compared to those activated by Dynabeads. **(k)** AICE have many properties that make them attractive for use in therapeutic T cell manufacturing, as shown by promising T cell expansion achieved here with little optimization.

### EXAMPLE 7: LOCAL TUMOR ERADICATION IN MOUSE MODEL BY THE COMBINATORIAL TREATMENT OF PRIMING PARTICLES AND AND-GATE T CELLS

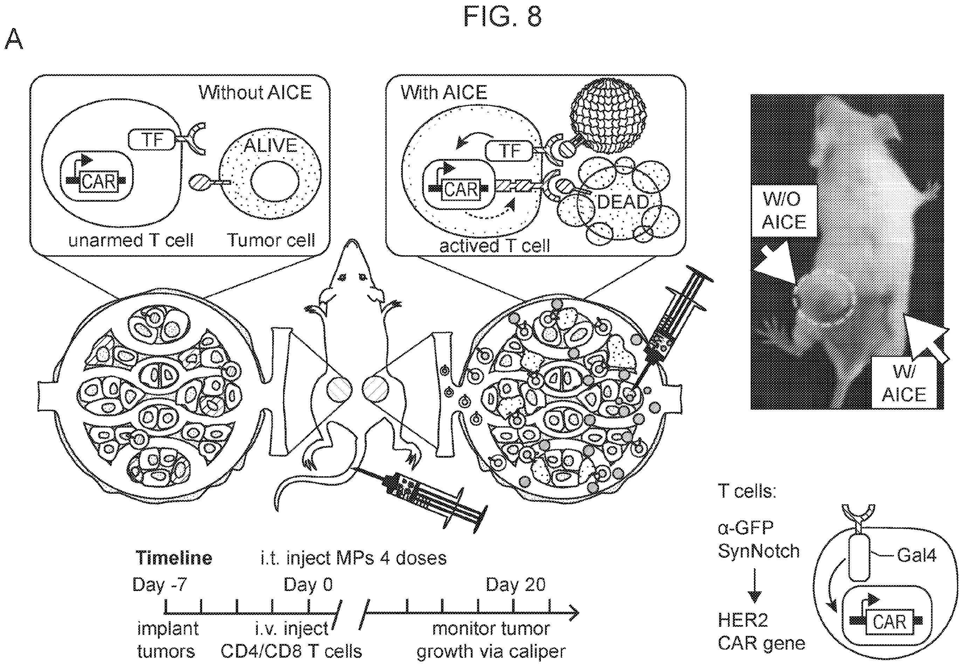

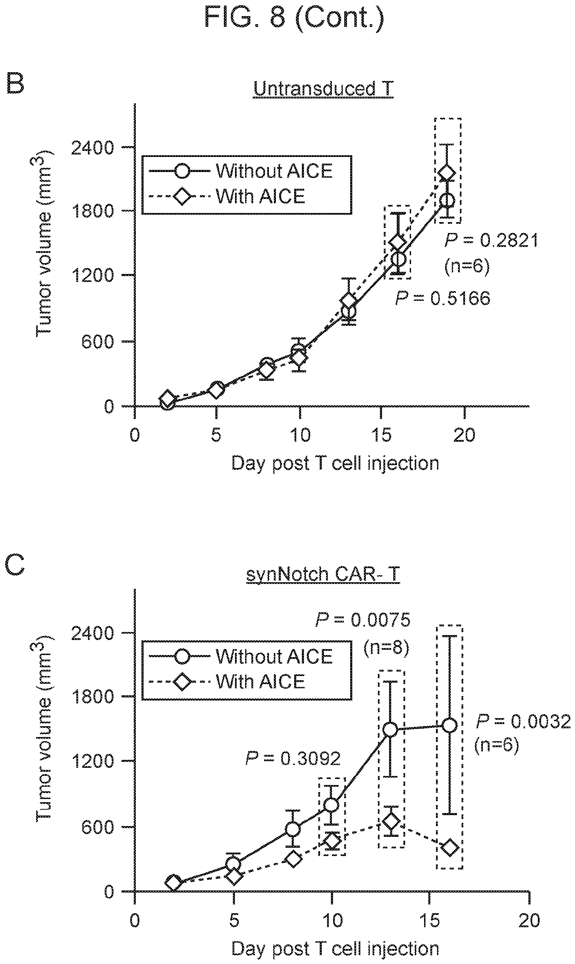

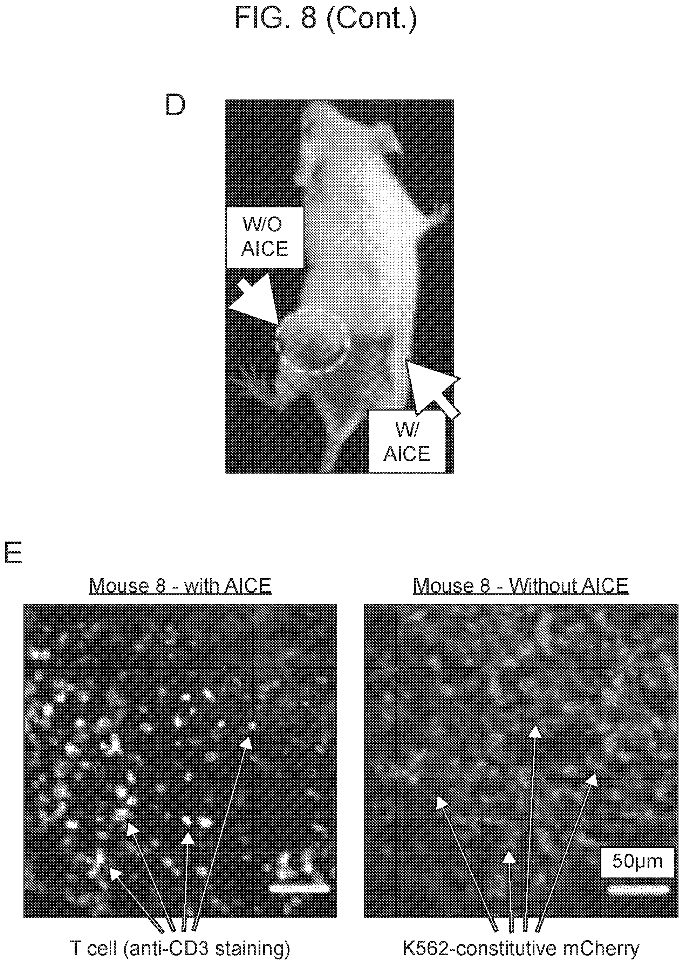

AICE particles (GFP decorated PLGA microparticles) were injected intratumorally as the local activator for systemically administered anti-GFP synNotch/anti-HER2 T cells **(****Fig. 8A****).** NSG mice were implanted subcutaneously with the same HER2-overexpressed K562 xenograft tumors in bilateral flanks as a model for local tumor and distal cross-reactive tissue. After a week, mice were administered with synNotch CAR-T cells intravenously in combination with AICE intratumorally injected into only one tumor, followed by another 3 doses of AICE into the same tumor every 4 days or starting the subsequent dose as the tumor grew over 500 mm³ in volume **(****Fig. 8A****).** The size of AICE-injected tumors decreased over time, in contrast to the distal tumors within the same mice without AICE injection **(****Fig. 8B-8C****)** and mice injected with AICE plus untransduced primary T cells **(****Fig. 8B****).** We also performed fluorescence microscopy on fixed tumor samples of mice sacrificed at an early timepoint and observed selective T cell infiltration in the AICE-injected tumor **(****Fig. 8D****).** These results demonstrate that AICE can provide a spatially controlled signal *in vivo* for the local activation of synNotch CAR-T cells and induction of precision tumor clearance, while sparing attack of potentially cross-reactive distal tissues.

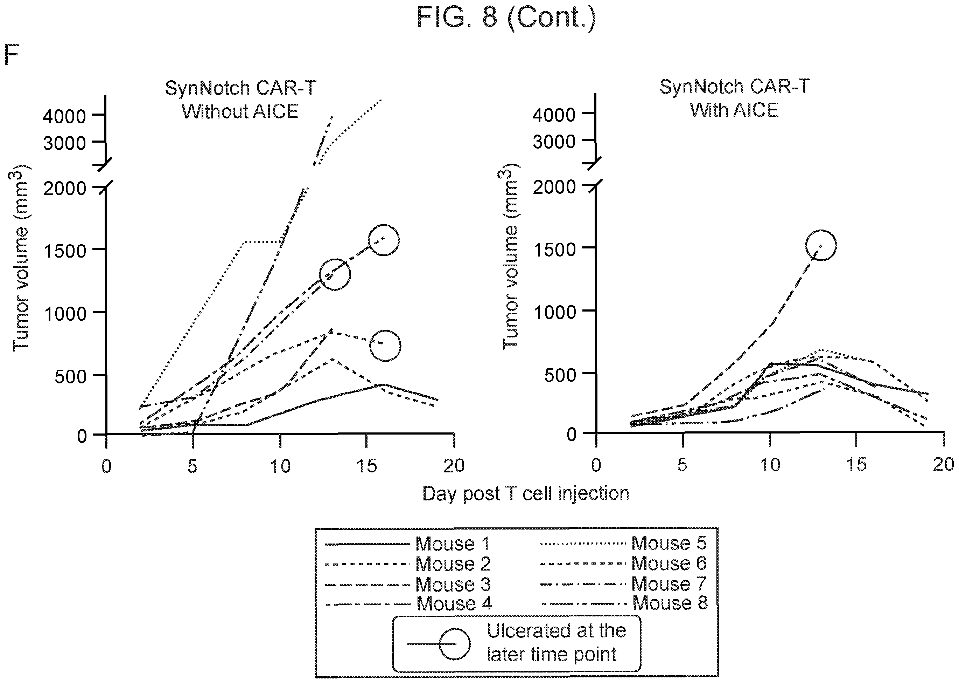

**Figure 8****.** Selective tumor killing *in vivo* by local activation of synNotch CAR-T cells using AICE. Fig. 8A. Schematic of two tumor model for selected clearance by AICE-primed synNotch CAR-T cell activation through local intratumoral injection, and the overall treatment timeline. Fig. 8B. Tumor volume of AICE-injected tumor versus the distal tumor over 19 days post the combinatorial treatment of untransduced primary T cell and GFP-coated AICE. Data are mean ± s.e.m. (n = 6 mice), and *P* values were determined by two-tailed paired t test. Fig. 8C. Tumor volume of AICE-injected tumor versus the distal tumor over 19 days post the combinatorial treatment of synNotch CAR-T cell and GFP-coated AICE. Data are mean ± s.e.m. (n = 8 mice, and 2 mice met the euthanasia criteria at day 13-15), and *P* values were determined by two-tailed paired *t* test. Fig. 8D. Representative image of the mice intravenously injected with synNotch CAR-T cells, showing diminished tumor on AICE injection site but growing tumor at the distal site. Fig. 8E. Representative images of fixed staining of CD3+ T cells in tumors collected from mouse 8 in , which is sacrificed in earlier date meeting euthanasia criteria (n = 10 technical replicates). Tumor volume of individual mice is shown in Fig. 8F.

### EXAMPLE 8: DENSITY-DEPENDENT ACTIVATION OF SYNNOTCH RECEPTOR FOR CYTOKINE RELEASE

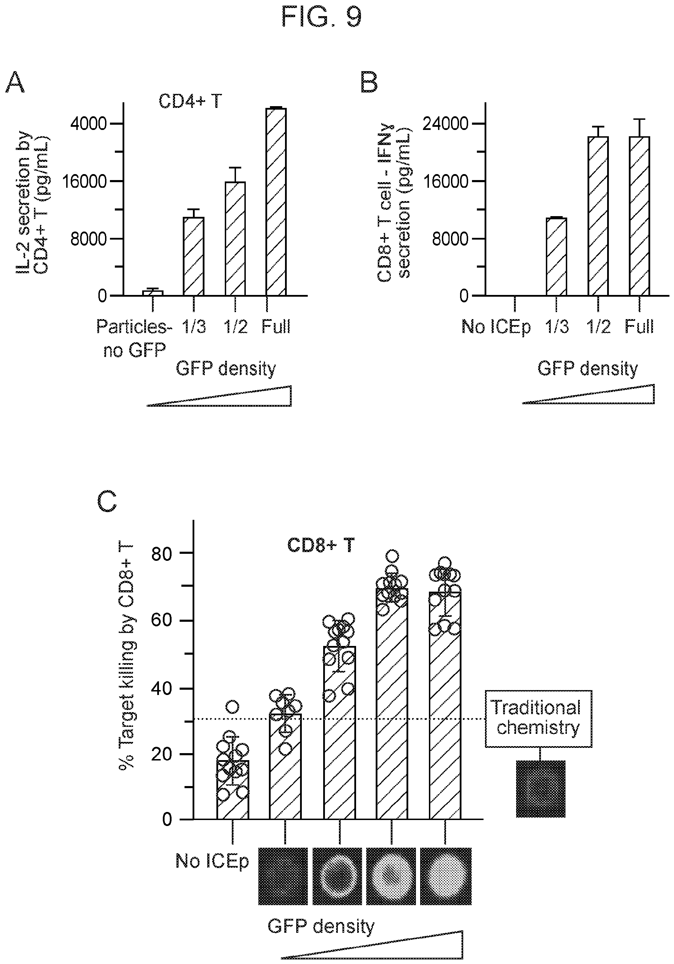

As described in Example 2 above, synNotch T cells showed AND-gate killing behavior, selectively killing HER2+ A375 cells in the presence of GFP-presented PLGA microparticles (termed "AICE" particles; also termed "ICEp") with GFP at the maximum density. Additionally, we observed a density-dependent activation of synNotch receptor for cytokine release in both CD4+ and CD8+ CAR-T cells **(****Fig. 9a** and **b),** and target killing by CD8+ cells **(****Fig. 9c****).** PLGA microparticles presenting lower density of GFP antigen may result in inadequate presentation of HER2-CAR on T cells for robust activity, explaining why initial attempts using particles functionalized by traditional surface conjugation chemistry and with far lower GFP density failed to activate synNotch CAR-T cell toxicity **(****Fig. 9c****).** This further emphasizes the importance of this new protein loading strategy for logic-gated cell modulation.

**Figure 9****.** (a) IL-2 secretion by primary human CD4+ after 48-hour co-culture with GFP-presented microparticles (ICEp-GFP) and Her2+ A375 target cells, and the amount of secretion depends on ICEp-GFP density. Data are mean ± s.d. (n = 2 technical replicates from 4 biologically independent samples). **(b)** IFN-γ secretion by CD8+ T cell, 2 days after co-incubation with ICEp of different GFP densities and HER2+ A375 target cells. Data are mean ± s.d. (n = 2 technical replicates from 4 biologically independent samples). **(c)** Target killing efficacy of AND-gate T cells primed by ICEp-GFP with varying densities, and strong killing can only be primed by particles with higher GFP density than was possible using traditional chemistry. Data are mean ± s.d. (n = 8 biological replicates from 2 independent experiments).

### EXAMPLE 9: SERUM CYTOKINE AND CHEMOKINE QUANTIFICATION AND CLINICAL CHEMISTRY OF DNA-SCAFFOLDED PARTICLES

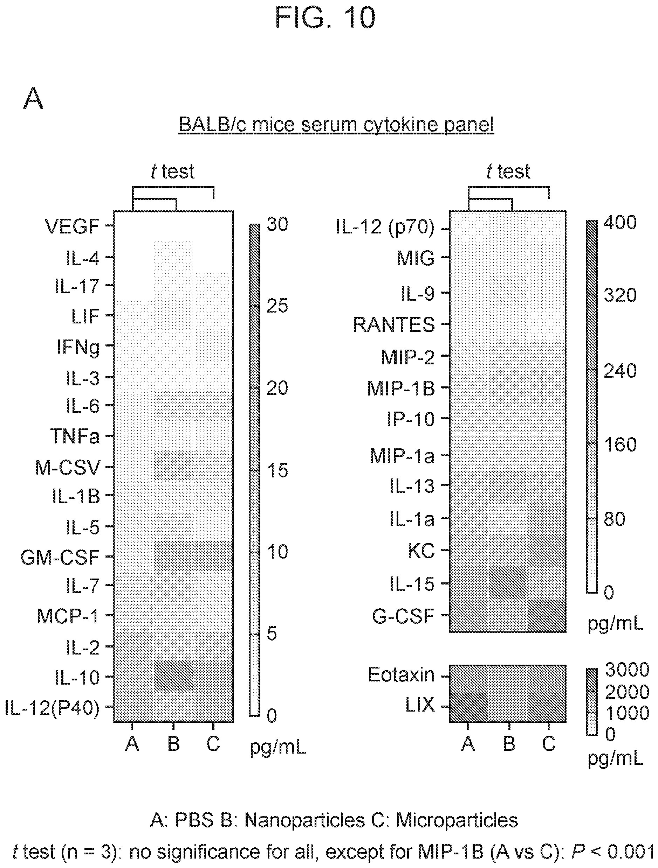

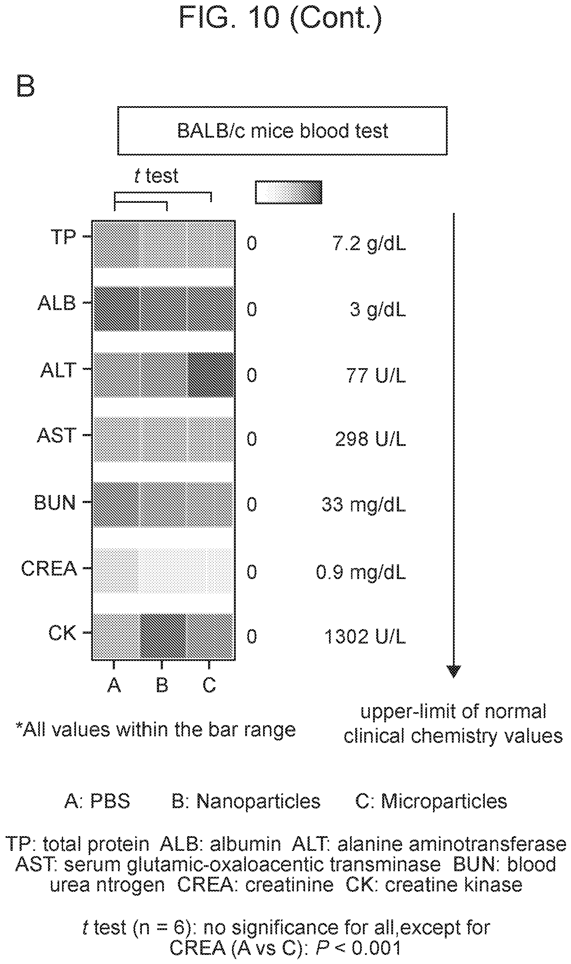

DNA-scaffolded particle constructs were injected in BALB/c mice through intravenous injection followed by serum cytokine/chemokine quantification (mouse cytokine/chemokine 31-plex) and clinical chemistry test of the blood collected 2 days later. Treatments with PLGA nanoparticles (~250 nm diameter) and microparticles (~1.5 µm diameter) tethered with DNA scaffolds and mouse isotype IgG both showed similar level of blood cytokine as PBS treatment control **(****Fig. 10a****).** Meanwhile, their blood clinical chemistry values are generally similar as the PBS control, falling within the normal range of each parameter from references **(****Fig. 10b****).**

**Figure 10****.** Serum cytokine panel (a) and blood test **(b)** of BALB/c mice 2 days after intravenous injection of isotype-IgG tethered nanoparticles (~250 nm diameter) and microparticles (~1.5 µm diameter). Data in (**a**) are mean from n = 3 mice, data in (**b**) are mean from n = 6 mice, and significance between treatment A vs B and A vs C. P values were determined by two-tailed paired t test. Cytokine secretion levels (32-panel) from nanoparticle and microparticle treatments are not different from PBS control, except for MIP-1B. Clinical chemistry values of all three treatments fall below the upper limits of normal ranges of each test, referenced from University of Arizona animal care and TREAT-NMD. SOP. Blood chemistry values of treatments of nanoparticles and microparticles are not different from PBS control, except for creatinine (CREA).

### EXAMPLE 10: ACTIVATION OF CAR-T CELLS USING CAR ANTIGENS PRESENTED ON SYNTHETIC PARTICLES

The use of chimeric antigen receptor (CAR) T cells targeting the B cell antigen CD19 has yielded remarkable clinical response in acute lymphocytic leukemia and diffuse large B cell lymphoma. However, broader clinical application of CAR-T cells still faces many challenges including i) the expansion of sufficient quantities of engineered T cells for clinical treatment with causing T cell anergy and exhaustion, and ii) the persistence and activity of potent memory CAR-T cells for durable leukemia eradication in vivo, which are important prognostic factors in achieving a meaningful clinical response in patients. In B cell cancers, CD19 antigens become depleted with the removal of both healthy and malignant B cells over the course of treatment. As a result, CAR-T counts drop and eventually become difficult to detect in patients. An approach to improve the in vivo persistence of CAR-T cells by providing replenished CD19 antigen on engineered T cells as artificial antigen presenting cells (T APC-CD19) is currently in clinical trial (clinicaltrials.gov NCT03186118). Strategies to improve in vivo expansion, persistence, and killing potential of CAR-T cells will prove critical for indications outside of B cell malignancies.

It was unexpectedly found that CAR antigens present on synthetic particles (CAPP) can activate CAR-T cells to massively proliferate, with significantly lower production of cytokines than through activation by target human leukemia cells (K562). Examples of synthetic particles include polymeric particles, magnetic particles and liposomes. This effect was observed with various antigens, including HER2, EGFR, CD22 and GFP. Staining for exhaustion markers suggests that cells expanded by CAPP exhibit much less exhaustion than those activated by target cells. The killing potency of expanded cells from CAPP activation remained the same as the original cells, whereas those expanded from target cell activation show a reduced killing ability. These features can translate into the manufacturing of CAR-T cells for ex vivo enrichment, as well as the therapeutic treatment to augment CAR-T cell abundancy in patients, simply through the dosing of CAPP. Advantages of this approach compared with the current strategy used in the T APC-CD19 clinical trial include the ease in manufacturing, potential to reduce cytokine release syndrome, and longer duration with less exhaustion of CAR-T cells. Meanwhile, local administration of CAPPs may advance CAR-T efficacy in solid tumor treatment.

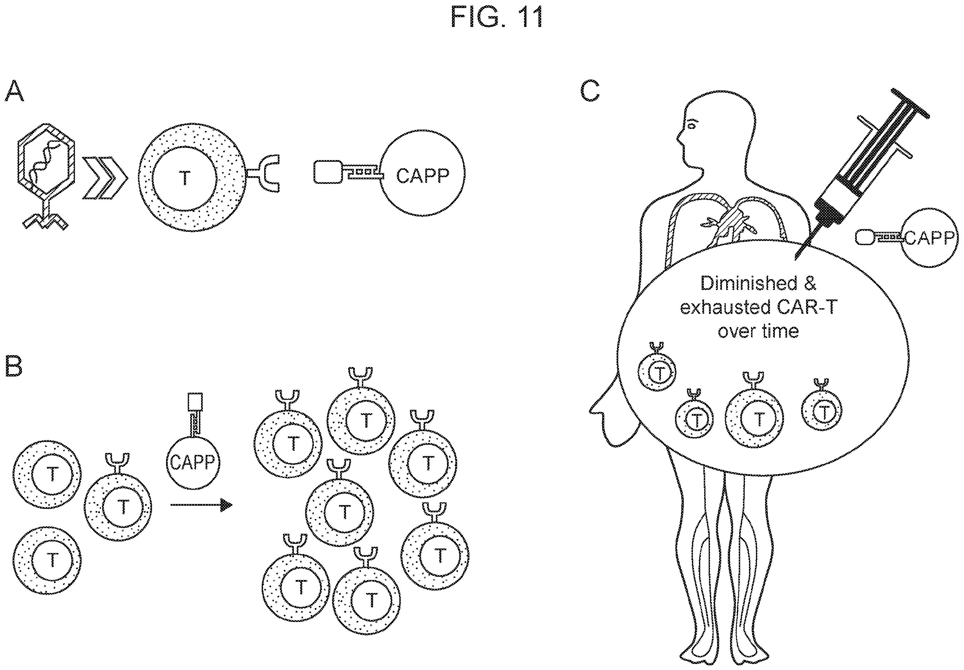

**Figure 11****.** CAR-antigen presentation particles (CAPP) for CAR-T cell proliferation. **(a, b)** Schematic illustrating use of CAPP for CAR T-cell expansion. CAR-T cells are generated targeting a particular antigen (a). Synthetic particles presenting the antigen (CAPP) can be used to drive expansion of the CAR T-cells **(b). (c)** CAPP can be used as a therapeutic treatment to augment CAR-T cell abundancy in patients, in particular in patients where cells expressing the antigen become depleted over time, resulting in a drop in CAR-T cell count (e.g. B cell cancers).

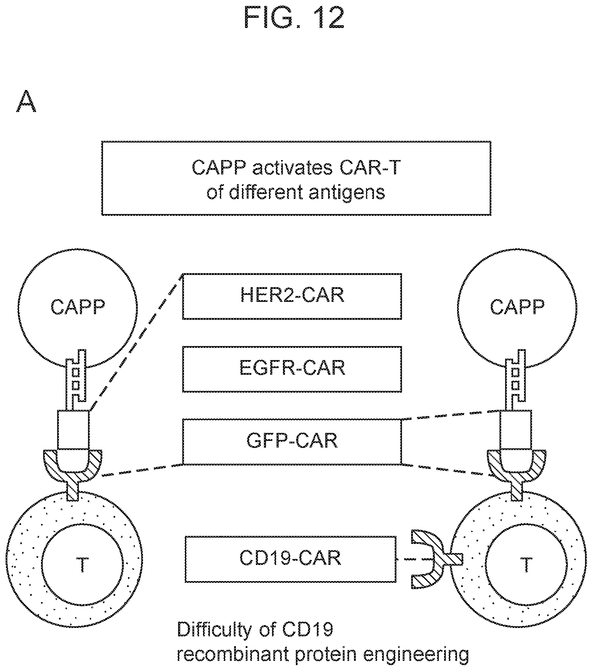

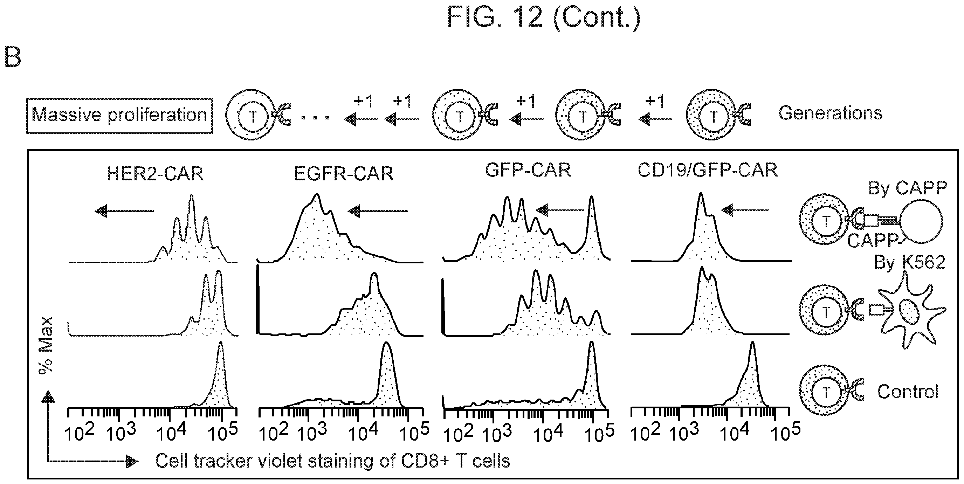

**Figure 12****.** CAPP activates CAR-T cells with different antigens. **(a)** Polymeric particles were produced presenting different antigens, namely HER2, EGFR, GFP and CD19, which were capable of activating HER2-CAR, EGFR-CAR, GFP-CAR and CD19-CAR, respectively. The polymeric CAPPs were produced as set out in the materials and methods section set out above. **(b)** CAPP presenting HER2, EGFR, GRP and CD19 were capable of inducing proliferation of the respective CAR-T cells.

CAR-T cell proliferation assay protocol:
Constitutive CAR-T cells are stained with CellTrace Violet dye (Cat# C34557) following instructions. CAR-antigen presenting particles, including PLGA nano-/microparticles, liposomes or magnetic particles are combined with cells at 10:1 (particle to cell) ratio, and incubated at 37 C for 4 days. Cells are then analyzed using flow cytometry for proliferation.

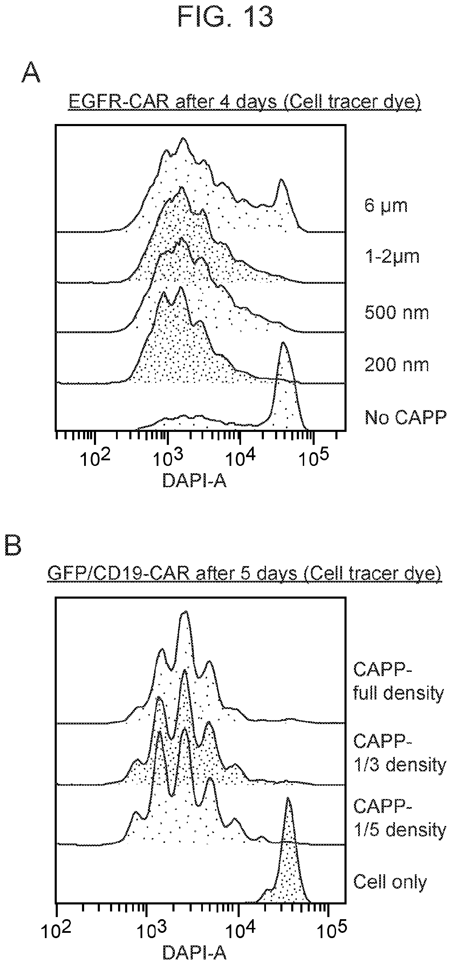

**Figure 13****.** CAPP induces cell expansion across a range of particle sizes and antigen densities. **(a)** CAPP presenting EGFR induced cell expansion of EGFR-CAR at a range of different particle sizes, **(b)** CAR-antigen density on CAPP did not affect CAPP-mediated cell expansion.

In order to determine whether synthetic particles presenting the CAR antigen other than polymeric particles were capable of inducing cell expansion, magnetic particles and liposome particles presenting CAR antigens were generated.

Magnetic particles with surface-biotin are purchased from Invitrogen (Cat #11047). Liposome particles with surface biotin were fabricated using the following protocol: 10 mg/mL DSPE (Avanti) is mixed with 19% (weight) DSPE-PEG(2000)-biotin (Avanti 880129) to prepare liposomes by traditional extrusion method. Briefly, lipid mixture dissolved in chloroform are deposited in a tube and dried to a lipid film under a stream of nitrogen followed by high vacuum for 2 hours. Samples are then hydrated in PBS buffer followed by extrusions. Streptavidin is then added to the particles as described above, followed by the addition of biotinylated protein (EGFR or GFP) for particle surface loading.

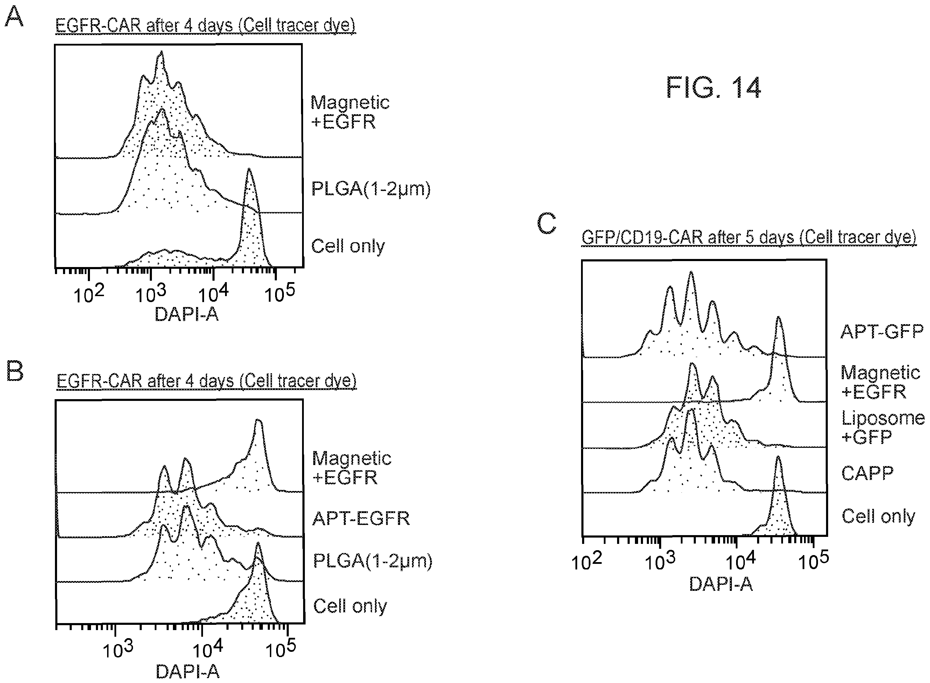

**Figure 14****.** CAR-antigen presentation on synthetic particles other than polymeric particles was also able to induce cell expansion. **(a,b)** Magnetic beads presenting EGFR (a) and antigen presenting T cells presenting EGFR (APT-EGFR) **(b)** were able to induce proliferation of EGFR-CAR. (c) APT presenting GFP (APT-GFP) and liposomes presenting GFP were able to induce proliferation of GFP/CD19-CAR.

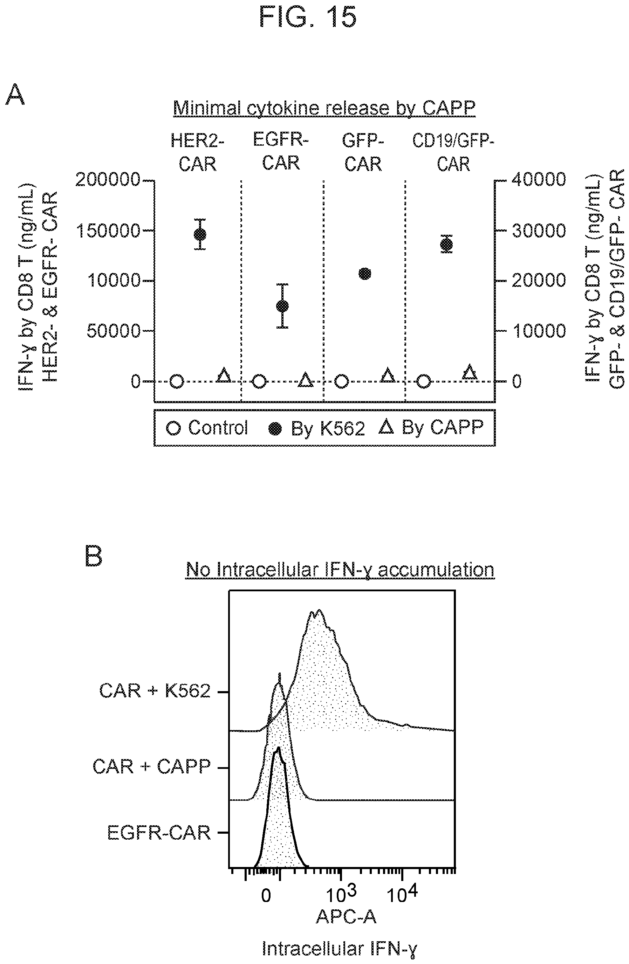

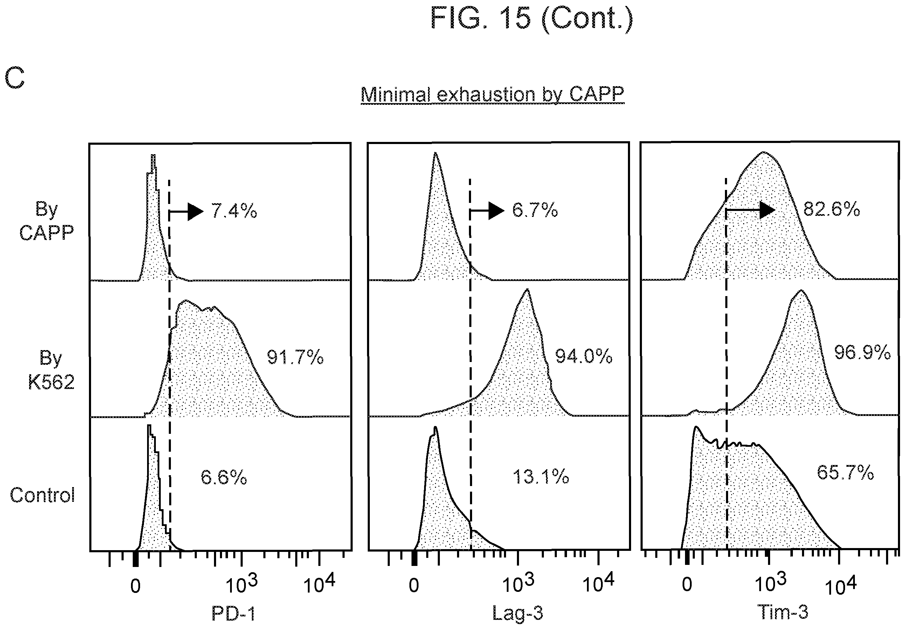

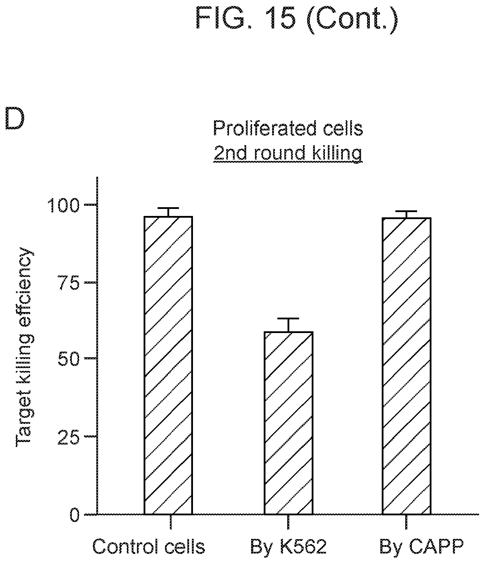

**Figure 15****.** CAPP-mediated cell proliferation does not induce cytokine production (both extracellularly and intracellularly) and does not lead to cell exhaustion. **(a,b)** Minimal IFN-γ extracellular release **(a)** and no intracellular IFN-γ accumulation **(b)** was observed using polymeric CAPP, whereas cytokine release was observed using K562 cells presenting the CAR antigen. **(c)** Staining for exhaustion markers suggests that cells expanded by CAPP exhibit much less exhaustion than those activated by target cells. **(d)** The killing potency of expanded cells from CAPP activation remained the same as the original cells, whereas those expanded from target cell activation show a reduced killing ability.

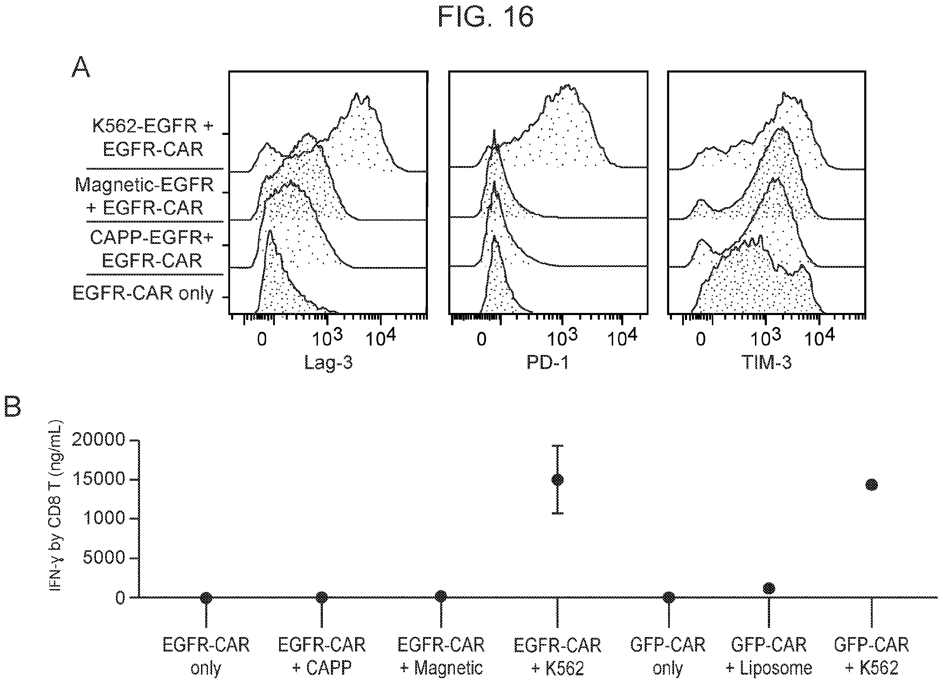

**Figure 16****.** CAR-antigen presentation on magnetic beads and liposomes does not lead to cell exhaustion and did not induce significant cytokine release. **(a)** Cell exhaustion profile of CAR-T cells expanded by magnetic-EGFR particles is similar to those activated by PLGA-EGFR (CAPP-EGFR), both significantly less than activated by K562-EGFR. **(b)** Minimal IFN-γ release was observed from CAR-T cells expanded using liposomes, magnetic particles and polymeric particles presenting the CAR antigen (termed 'Liposome', 'Magnetic', and 'CAPP', respectively), whereas IFN-γ release was observed using K562 cells presenting the CAR antigen.

### EXAMPLE 11: PCL FILM WITH DNA-HANGING PORES

DNA scaffolds were incorporated into porous thin films made of biocompatible polymer (e.g. PCL, PLGA, PLA, etc.), providing a convenient way for the attachment of functional biomolecules (e.g. proteins, antibodies, peptides, nucleic acids, etc.) with extremely high density and ratiometric control. Thin film implantable device with biomolecules functionalized through this way, can be used in various biomedical applications, such as adoptive cell transplantation for diabetes, cancer therapy, autoimmune diseases, etc, where functional biomolecule-coated film can provide a friendly environment for engineered cells. This device can be used in other local drug delivery applications as well.

**Film casting.** PCL(4k)-PEG(2k)-Mal (Creative PEGWorks Inc. Catlog #DCM-2k4k) was reacted with 3' thiol-modified DNA 17mer (5' GTTCATCTGCACCACCG 3') at 200 µM 1:1 molar ratio in the solvent of DMF:H₂O (v/v, 90:10) for overnight at room temperature. The reaction mixture was dried by vacuum evaporation at 70 °C for 3 hours. 30 nmol to 100 nmol polymer-DNA conjugates pre-dissolved in 60 µL solvent (TFE:H₂O v/v, 50:50) were mixed with 0.12mg PCL (Sigma #440744, Mn 80,000) and 0.12 mg PEG (Sigma #295906, Mn 2050) pre-dissolved in 800 µL TFE using a 3mL-syringe. The solution was applied on a clean glass board, followed by immediate film casting using a multiple clearance square applicators (Thomas Scientific Mfr. No. 5363, side 3). The glass board was air-dried in the hood for 25 minutes, followed by tearing off the film with water flush. The film was then incubated in ultrapure water for overnight with shaking, and air-dried on paper towel.

**DNA scaffold analysis.** 0.25 inch diameter film punches are incubated with excess amount of 5'FITC-labeled complementary strand (5' CGGTGGTGCAGATGAACTTCAG 3') at 37°C for 30 minutes in PBS buffer with 600 mM Na+ and 0.01% Tween-20 supplemented, followed by 3 washes using PBS buffer. Washed films are then used for confocal imaging, or dissolved in 50 uL TFE and diluted in Tris-EDTA buffer for 10 folds for fluorescence-based analysis.

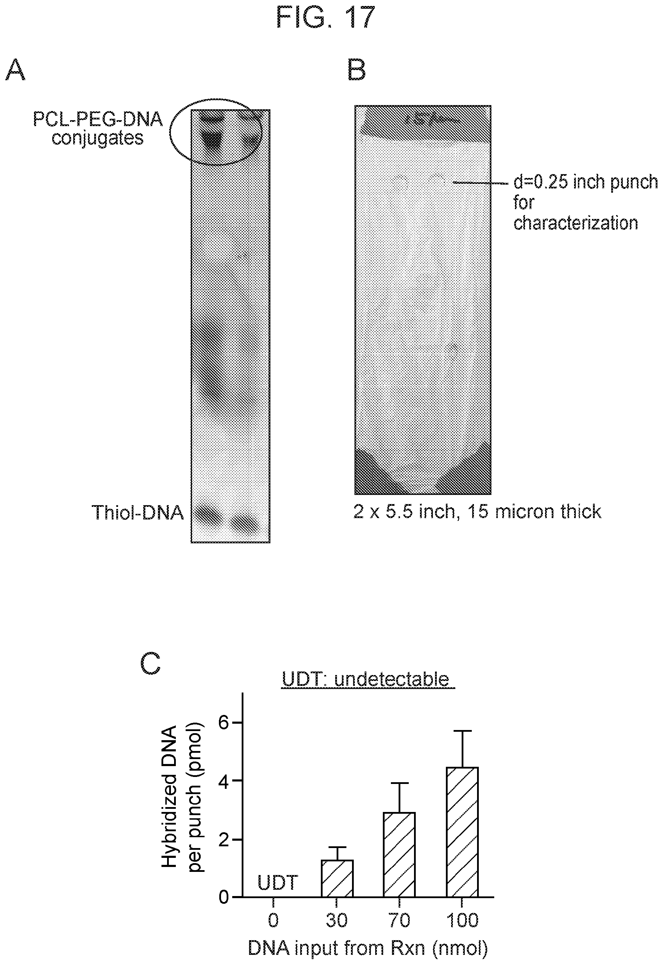

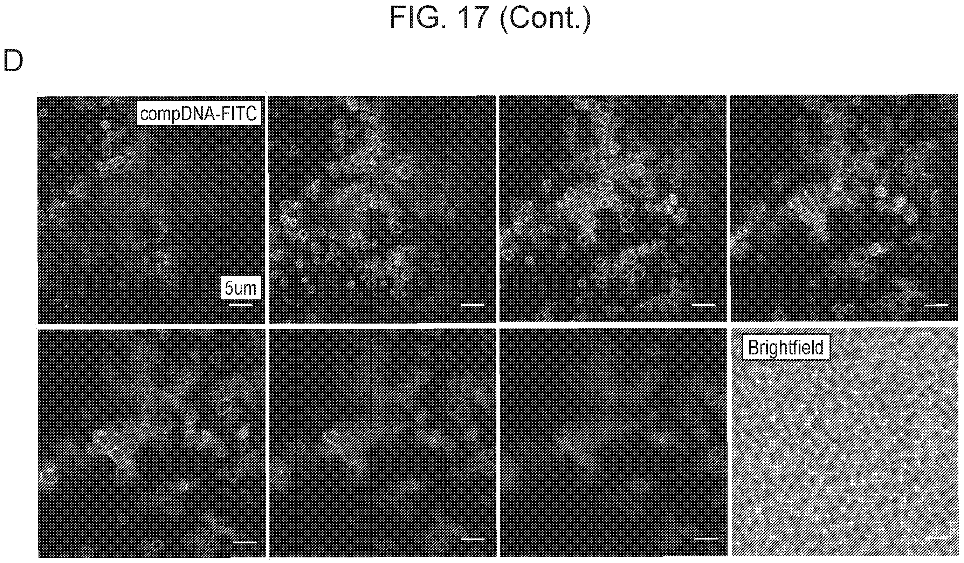

**Figure 17****.** Demonstration of DNA scaffolds in porous film device. **(a)** PCL-PEG-DNA conjugation using PCL(4k)-PEG(2k)-Mal (Creative PEGWorks Inc.). Gel shows presence of PCL-PEG-DNA conjugates, **(b)** Film casting using 4.5mg PCL(80k) + 4.5mg PEG(2k) in TFE + PCL-PEG-DNA (30, 70, 100 nmol). **(c)** DNA hybridization test. 23nt complementary strand with FITC label. Results demonstrate that DNA hybridization occurred under all PCL-PEG-DNA concentrations tested, (d) DNA pores confocal microscope Z-stack scanning, 0.6 µm between slides.

## Claims

1. A method of providing a first member of a specific-binding pair to a cell comprising a second member of the specific-binding pair, the method comprising:
contacting a polymeric particle with the cell, the particle comprising:
a polymeric core;
a nucleic acid-polymer conjugate comprising a first single stranded nucleic acid covalently attached to a polymer, wherein the polymer is non-covalently associated with the polymeric core thereby presenting the first single stranded nucleic acid on a surface of the polymeric core; and
a first binding member-nucleic acid conjugate comprising a second single stranded nucleic acid covalently attached with the first binding member, wherein the second single stranded nucleic acid is complementary to the first single stranded nucleic acid and is associated with the first single stranded nucleic acid via hybridization thereby presenting the first binding member on a surface of the polymeric particle,
wherein the first binding member specifically binds to the second binding member and wherein the second binding member is present on a surface of the cell,
wherein the particle is a nanoparticle or a microparticle.

2. The method of claim 1, wherein the polymeric core comprises poly(D,L-lactide-co-glycolide) (PLGA), poly(D,L-lactide) (PLA), polyglycolic acid (PGA), poly(e-caprolactone) (PCL), or polyethylene glycol (PEG).

3. The method of any preceding claim, wherein the cell is
i) an immune cell selected from the group consisting of a T-cell, natural killer (NK) cell, dendritic cell, macrophage, neutrophil, myeloid immune cell and B-cell, optionally wherein the immune cell has been genetically engineered, and optionally wherein the T-cell comprises regulatory T cells; or
ii) a stem cell.

4. The method of any preceding claim, wherein the cell comprises a binding-triggered transcription switch (BTSS) comprising:
a) an extracellular domain comprising the second member of the specific-binding pair that specifically binds to the first member of the specific-binding pair;
b) a binding transducer; and
c) an intracellular domain comprising a transcriptional activator or a transcriptional repressor, wherein binding of the first member of the specific-binding pair to the second member of the specific-binding pair activates the intracellular domain.

5. The method of claim 4, wherein the BTTS is a chimeric Notch polypeptide comprising, from N-terminus to C-terminus and in covalent linkage:
a) an extracellular domain comprising the second member of the specific-binding pair that is not naturally present in a Notch receptor polypeptide and that specifically binds to the first member of the specific-binding pair;
b) a Notch regulatory region comprising a Lin 12-Notch repeat, an S2 proteolytic cleavage site, and a transmembrane domain comprising an S3 proteolytic cleavage site;
c) an intracellular domain comprising a transcriptional activator or a transcriptional repressor that is heterologous to the Notch regulatory region and replaces a naturally-occurring intracellular Notch domain, wherein binding of the first member of the specific-binding pair to the second member of the specific-binding pair induces cleavage at the S2 and S3 proteolytic cleavage sites, thereby releasing the intracellular domain.

6. The method of any preceding claim, wherein the first binding member or the second binding member is selected from the group consisting of: an antibody, an antibody-based recognition scaffold, a non-antibody-based recognition scaffold, an antigen, a ligand for a receptor, a receptor, a target of a non-antibody-based recognition scaffold, an extracellular matrix component and an adhesion molecule.

7. The method of any preceding claim, wherein the first binding member comprises IL-2, such that the IL-2 is presented on the surface of the polymeric particle,
optionally wherein the second binding member is a receptor that specifically binds to the IL-2 presented on the surface of the polymeric particle.

8. The method of any preceding claim, wherein the second binding member is a single-chain Fv (scFv) or a nanobody that specifically binds to an antigen, wherein the first binding member is the antigen.

9. The method of any of claims 4 to 8, wherein the cell further comprises a transcriptional control element, responsive to the transcriptional activator, operably linked to a nucleotide sequence encoding a chimeric antigen receptor (CAR).

10. The method of any preceding claim, wherein the particle comprises a second nucleic acid-polymer conjugate comprising a third single stranded nucleic acid covalently attached to the polymer, wherein the polymer is non-covalently associated with the polymeric core thereby presenting the third single stranded nucleic acid on the surface of the polymeric core and a second first binding member-nucleic acid conjugate comprising a fourth single stranded nucleic acid covalently attached to the first binding member of a second specific-binding pair, wherein the fourth single stranded nucleic acid is complementary to the third single stranded nucleic acid and is associated with the third single stranded nucleic acid via hybridization thereby presenting the first binding member of the second specific-binding pair on a surface of the polymeric particle,
wherein the first binding member of the first specific-binding pair and the first binding member of the second specific-binding pair are present at a ratio of 10:1 to 1:10.

11. The method of claim 10, wherein the first binding member of the second specific-binding pair is an antibody that binds to a second binding member of the second specific-binding pair expressed on cell surface of a tumor cell, wherein the method comprises contacting the cell expressing the second binding member of the first specific-binding pair and the tumor cell expressing the second binding member of the second specific-binding pair with the particle, wherein the cell expressing the second binding member of the first specific-binding pair is a T cell.

12. The method of claim 10, wherein the first binding member of the first specific-binding pair is an antibody that binds to a second binding member of the first specific-binding pair, wherein the first binding member of the second specific-binding pair is an antibody that binds to a second binding member of the second specific-binding pair, wherein the second binding members of the first and second specific-binding pair are both expressed on the cell surface of a T-cell; and
wherein the method comprises contacting the T-cell expressing the second binding members of the first and second specific-binding pair with the particle, wherein binding of the first binding members to the second binding members of the first and second specific-binding pairs induces T-cell proliferation without significant increase in cytokine production.

13. The method of claim 12, wherein one of the second binding members of the first or second specific-binding pairs is CD3 and the other is CD28,
optionally wherein the first binding member that binds CD3 and the first binding member that binds CD28 are present at a ratio of 1:3 to 5:1, further optionally wherein the first binding member that binds CD3 and the first binding member that binds CD28 are present at a ratio of 3:1.

14. The method of claim 9, wherein the particle comprises a second nucleic acid-polymer conjugate comprising a third single stranded nucleic acid covalently attached to the polymer, wherein the polymer is non-covalently associated with the polymeric core thereby presenting the third single stranded nucleic acid on the surface of the polymeric core and a second first binding member-nucleic acid conjugate comprising a fourth single stranded nucleic acid covalently attached to the first binding member of a second specific-binding pair, wherein the fourth single stranded nucleic acid is complementary to the third single stranded nucleic acid and is associated with the third single stranded nucleic acid via hybridization thereby presenting the first binding member of the second specific-binding pair on a surface of the polymeric particle,
wherein the first binding member of the second specific-binding pair is an antigen that binds to CAR expressed by the cell in response to binding of the first member of the first specific-binding pair to the BTTS expressed by the cell,
wherein the cell is a T-cell and wherein binding of the CAR antigen to the T-cell induces T-cell proliferation without significant increase in cytokine production,
optionally wherein the BTTS is a chimeric Notch polypeptide.

## Patentansprüche

1. Verfahren zur Bereitstellung eines ersten Mitglieds eines spezifisch bindenden Paares für eine Zelle, die ein zweites Mitglied des spezifisch bindenden Paares umfasst, wobei das Verfahren Folgendes umfasst:
Inkontaktbringen eines polymeren Partikels mit der Zelle, wobei das Partikel umfasst:
einen polymeren Kern;
ein Nukleinsäure-Polymer-Konjugat, umfassend eine erste einzelsträngige Nukleinsäure, die kovalent an ein Polymer gebunden ist, wobei das Polymer nicht-kovalent mit dem polymeren Kern assoziiert ist, wodurch die erste einzelsträngige Nukleinsäure auf einer Oberfläche des Polymerkerns präsentiert wird; und
ein erstes Bindungselement-Nukleinsäure-Konjugat, umfassend eine zweite einzelsträngige Nukleinsäure, die kovalent an das erste Bindungselement gebunden ist, wobei die zweite einzelsträngige Nukleinsäure komplementär zu der ersten einzelsträngigen Nukleinsäure und mit ihr über Hybridisierung assoziiert ist, wodurch das erste Bindungselement auf einer Oberfläche des polymeren Partikels präsentiert wird,
wobei das erste Bindungselement spezifisch an das zweite Bindungselement bindet und wobei das zweite Bindungselement auf einer Oberfläche der Zelle angeordnet ist, wobei das Partikel ein Nanopartikel oder ein Mikropartikel ist.

2. Verfahren nach Anspruch 1, wobei der polymere Kern Poly(lactid-co-glycolid) (PLGA), Poly(D,L-lactid) (PLA), Polyglycolsäure (PGA), Poly(e-Caprolacton) (PCL), oder Polyethylenglycol (PEG) umfasst.

3. Verfahren nach einem der vorhergehenden Ansprüche, wobei die Zelle
i) eine Immunzelle ist, ausgewählt aus der Gruppe bestehend aus einer T-Zelle, natürlichen Killerzellen (NK)-Zellen, dendritischen Zellen, Makrophagen, Neutrophilen, myeloischen Immunzellen und B-Zellen, wobei die Immunzellen vorzugsweise gentechnisch verändert worden sind, und wobei die T-Zellen vorzugsweise regulatorische T-Zellen umfassen.
ii) eine Stammzelle.

4. Verfahren nach einem der vorhergehenden Ansprüche, wobei die Zelle einen Bindungsgetriggerten Transkiptionsschalter (BTSS) umfasst, der Folgendes umfasst:
a) eine extrazelluläre Domäne, die das zweite Mitglied des spezifisch bindenden Paares umfasst, welches spezifisch an das erste Mitglied des spezifisch bindenden Paares bindet;
b) einen Bindungstransduktor, und
c) eine intrazelluläre Domäne umfassend einen Transkriptionsaktivator oder einen Transkriptionsrepressor, wobei die Bindung des ersten Mitglieds des spezifisch bindenden Paares an das zweite Mitgliedes des spezifisch bindenden Paares die intrazelluläre Domäne aktiviert.

5. Verfahren nach Anspruch 4, wobei der BTSS ein chimäres Notch-Polypetid ist, das vom N-Terminus zum C-Terminus und unter kovalenter Bindung Folgendes umfasst:
a) eine extrazelluläre Domäne, die das zweite Mitglied des spezifisch bindenden Paares umfasst, welches nicht natürlicherweise im Notch-Rezeptor-Polypeptid vorkommt und welches spezifisch an das erste Mitglied des spezifisch bindenden Paares bindet;
b) eine Notch-Regulierungsregion, die eine Lin 12-Notch-Wiederholung, eine S2-proteolytische Spaltstelle, und eine Transmembrandomäne umfasst, die eine S3-Proteolysespaltstelle umfasst;
c) eine intrazelluläre Domäne, umfassend einen Transkriptionsaktivator oder einen Transkriptionsrepressor, der heterolog zu der Notch-Regulationsregion ist und eine natürlich vorkommende intrazelluläre Notch-Domäne ersetzt, wobei die Bindung des ersten Mitglieds des spezifisch bindenden Paares an das zweite Mitglied des spezifisch bindenden Paares die Spaltung an den S2 und S3 proteolytischen Spaltstellen induziert, wodurch die intrazelluläre Domäne freigesetzt wird.

6. Verfahren nach einem der vorhergehenden Ansprüche, wobei das erste Bindungselement oder das zweite Bindungselement ausgewählt sind aus der Gruppe bestehend aus: einem Antikörper, einem Erkennungsgerüst auf Antikörperbasis, einem Erkennungsgerüst auf nicht-Antikörperbasis, einem Antigen, einem Liganden für einen Rezeptor, einem Rezeptor, einem Ziel für ein Erkennungsgerüst auf nicht-Antikörperbasis, einer extrazellulären Matrixkomponente, und einem Adhäsionsmolekül.

7. Verfahren nach einem der vorhergehenden Ansprüche, wobei das erste Bindungselement IL-2 umfasst, wodurch IL-2 auf der Oberfläche des polymeren Partikels präsentiert wird, wobei insbesondere das zweite Bindungselement ein Rezeptor ist der spezifisch IL-2 bindet, welches auf der Oberfläche des polymeren Partikels präsentiert wird.

8. Verfahren nach einem der vorhergehenden Ansprüche, wobei das zweite Bindungselement ein einkettiges Fv (scFv) oder ein Nanokörper ist, welche spezifisch an ein Antigen binden, wobei das erste Bindungselement das Antigen ist.

9. Verfahren nach einem der Ansprüche 4 bis 8, wobei die Zelle außerdem ein Transkriptionskontrollelement umfasst, welches responsiv zu dem Transkriptionsaktivator ist und welches operativ mit einer Nukleotidsequenz verbunden ist, die für einen chimären Antigenrezeptor (CAR) kodiert.

10. Verfahren nach einem der vorhergehenden Ansprüche, wobei das Partikel ein zweites Nukleinsäure-Polymer-Konjugat umfasst, das eine dritte einzelsträngige Nukleinsäure umfasst, welche kovalent an das Polymer gebunden ist, wobei das Polymer nicht-kovalent mit dem polymeren Kern assoziiert ist, wodurch die dritte einzelsträngige Nukleinsäure auf der Oberfläche des polymeren Kerns präsentiert wird, und ein zweites erstes Bindungselement-Nukleinsäure-Konjugat umfasst, das eine vierte einzelsträngige Nukleinsäure aufweist, welche kovalent mit dem ersten Bindungselement eines zweiten spezifisch bindenden Paares verbunden ist, wobei die vierte einzelsträngige Nukleinsäure komplementär zur dritten einzelsträngigen Nukleinsäure ist und mit ihr über Hybridisierung assoziiert ist, wodurch das erste Bindungselement des zweiten spezifisch bindenden Paares auf einer Oberfläche des polymeren Partikels präsentiert wird, wobei das erste Bindungselement des ersten spezifisch bindenden Paares und das erste Bindungselement des zweiten spezifisch bindenden Paares in einem Verhältnis von 10:1 bis 1:10 auftreten.

11. Verfahren nach Anspruch 10, wobei das erste Bindungselement des zweiten spezifisch bindenden Paares ein Antikörper ist, der an das zweite Bindungselement des zweiten spezifisch bindenden Paares bindet, welches auf der Zelloberfläche einer Tumorzelle exprimiert wird, wobei das Verfahren umfasst: Inkontaktbringen der Zelle, die das zweite Bindungselement des ersten spezifisch bindenden Paares exprimiert, und der Tumorzelle, die die das zweite Bindungselement des zweiten spezifisch bindenden Paares exprimiert, mit dem Partikel, wobei Zelle, die das zweite Bindungselement des ersten spezifisch bindenden Paares exprimiert, eine T-Zelle ist.

12. Verfahren nach Anspruch 10, wobei das erste Bindungselement des ersten spezifisch bindenden Paares ein Antikörper ist, der an das zweite Bindungselement des ersten spezifisch bindenden Paares bindet, wobei das erste Bindungselement des zweiten spezifisch bindenden Paares ein Antikörper ist, der an das zweite Bindungselement des zweiten spezifisch bindenden Paares bindet, wobei die zweiten Bindungselemente des ersten und des zweiten spezifisch bindenden Paares jeweils auf der Zelloberfläche einer T-Zelle exprimiert werden; und
wobei das Verfahren umfasst: Inkontaktbringen der T-Zelle, die das zweite Bindungselement des ersten und zweiten spezifisch bindenden Paares exprimiert, mit dem Partikel, wobei die Bindung des ersten Bindungselements an das zweite Bindungselement des ersten oder zweiten spezifisch bindenden Paares eine T-Zell-Proliferation ohne signifikanten Anstieg der Zytokinproduktion inudziert.

13. Verfahren nach Anspruch 12, wobei eins der zweiten Bindungselemente des ersten und zweiten spezifisch bindenden Paares CD3 und das jeweils andere CD28 ist, wobei insbesondere das erste Bindungselement, welches CD3 bindet und das erste Bindungselement, welches CD28 bindet, in einem Verhältnis von 1:3 bis 5:1 auftreten, wobei insbesondere das erste Bindungselement, welches CD3 bindet und das erste Bindungselement, welches CD28 bindet, in einem Verhältnis von 3:1 auftreten.

14. Verfahren nach Anspruch 9, wobei das Partikel ein zweites Nukleinsäure-Polymer-Konjugat umfasst, das eine dritte einzelsträngige Nukleinsäure umfasst, welche kovalent an das Polymer gebunden ist, wobei das Polymer nicht-kovalent mit dem polymeren Kern assoziiert ist, wodurch die dritte einzelsträngige Nukleinsäure auf der Oberfläche des polymeren Kerns präsentiert wird, und ein zweites erstes Bindungselement-Nukleinsäure-Konjugat umfasst, die eine vierte einzelsträngige Nukleinsäure aufweist, welche kovalent mit dem ersten Bindungselement eines zweiten spezifisch bindenden Paares verbunden ist, wobei die vierte einzelsträngige Nukleinsäure komplementär zur dritten einzelsträngigen Nukleinsäure ist und mit ihr über Hybridisierung assoziiert ist, wodurch das erste Bindungselement des zweiten spezifisch bindenden Paares auf einer Oberfläche des polymeren Partikels präsentiert wird, wobei das erste Bindungselement des zweiten spezifisch bindenden Paares ein Antigen ist, das an CAR bindet, das von den Zellen als Antwort auf die Bindung des ersten Bindungselements des ersten spezifisch bindenden Paares an von den Zellen exprimiertes BTTS exprimiert wird, wobei die Zelle eine T-Zelle ist und wobei die Bindung von CAR-Antigen an die T-Zelle eine T-Zell-Proliferation ohne signifikanten Anstieg der Zytokinproduktion induziert, wobei BTTS insbesondere ein chimäres Notch-Polypeptid ist.

## Revendications

1. - Procédé pour fournir un premier membre d'une paire à liaison spécifique à une cellule comprenant un second membre de la paire à liaison spécifique, le procédé comprenant :
la mise en contact d'une particule polymère avec la cellule, la particule comprenant :
- un noyau polymère ;
- un conjugué acide nucléique-polymère comprenant un premier acide nucléique simple brin fixé de manière covalente à un polymère, le polymère étant associé de manière non covalente au noyau polymère, présentant ainsi le premier acide nucléique simple brin sur une surface du noyau polymère ; et
- un conjugué premier membre de liaison-acide nucléique comprenant un deuxième acide nucléique simple brin fixé de manière covalente au premier membre de liaison, le deuxième acide nucléique simple brin étant complémentaire du premier acide nucléique simple brin et étant associé au premier acide nucléique simple brin par hybridation, présentant ainsi le premier membre de liaison sur une surface de la particule polymère,
le premier membre de liaison se liant spécifiquement au second membre de liaison et le second membre de liaison étant présent sur une surface de la cellule,
la particule étant une nanoparticule ou une microparticule.

2. - Procédé selon la revendication 1, dans lequel le noyau polymère comprend du poly(D,L-lactide-co-glycolide) (PLGA), du poly(D,L-lactide) (PLA), de l'acide polyglycolique (PGA), du poly(e-caprolactone) (PCL) ou du polyéthylène glycol (PEG).

3. - Procédé selon l'une quelconque des revendications précédentes, dans lequel la cellule est
i) une cellule immunitaire choisie dans le groupe constitué d'un lymphocyte T, d'une cellule tueuse naturelle (NK), d'une cellule dendritique, d'un macrophage, d'un granulocyte neutrophile, d'une cellule immunitaire myéloïde et d'un lymphocyte B, facultativement dans lequel la cellule immunitaire a été génétiquement modifiée, et facultativement dans lequel le lymphocyte T comprend des lymphocytes T régulateurs ; ou
ii) une cellule souche.

4. - Procédé selon l'une quelconque des revendications précédentes, dans lequel la cellule comprend un commutateur transcriptionnel déclenché par liaison (BTSS) comprenant :
a) un domaine extracellulaire comprenant le second membre de la paire à liaison spécifique qui se lie spécifiquement au premier membre de la paire à liaison spécifique ;
b) un transducteur de liaison ; et
c) un domaine intracellulaire comprenant un activateur transcriptionnel ou un répresseur transcriptionnel, une liaison du premier membre de la paire à liaison spécifique au second membre de la paire à liaison spécifique activant le domaine intracellulaire.

5. - Procédé selon la revendication 4, dans lequel le BTTS est un polypeptide Notch chimérique comprenant, de l'extrémité N-terminale à l'extrémité C-terminale et en liaison covalente :
a) un domaine extracellulaire comprenant le second membre de la paire à liaison spécifique qui n'est pas naturellement présent dans un polypeptide récepteur Notch et qui se lie spécifiquement au premier membre de la paire à liaison spécifique ;
b) une région régulatrice Notch comprenant une répétition Lin l2-Notch, un site de clivage protéolytique S2 et un domaine transmembranaire comprenant un site de clivage protéolytique S3 ;
c) un domaine intracellulaire comprenant un activateur transcriptionnel ou un répresseur transcriptionnel qui est hétérologue à la région régulatrice de Notch et remplace un domaine Notch intracellulaire d'origine naturelle, une liaison du premier membre de la paire à liaison spécifique au second membre de la paire à liaison spécifique induisant un clivage aux sites de clivage protéolytique S2 et S3, libérant ainsi le domaine intracellulaire.

6. - Procédé selon l'une des revendications précédentes, dans lequel le premier membre de liaison ou le second membre de liaison est choisi dans le groupe consistant en : un anticorps, un échafaudage de reconnaissance à base d'anticorps, un échafaudage de reconnaissance non à base d'anticorps, un antigène, un ligand pour un récepteur, un récepteur, une cible d'un échafaudage de reconnaissance non à base d'anticorps, un composant de matrice extracellulaire et une molécule d'adhésion.

7. - Procédé selon l'une quelconque des revendications précédentes, dans lequel le premier membre de liaison comprend IL-2, de telle sorte que l'IL-2 est présenté sur la surface de la particule polymère, facultativement dans lequel le second membre de liaison est un récepteur qui se lie spécifiquement à l'IL-2 présentée sur la surface de la particule polymère.

8. - Procédé selon l'une quelconque des revendications précédentes, dans lequel le second membre de liaison est un Fv à chaîne unique (scFv) ou un nanocorps qui se lie spécifiquement à un antigène, le premier membre de liaison étant l'antigène.

9. - Procédé selon l'une quelconque des revendications 4 à 8, dans lequel la cellule comprend en outre un élément de contrôle transcriptionnel, sensible à l'activateur transcriptionnel, lié de manière fonctionnelle à une séquence nucléotidique codant pour un récepteur antigénique chimérique (CAR).

10. - Procédé selon l'une quelconque des revendications précédentes, dans lequel la particule comprend un second conjugué acide nucléique-polymère comprenant un troisième acide nucléique simple brin fixé de manière covalente au polymère, dans lequel le polymère est associé de manière non covalente au noyau polymère, présentant ainsi le troisième acide nucléique simple brin sur la surface du noyau polymère, et un second conjugué premier membre de liaison-acide nucléique comprenant un quatrième acide nucléique simple brin fixé de manière covalente au premier membre de liaison d'une seconde paire à liaison spécifique, dans lequel le quatrième acide nucléique simple brin est complémentaire du troisième acide nucléique simple brin et est associé au troisième acide nucléique simple brin par hybridation, présentant ainsi le premier membre de liaison de la seconde paire à liaison spécifique sur une surface de la particule polymère,
dans lequel le premier membre de liaison de la première paire à liaison spécifique et le premier membre de liaison de la seconde paire à liaison spécifique sont présents dans un rapport de 10:1 à 1:10.

11. - Procédé selon la revendication 10, dans lequel le premier membre de liaison de la seconde paire à liaison spécifique est un anticorps qui se lie à un second membre de liaison de la seconde paire à liaison spécifique exprimée sur une surface cellulaire d'une cellule tumorale, dans lequel le procédé comprend la mise en contact de la cellule exprimant le second membre de liaison de la première paire à liaison spécifique et de la cellule tumorale exprimant le second membre de liaison de la seconde paire à liaison spécifique avec la particule, la cellule exprimant le second membre de liaison de la première paire à liaison spécifique étant un lymphocyte T.

12. - Procédé selon la revendication 10, dans lequel le premier membre de liaison de la première paire à liaison spécifique est un anticorps qui se lie à un second membre de liaison de la première paire à liaison spécifique, dans lequel le premier membre de liaison de la seconde paire à liaison spécifique est un anticorps qui se lie à un second membre de liaison de la seconde paire à liaison spécifique, dans lequel les seconds membres de liaison de la première et de la seconde paire à liaison spécifique sont tous deux exprimés sur la surface cellulaire d'un lymphocyte T ; et
le procédé comprenant la mise en contact du lymphocyte T exprimant les seconds membres de liaison de la première et de la seconde paire à liaison spécifique avec la particule, la liaison des premiers membres de liaison aux seconds membres de liaison des première et seconde paires à liaison spécifique induisant une prolifération du lymphocyte T sans augmentation significative de la production de cytokine.

13. - Procédé selon la revendication 12, dans lequel l'un des seconds membres de liaison des première ou seconde paires à liaison spécifique est CD3 et l'autre est CD28,
facultativement, dans lequel le premier membre de liaison qui lie CD3 et le premier membre de liaison qui lie CD28 sont présents dans un rapport de 1:3 à 5:1, également facultativement dans lequel le premier membre de liaison qui lie CD3 et le premier membre de liaison qui lie CD28 sont présents dans un rapport de 3:1.

14. - Procédé selon la revendication 9, dans lequel la particule comprend un second conjugué acide nucléique-polymère comprenant un troisième acide nucléique simple brin fixé de manière covalente au polymère, dans lequel le polymère est associé de manière non covalente au noyau polymère, présentant ainsi le troisième acide nucléique simple brin sur la surface du noyau polymère et un second conjugué premier membre de liaison-acide nucléique comprenant un quatrième acide nucléique simple brin fixé de manière covalente au premier membre de liaison d'une seconde paire à liaison spécifique, dans lequel le quatrième acide nucléique simple brin est complémentaire du troisième acide nucléique simple brin et est associé au troisième acide nucléique simple brin par hybridation, présentant ainsi le premier membre de liaison de la seconde paire à liaison spécifique sur une surface de la particule polymère,
dans lequel le premier membre de liaison de la seconde paire à liaison spécifique est un antigène qui se lie au CAR exprimé par la cellule en réponse à la liaison du premier membre de la première paire à liaison spécifique au BTTS exprimé par la cellule,
dans lequel la cellule est un lymphocyte T et dans lequel une liaison de l'antigène CAR au lymphocyte T induit une prolifération du lymphocyte T sans augmentation significative de la production de cytokine,
facultativement dans lequel le BTTS est un polypeptide Notch chimérique.
